# EUROPEAN PATENT APPLICATION

(11) **EP 2 339 034 A1**
(43) Date of publication of application: **29.06.2011**
(21) Application number: 10181608.0
(22) Date of filing: 04.11.1997
(51) Int. Cl.: C12Q 1/68

(54) **Species-specific, genus-specific and universal DNA probes and amplification primers to rapidly detect and identify common bacterial and fungal pathogens and associated antibiotic resistance genes from clinical specimens for diagnosis in microbiology laboratories**

(30) Priority: 04.11.1996 US 743637
(62) Divisional of application: 09163281.0
(71) Applicant: Geneohm Sciences Canada, Inc., Sainte-Foy, QC G1V 2K8 (CA)
(72) Inventor: Bergeron, Michel G., Sillery Québec G1T 1G2 (CA); Picard, Francois, Cap-Rouge Québec G1Y 1A1 (CA); Ouellette, Marc, Sillery Québec G1S 3S1 (CA); Roy, Paul H., Loretteville Québec G2A 2X8 (CA)
(74) Representative: Pohlman, Sandra M.

(57) **Abstract**

DNA-based methods employing amplification primers or probes for detecting, identifying, and quantifying in a test sample DNA from (i) any bacterium, (ii) the species *Streptococcus agalactiae, Staphylococcus saprophyticus, Enterococcus faecium, Neisseria meningitidis, Listeria monocytogenes* and *Candida albicans,* and (iii) any species of the genera *Streptococcus, Staphylococcus, Enterococcus, Neisseria* and *Candida* are disclosed. DNA-based methods employing amplification primers or probes for detecting, identifying, and quantifying in a test sample antibiotic resistance genes selected from the group consisting of *bla ₜₑₘ, bla _{rob}, bla ₛₕᵥ, bla ₒₓₐ , blaZ, aadB*, *aacC1, aacC2*, *aacC3*, *aacA4, aac6'-IIa*, *ermA, ermB, ermC, mecA, vanA, vanB, vanC, satA, aac(6')-aph(2"), aad(6')*, *vat, vga, msrA, sul* and *int* are also disclosed. The above microbial species, genera and resistance genes arc all clinically relevant and commonly encountered in a variety of clinical specimens. These DNA-based assays are rapid, accurate and can be used in clinical microbiology laboratories for routine diagnosis. These novel diagnostic tools should be useful to improve the speed and accuracy of diagnosis of microbial infections, thereby allowing more effective treatments. Diagnostic kits for (i) the universal detection and quantification of bacteria, and/or (ii) the detection, identification and quantification of the above-mentioned bacterial and fungal species and/or genera, and/or (iii) the detection, identification and quantification of the above-mentioned antibiotic resistance genes arc also claimed.

## Description

### BACKGROUND OF THE INVENTION

### Classical methods for the identification and susceptibility testing of bacteria

Bacteria are classically identified by their ability to utilize different substrates as a source of carbon and nitrogen through the use of biochemical tests such as the API20E™ system (bioMérieux). For susceptibility testing, clinical microbiology laboratories use methods including disk diffusion, agar dilution and broth microdilution. Although identifications based on biochemical tests and antibacterial susceptibility tests are cost-effective, at least two days are required to obtain preliminary results due to the necessity of two successive overnight incubations to identify the bacteria from clinical specimens as well as to determine their susceptibility to antimicrobial agents. There are some commercially available automated systems (i.e. the MicroScan system from Dade Diagnostics Corp. and the Vitek system from bioMérieux) which use sophisticated and expensive apparatus for faster microbial identification and susceptibility testing (Stager and Davis, 1992, Clin. Microbiol. Rev. 5:302-327). These systems require shorter incubation periods, thereby allowing most bacterial identifications and susceptibility testing to be performed in less than 6 hours. Nevertheless, these faster systems always require the primary isolation of the bacteria as a pure culture, a process which takes at least 18 hours for a pure culture or 2 days for a mixed culture. The fastest identification system, the autoSCAN-Walk-Away™ system (Dade Diagnostics Corp.) identifies both gram-negative and gram-positive bacterial species from standardized inoculum in as little as 2 hours and gives susceptibility patterns to most antibiotics in 5.5 hours. However, this system has a particularly high percentage (i.e. 3.3 to 40.5%) of non-conclusive identifications with bacterial species other than *Enterobacteriaceae* (Croizé J., 1995, Lett. Infectiol. 10:109-113; York et al., 1992, J. Clin. Microbiol. 30:2903-2910). For *Enterobacteriaceae,* the percentage of non-conclusive identifications was 2.7 to 11.4%.

A wide variety of bacteria and fungi are routinely isolated and identified from clinical specimens in microbiology laboratories. Tables 1 and 2 give the incidence for the most commonly isolated bacterial and fungal pathogens from various types of clinical specimens. These pathogens are the most frequently associated with nosocomial and community-acquired human infections and are therefore considered the most clinically important.

### Clinical specimens tested in clinical microbiology laboratories

Most clinical specimens received in clinical microbiology laboratories are urine and blood samples. At the microbiology laboratory of the Centre Hospitalier de l'Université Laval (CHUL), urine and blood account for approximately 55% and 30% of the specimens received, respectively (Table 3). The remaining 15% of clinical specimens comprise various biological fluids including sputum, pus, cerebrospinal fluid, synovial fluid, and others (Table 3). Infections of the urinary tract, the respiratory tract and the bloodstream are usually of bacterial etiology and require antimicrobial therapy. In fact, all clinical samples received in the clinical microbiology laboratory are tested routinely for the identification of bacteria and susceptibility testing.

### Conventional pathogen identification from clinical specimens

### Urine specimens

The search for pathogens in urine specimens is so preponderant in the routine microbiology laboratory that a myriad of tests have been developed. However, the gold standard remains the classical semi-quantitative plate culture method in which 1 µL of urine is streaked on plates and incubated for 18-24 hours. Colonies are then counted to determine the total number of colony forming units (CFU) per liter of urine. A bacterial urinary tract infection (UTI) is normally associated with a bacterial count of 10⁷ CFU/L or more in urine. However, infections with less than 10⁷ CFU/L in urine are possible, particularly in patients with a high incidence of diseases or those catheterized (Stark and Maki, 1984, N. Engl. J. Med. 311:560-564). Importantly, approximately 80% of urine specimens tested in clinical microbiology laboratories are considered negative (i.e. bacterial count of less than 10⁷ CFU/L; Table 3). Urine specimens found positive by culture are further characterized using standard biochemical tests to identify the bacterial pathogen and are also tested for susceptibility to antibiotics. The biochemical and susceptibility testing normally require 18-24 hours of incubation.

Accurate and rapid urine screening methods for bacterial pathogens would allow a faster identification of negative specimens and a more efficient treatment and care management of patients. Several rapid identification methods (Uriscreen™, UTIscreen™, Flash Track™ DNA probes and others) have been compared to slower standard biochemical methods, which are based on culture of the bacterial pathogens. Although much faster, these rapid tests showed low sensitivities and poor specificities as well as a high number of false negative and false positive results (Koening et al., 1992, J. Clin. Microbiol. 30:342-345; Pezzlo et al., 1992, J. Clin. Microbiol. 30:640-684).

### Blood specimens

The blood specimens received in the microbiology laboratory are always submitted for culture. Blood culture systems may be manual, semi-automated or completely automated. The BACTEC system (from Becton Dickinson) and the BacTAlert system (from Organon Teknika Corporation) are the two most widely used automated blood culture systems. These systems incubate blood culture bottles under optimal conditions for bacterial growth. Bacterial growth is monitored continuously to detect early positives by using highly sensitive bacterial growth detectors. Once growth is detected, a Gram stain is performed directly from the blood culture and then used to inoculate nutrient agar plates. Subsequently, bacterial identification and susceptibility testing are carried out from isolated bacterial colonies with automated systems as described previously. The bottles are normally reported as negative if no growth is detected after an incubation of 6 to 7 days. Normally, the vast majority of blood cultures are reported negative. For example, the percentage of negative blood cultures at the microbiology laboratory of the CHUL for the period February 1994-January 1995 was 93.1 % (Table 3).

### Other clinical samples

Upon receipt by the clinical microbiology laboratory, all body fluids other than blood and urine that are from normally sterile sites (i.e. cerebrospinal, synovial, pleural, pericardial and others) are processed for direct microscopic examination and subsequent culture. Again, most clinical samples are negative for culture (Table 3).

Regarding clinical specimens which are not from sterile sites such as sputum or stool specimens, the laboratory diagnosis by culture is more problematic because of the contamination by the normal flora. The bacterial pathogens potentially associated with the infection are purified from the contaminants and then identified as described previously. Of course, the universal detection of bacteria would not be useful for the diagnosis of bacterial infections at these non sterile sites. On the other hand, DNA-based assays for species or genus detection and identification as well as for the detection of antibiotic resistance genes from these specimens would be very useful and would offer several advantages over classical identification and susceptibility testing methods.

### DNA-based assays with any clinical specimens

There is an obvious need for rapid and accurate diagnostic tests for bacterial detection and identification directly from clinical specimens. DNA-based technologies are rapid and accurate and offer a great potential to improve the diagnosis of infectious diseases (Persing et al., 1993, Diagnostic Molecular Microbiology: Principles and Applications, American Society for Microbiology, Washington, D.C.). The DNA probes and amplification primers which are objects of the present invention are applicable for bacterial or fungal detection and identification directly from any clinical specimens such as blood cultures, blood, urine, sputum, cerebrospinal fluid, pus and other type of specimens (Table 3). The DNA-based tests proposed in this invention are superior in terms of both rapidity and accuracy to standard biochemical methods currently used for routine diagnosis from any clinical specimens in microbiology laboratories. Since these tests are performed in around only one hour, they provide the clinicians with new diagnostic tools which should contribute to increase the efficiency of therapies with antimicrobial agents. Clinical specimens from organisms other than humans (e.g. other primates, birds, plants, mammals, farm animals, livestock and others) may also be tested with these assays.

### A high percentage of culture negative specimens

Among all the clinical specimens received for routine diagnosis, approximately 80% of urine specimens and even more (around 95%) for other types of clinical specimens are negative for the presence of bacterial pathogens (Table 3). It would also be desirable, in addition to identify bacteria at the species or genus level in a given specimen, to screen out the high proportion of negative clinical specimens with a test detecting the presence of any bacterium (i.e. universal bacterial detection). Such a screening test may be based on the DNA amplification by PCR of a highly conserved genetic target found in all bacteria. Specimens negative for bacteria would not be amplified by this assay. On the other hand, those that are positive for bacteria would give a positive amplification signal with this assay.

### Towards the development of rapid DNA-based diagnostic tests

A rapid diagnostic test should have a significant impact on the management of infections. DNA probe and DNA amplification technologies offer several advantages over conventional methods for the identification of pathogens and antibiotic resistance genes from clinical samples (Persing et al., 1993, Diagnostic Molecular Microbiology: Principles and Applications, American Society for Microbiology, Washington, D.C.; Ehrlich and Greenberg, 1994, PCR-based Diagnostics in Infectious Disease, Blackwell Scientific Publications, Boston, MA). There is no need for culture of the bacterial pathogens, hence the organisms can be detected directly from clinical samples, thereby reducing the time associated with the isolation and identification of pathogens. Furthermore, DNA-based assays are more accurate for bacterial identification than currently used phenotypic identification systems which are based on biochemical tests. Commercially available DNA-based technologies are currently used in clinical microbiology laboratories, mainly for the detection and identification of fastidious bacterial pathogens such as *Mycobacterium tuberculosis, Chlamydia trachomatis, Neisseria gonorrhoeae* as well as for the detection of a variety of viruses (Podzorski and Persing, Molecular detection and identification of microorganisms, In : P. Murray et al., 1995, Manual of Clinical Microbiology, ASM press, Washington D.C.). There are also other commercially available DNA-based assays which are used for culture confirmation assays.

Others have developed DNA-based tests for the detection and identification of bacterial pathogens which are objects of the present invention: *Staphylococcus* spp. (US patent application serial No. US 5 437 978), *Neisseria* spp. (US patent application serial No. US 5 162 199 and European patent application serial No. EP 0 337 896 131) and *Listeria monocytogenes* (US patent applications serial Nos US 5 389 513 and US 5 089 386). However, the diagnostic tests described in these patents are based either on rRNA genes or on genetic targets different from those described in the present invention.

Although there are diagnostic kits or methods already used in clinical microbiology laboratories, there is still a need for an advantageous alternative to the conventional culture identification methods in order to improve the accuracy and the speed of the diagnosis of commonly encountered bacterial infections. Besides being much faster, DNA-based diagnostic tests are more accurate than standard biochemical tests presently used for diagnosis because the bacterial genotype (e.g. DNA level) is more stable than the bacterial phenotype (e.g. metabolic level).

Knowledge of the genomic sequences of bacterial and fungal species continuously increases as testified by the number of sequences available from databases. From the sequences readily available from databases, there is no indication therefrom as to their potential for diagnostic purposes. For determining good candidates for diagnostic purposes, one could select sequences for DNA-based assays for (i) the species-specific detection and identification of commonly encountered bacterial or fungal pathogens, (ii) the genus-specific detection and identification of commonly encountered bacterial or fungal pathogens, (iii) the universal detection of bacterial or fungal pathogens and/or (iv) the specific detection and identification of antibiotic resistance genes. All of the above types of DNA-based assays may be performed directly from any type of clinical specimens or from a microbial culture.

In our co-pending U.S. (N.S. 08/526,840) and PCT (PCT/CA/95/00528) patent applications, we described DNA sequences suitable for (i) the species-specific detection and identification of 12 clinically important bacterial pathogens, (ii) the universal detection of bacteria, and (iii) the detection of 17 antibiotic resistance genes. This co-pending application described proprietary DNA sequences and DNA sequences selected from databases (in both cases, fragments of at least 100 base pairs), as well as oligonucleotide probes and amplification primers derived from these sequences. All the nucleic acid sequences described in this patent application enter the composition of diagnostic kits and methods capable of a) detecting the presence of bacteria, b) detecting specifically the presence of 12 bacterial species and 17 antibiotic resistance genes. However, these methods and kits need to be improved, since the ideal kit and method should be capable of diagnosing close to 100% of microbial pathogens and antibiotic resistance genes. For example, infections caused by *Enterococcus faecium* have become a clinical problem because of its resistance to many antibiotics. Both the detection of these bacteria and the evaluation of their resistance profiles are desirable. Besides that, novel DNA sequences (probes and primers) capable of recognizing the same and other microbial pathogens or the same and additional antibiotic resistance genes are also desirable to aim at detecting more target genes and complement our earlier patent application.

### STATEMENT OF THE INVENTION

It is an object of the present invention to provide a specific, ubiquitous and sensitive method using probes and/or amplification primers for determining the presence and/or amount of nucleic acids:
- from specific microbial species or genera selected from the group consisting of *Streptococcus* species, *Streptococcus agalactiae, Staphylococcus* species, *Staphylococcus saprophyticus, Enterococcus* species, *Enterococcus faecium, Neisseria* species, *Neisseria meningitidis, Listeria monocytogenes, Candida* species and *Candida albicans*
- from an antibiotic resistance gene selected from the group consisting of *blaₜₑₘ, bla_{rob}, blaₛₕᵥ, blaₒₓₐ, blaZ, aadB, aacC1, aacC2, aacC3, aacA4, aac6'-IIa, ermA, ermB, ermC, mecA, vanA, vanB, vanc, satA, aac(6')-aph(2"), aad(6'), vat, vga, msrA, sul* and *int,* and optionally,
- from any bacterial species
in any sample suspected of containing said nucleic acids,
wherein each of said nucleic acids or a variant or part thereof comprises a selected target region hybridizable with said probe or primers;
said method comprising the steps of contacting said sample with said probes or primers and detecting the presence and/or amount of hybridized probes or amplified products as an indication of the presence and/or amount of said any bacterial species, specific microbial species or genus and antibiotic resistance gene.

In a specific embodiment, a similar method directed to each specific microbial species or genus detection and identification, antibiotic resistance genes detection, and universal bacterial detection, separately, is provided.

In a more specific embodiment, the method makes use of DNA fragments (proprietary fragments and fragments obtained from databases), selected for their capacity to sensitively, specifically and ubiquitously detect the targeted bacterial or fungal nucleic acids.

In a particularly preferred embodiment, oligonucleotides of at least 12 nucleotides in length have been derived from the longer DNA fragments, and are used in the present method as probes or amplification primers.

The proprietary oligonucleotides (probes and primers) are also another object of the invention.

Diagnostic kits comprising probes or amplification primers for the detection of a microbial species or genus selected from the group consisting of *Streptococcus* species, *Streptococcus agalactiae, Staphylococcus* species, *Staphylococcus saprophyticus, Enterococcus* species, *Enterococcus faecium, Neisseria* species, *Neisseria meningitidis, Listeria monocytogenes, Candida* species and *Candida albicans* are also objects of the present invention.

Diagnostic kits further comprising probes or amplification primers for the detection of an antibiotic resistance gene selected from the group consisting of *blaₜₑₘ, blaᵣₒₙ blaₛₕᵥ, blaₒₓₐ, hlaZ, caadB, acrcC1, aacC2, cracC3, aacA4, aac6'-IIa, ermA, ermB, ermC, mecA, vanA, ansB, vanC, satA, aac(6)-aph(2"), aad(6'), vat, vga, msrA, sul* and *int* are also objects of this invention.

Diagnostic kits further comprising probes or amplification primers for the detection of any bacterial or fungal species, comprising or not comprising those for the detection of the specific microbial species or genus listed above, and further comprising or not comprising probes and primers for the antibiotic resistance genes listed above, are also objects of this invention.

In a preferred embodiment, such a kit allows for the separate or the simultaneous detection and identification of the above-listed microbial species or genus, antibiotic resistance genes and for the detection of any bacterium.

In the above methods and kits, amplification reactions may include a) polymerase chain reaction (PCR), b) ligase chain reaction, c) nucleic acid sequence-based amplification, d) self-sustained sequence replication, e) strand displacement amplification, f) branched DNA signal amplification, g) transcription-mediated amplification, h) cycling probe technology (CPT) i) nested PCR, or j) multiplex PCR.

In a preferred embodiment, a PCR protocol is used as an amplification reaction.

In a particularly preferred embodiment, a PCR protocol is provided, comprising, for each amplification cycle, an annealing step of 30 seconds at 45-55°C and a denaturation step of only one second at 95°C, without any time allowed specifically for the elongation step. This PCR protocol has been standardized to be suitable for PCR reactions with all selected primer pairs, which greatly facilitates the testing because each clinical sample can be tested with universal, species-specific, genus-specific and antibiotic resistance gene PCR primers under uniform cycling conditions. Furthermore, various combinations of primer pairs may be used in multiplex PCR assays.

We aim at developing a rapid test or kit to discard rapidly all the samples which are negative for bacterial cells and to subsequently detect and identify the above bacterial and/or fungal species and genera and to determine rapidly the bacterial resistance to antibiotics. Although the sequences from the selected antibiotic resistance genes are available from databases and have been used to develop DNA-based tests for their detection, our approach is unique because it represents a major improvement over current gold standard diagnostic methods based on bacterial cultures. Using an amplification method for the simultaneous bacterial detection and identification and antibiotic resistance genes detection, there is no need for culturing the clinical sample prior to testing. Moreover, a modified PCR protocol has been developed to detect all target DNA sequences in approximately one hour under uniform amplification conditions. This procedure will save lives by optimizing treatment, will diminish antibiotic resistance because less antibiotics will be prescribed, will reduce the use of broad spectrum antibiotics which are expensive, decrease overall health care costs by preventing or shortening hospitalizations, and decrease the time and costs associated with clinical laboratory testing.

In the methods and kits described herein below, the oligonucleotide probes and amplification primers have been derived from larger sequences (i.e. DNA fragments of at least 100 base pairs). All DNA fragments have been obtained either from proprietary fragments or from databases. DNA fragments selected from databases are newly used in a method of detection according to the present invention, since they have been selected for their diagnostic potential.

It is clear to the individual skilled in the art that other oligonucleotide sequences appropriate for (i) the universal bacterial detection, (ii) the detection and identification of the above microbial species or genus and (iii) the detection of antibiotic resistance genes other than those listed in Annex VI may also be derived from the proprietary fragments or selected database sequences. For example, the oligonucleotide primers or probes may be shorter or longer than the ones we have chosen; they may also be selected anywhere else in the proprietary DNA fragments or in the sequences selected from databases; they may be also variants of the same oligonucleotide. If the target DNA or a variant thereof hybridizes to a given oligonucleotide, or if the target DNA or a variant thereof can be amplified by a given oligonucleotide PCR primer pair, the converse is also true; a given target DNA may hybridize to a variant oligonucleotide probe or be amplified by a variant oligonucleotide PCR primer. Alternatively, the oligonucleotides may be designed from any DNA fragment sequences for use in amplification methods other than PCR. Consequently, the core of this invention is the identification of universal, species-specific, genus-specific and resistance gene-specific genomic or non-genomic DNA fragments which are used as a source of specific and ubiquitous oligonucleotide probes and/or amplification primers. Although the selection and evaluation of oligonucleotides suitable for diagnostic purposes requires much effort, it is quite possible for the individual skilled in the art to derive, from the selected DNA fragments, oligonucleotides other than the ones listed in Annex VI which are suitable for diagnostic purposes. When a proprietary fragment or a database sequence is selected for its specificity and ubiquity, it increases the probability that subsets thereof will also be specific and ubiquitous.

Since a high percentage of clinical specimens are negative for bacteria (Table 3), DNA fragments having a high potential for the selection of universal oligonucleotide probes or primers were selected from proprietary and database sequences. The amplification primers were selected from a gene highly conserved in bacteria and fungi, and are used to detect the presence of any bacterial pathogen in clinical specimens in order to determine rapidly (approximately one hour) whether it is positive or negative for bacteria. The selected gene, designated *tuf,* encodes a protein (EF-Tu) involved in the translational process during protein synthesis. The *tuf* gene sequence alignments used to derive the universal primers include both proprietary and database sequences (Example 1 and Annex I). This strategy allows the rapid screening of the numerous negative clinical specimens (around 80% of the specimens received, see Table 3) submitted for bacteriological testing. Tables 4, 5 and 6 provide a list of the bacterial or fungal species used to test the specificity of PCR primers and DNA probes. Table 7 gives a brief description of each species-specific, genus-specific and universal amplification assays which are objects of the present invention. Tables 8, 9 and 10 provide some relevant information about the proprietary and database sequences selected for diagnostic puposes.

### DETAILED DESCRIPTION OF THE INVENTION

### Development of species-specific, genus-specific, universal and antibiotic resistance gene-specific DNA probes and amplification primers for microorganisms Selection from databases of sequences suitable for diagnostic purposes

In order to select sequences which are suitable for species-specific or genus-specific detection and identification of bacteria or fungi or, alternatively, for the universal detection of bacteria, the database sequences (GenBank, EMBL and Swiss-Prot) were chosen based on their potential for diagnostic purposes according to sequence information and computer analysis performed with these sequences. Initially, all sequence data available for the targeted microbial species or genus were carefully analyzed. The gene sequences which appeared the most promising for diagnostic purposes based on sequence information and on sequence comparisons with the corresponding gene in other microbial species or genera performed with the Genetics Computer Group (GCG, Wisconsin) programs were selected for testing by PCR. Optimal PCR amplification primers were chosen from the selected database sequences with the help of the Oligo™ 4.0 primer analysis software (National Biosciences Inc., Plymouth, Minn.). The chosen primers were tested in PCR assays for their specificity and ubiquity for the target microbial species or genus. In general, the identification of database sequences from which amplification primers suitable for species-specific or genus-specific detection and identification were selected involved the computer analysis and PCR testing of several candidate gene sequences before obtaining a primer pair which is specific and ubiquitous for the target microbial species or genus. Annex VI provides a list of selected specific and ubiquitous PCR primer pairs. Annexes I to V and Examples 1 to 4 illustrate the strategy used to select genus-specific, species-specific and universal PCR primers from *tuf* sequences or from the *recA* gene.

### Oligonucleotide primers and probes design and synthesis

The DNA fragments sequenced by us or selected from databases (GenBank and EMBL) were used as sources of oligonucleotides for diagnostic purposes. For this strategy, an array of suitable oligonucleotide primers or probes derived from a variety of genomic DNA fragments (size of more than 100 bp) selected from databases were tested for their specificity and ubiquity in PCR and hybridization assays as described later. It is important to note that the database sequences were selected based on their potential for being species-specific, genus-specific or universal for the detection of bacteria or fungi according to available sequence information and extensive analysis and that, in general, several candidate database sequences had to be tested in order to obtain the desired specificity, ubiquity and sensitivity.

Oligonucleotide probes and amplification primers derived from species-specific fragments selected from database sequences were synthesized using an automated DNA synthesizer (Perkin-Elmer Corp., Applied Biosystems Division). Prior to synthesis, all oligonucleotides (probes for hybridization and primers for DNA amplification) were evaluated for their suitability for hybridization or DNA amplification by polymerase chain reaction (PCR) by computer analysis using standard programs (i.e. the Genetics Computer Group (GCG) programs and the primer analysis software Oligo™ 4.0). The potential suitability of the PCR primer pairs was also evaluated prior to the synthesis by verifying the absence of unwanted features such as long stretches of one nucleotide and a high proportion of G or C residues at the 3' end *(*Persing et al., 1993, Diagnostic Molecular Microbiology: Principles and Applications, American Society for Microbiology, Washington, D.C.).

The oligonucleotide primers or probes may be derived from either strand of the duplex DNA. The primers or probes may consist of the bases A, G, C, or T or analogs and they may be degenerated at one or more chosen nucleotide position(s). The primers or probes may be of any suitable length and may be selected anywhere within the DNA sequences from proprietary fragments or from selected database sequences which are suitable for (i) the universal detection of bacteria, (ii) the species-specific detection and identification of *Enterococcus faecium, Listeria monocytogenes, Neisseria meningitidis, Staphylococcus saprophyticus, Streptococcus agalactiae* and *Candida albicans* (iii) the genus-specific detection of *Streptococcus* species, *Enterococcus* species, *Staphylococcus* species and *Neisseria* species or (iv) the detection of the 26 above-mentioned clinically important antibiotic resistance genes.

Variants for a given target bacterial gene are naturally occurring and are attributable to sequence variation within that gene during evolution *(*Watson et al., 1987, Molecular Biology of the Gene, 4th ed., The Benjamin/Cummings Publishing Company, Menlo Park, CA; Lewin, 1989, Genes IV, John Wiley & Sons, New York, NY). For example, different strains of the same bacterial species may have a single or more nucleotide variation(s) at the oligonucleotide hybridization site. The person skilled in the art is well aware of the existence of variant bacterial or fungal DNA sequences for a specific gene and that the frequency of sequence variations depends on the selective pressure during evolution on a given gene product. The detection of a variant sequence for a region between two PCR primers may be demonstrated by sequencing the amplification product. In order to show the presence of sequence variants at the primer hybridization site, one has to amplify a larger DNA target with PCR primers outside that hybridization site. Sequencing of this larger fragment will allow the detection of sequence variation at this site. A similar strategy may be applied to show variants at the hybridization site of a probe. Insofar as the divergence of the target sequences or a part thereof does not affect the specificity and ubiquity of the amplification primers or probes, variant bacterial DNA is under the scope of this invention. Variants of the selected primers or probes may also be used to amplify or hybridize to a variant DNA.

### Sequencing of tuf sequences from a variety of bacterial and fungal species

The nucleotide sequence of a portion of *tuf* genes was determined for a variety of bacterial and fungal species. The amplification primers SEQ ID NOs: 107 and 108, which amplify a *tuf* gene portion of approximately 890 bp, were used for the sequencing of bacterial *tuf* sequences. The amplification primers SEQ ID NOs: 109 and 172, which amplify a *tuf gene* portion of approximately 830 bp, were used for the sequencing of fungal *tuf* sequences. Both primer pairs can amplify *tufA* and *tufB* genes. This is not surprising because these two genes are nearly identical. For example, the entire *tufA* and *tufB* genes from *E. coli* differ at only 13 nucleotide positions (Neidhardt et al., 1996, Escherichia coli and Salmonella: Cellular and Molecular Biology, 2nd ed., American Society for Microbiology Press, Washington, D.C.). These amplification primers are degenerated at several nucleotide positions and contain inosines in order to allow the amplification of a wide range of *tuf* sequences. The strategy used to select these amplification primers is similar to that illustrated in Annex I for the selection of universal primers. The amplification primers SEQ ID NOs: 107 and 108 could be used to amplify the *tuf* genes from any bacterial species. The amplification primers SEQ ID NOs: 109 and 172 could be used to amplify the *tuf* genes from any fungal species.

The *tuf* genes were amplified directly from bacterial or yeast cultures using the following amplification protocol: One µL of cell suspension was transferred directly to 19 µL of a PCR reaction mixture containing 50 mM KCl, 10 mM Tris-HCl (pH 9.0), 0.1% Triton X-100, 2.5 mM MgCl₂, 1 µM of each of the 2 primers, 200 µM of each of the four dNTPs, 0.5 unit of *Taq* DNA polymerase (Promega Corp., Madison, WI). PCR reactions were subjected to cycling using a MJ Research PTC-200 thermal cycler (MJ Research Inc., Watertown, Mass.) as follows: 3 min at 96°C followed by 30-35 cycles of 1 min at 95°C for the denaturation step, 1 min at 30-50°C for the annealing step and 1 min at 72°C for the extension step. Subsequently, twenty microliters of the PCR-amplified mixture were resolved by electrophoresis in a 1.5% agarose gel. The gel was then visualized by staining with methylene blue (Flores *et al.,* 1992, Biotechniques, **13**:203-205). The size of the amplification products was estimated by comparison with a 100-bp molecular weight ladder. The band corresponding to the specific amplification product (i.e. approximately 890 or 830 bp for bacterial or fungal *tuf* sequences, respectively) was excised from the agarose gel and purified using the QIAquick™ gel extraction kit (QIAGEN Inc., Chatsworth, CA). The gel-purified DNA fragment was then used directly in the sequencing protocol. Both strands of the *tuf* genes amplification product were sequenced by the dideoxynucleotide chain termination sequencing method by using an Applied Biosystems automated DNA sequencer (model 373A) with their PRISM™ Sequenase® Terminator Double-stranded DNA Sequencing Kit (Perkin-Elmer Corp., Applied Biosystems Division, Foster City, CA). The sequencing reactions were all performed by using the amplification primers (SEQ ID NOs: 107 to 109 and 172) and 100 ng per reaction of the gel-purified amplicon. In order to ensure that the determined sequence did not contain errors attributable to the sequencing of PCR artefacts, we have sequenced two preparations of the gel-purified *tuf* amplification product originating from two independent PCR amplifications. For all target microbial species, the sequences determined for both amplicon preparations were identical. Furthermore, the sequences of both strands were 100% complementary thereby confirming the high accuracy of the determined sequence. The *tuf* sequences determined using the above strategy are all in the Sequence Listing (i.e. SEQ ID NOs:118 to 146). Table 13 gives the originating microbial species and the source for each *tuf* sequence in the Sequence Listing.

The alignment of the *tuf* sequences determined by us or selected from databases reveals clearly that the length of the sequenced portion of the *tuf* genes is variable. There may be insertions or deletions of several amino acids. This explains why the size of the sequenced *tuf* amplification product was variable for both bacterial and fungal species. Among the *tuf* sequences determined by our group, we found insertions and deletions adding up to 5 amino acids or 15 nucleotides. Consequently, the nucleotide positions indicated on top of each of Annexes I to V do not correspond for *tuf* sequences having insertions or deletions.

It should also be noted that the various *tuf* sequences determined by us occasionally contain degenerescences. These degenerated nucleotides correspond to sequence variations between *tufA* and *tufB* genes because the amplification primers amplify both *tuf* genes. These nucleotide variations were not attributable to nucleotide misincorporations by the *taq* DNA polymerase because the sequence of both strands were identical and also because the sequences determined with both preparations of the gel-purified *tuf* amplicons were identical.

### The selection of amplifications primers from tuf sequences

The *tuf* sequences determined by us or selected from databases were used to select PCR primers for (i) the universal detection of bacteria, (ii) the genus-specific detection and identification of *Enterococcus* spp. and *Staphylococcus* spp. and (iii) the species-specific detection and identification of *Candida albicans.* The strategy used to select these PCR primers was based on the analysis of multiple sequence alignments of various *tuf* sequences. For more details about the selection of PCR primers from *tuf* sequences, please refer to Examples 1 to 3 and Annexes I to IV.

### The selection of amplification primers from recA

The comparison of the nucleotide sequence for the *recA* gene from various bacterial species including 5 species of streptococci allowed the selection of *Streptococcus-specific* PCR primers. For more details about the selection of PCR primers from *recA,* please refer to Example 4 and Annex V.

### DNA fragment isolation from Staphylococcus saprophyticus by arbitrarily primed PCR

DNA sequences of unknown coding potential for the species-specific detection and identification of *Staphylococcus saprophyticus* were obtained by the method of arbitrarily primed PCR (AP-PCR).

AP-PCR is a method which can be used to generate specific DNA probes for microorganisms (Fani *et al.,* 1993, Mol. Ecol. **2**:243-250). A description of the AP-PCR protocol used to isolate a species-specific genomic DNA fragment from *Staphylococcus saprophyticus* follows. Twenty different oligonucleotide primers of 10 nucleotides in length (all included in the AP-PCR kit OPAD (Operon Technologies, Inc., Alameda, CA)) were tested systematically with DNAs from 3 bacterial strains of *Staphylococcus saprophyticats* (all obtained from the American Type Culture Collection (ATCC): numbers 15305, 35552 and 43867) as well as with DNA from four other staphylococcal species (*Staphylococcus aureus* ATCC 25923, *Staphylococcus epidennidis* ATCC 14990, *Staphylococcus haemolyticus* ATCC 29970 and *Staphylococcus hominis* ATCC 35982). For all bacterial species, amplification was performed from a bacterial suspension adjusted to a standard 0.5 McFarland which corresponds to approximately 1.5 x 10⁸ bacteria/mL. One µL of the standardized bacterial suspension was transferred directly to 19 µL of a PCR reaction mixture containing 50 mM KCl, 10 mM Tris-HCl (pH 9.0), 0.1% Triton X-100, 2.5 mM MgCl₂, 1.2 µM of only one of the 20 different AP-PCR primers OPAD, 200 µM of each of the four dNTPs and 0.5 unit of *Taq* DNA polymerase (Promega Corp., Madison, WI). PCR reactions were subjected to cycling using a MJ Research PTC-200 thermal cycler (MJ Research Inc.) as follows: 3 min at 96°C followed by 35 cycles of 1 min at 95°C for the denaturation step, 1 min at 32°C for the annealing step and 1 min at 72°C for the extension step. A final extension step of 7 min at 72°C was made after the 35 cycles to ensure complete extension of PCR products. Subsequently, twenty microliters of the PCR amplified mixture were resolved by electrophoresis in a 2% agarose gel containing 0.25 µg/mL of ethidium bromide. The size of the amplification products was estimated by comparison with a 50-bp molecular weight ladder.

Amplification patterns specific for *Staphylococcus saprophyticus* were observed with the AP-PCR primer OPAD-9 (SEQ ID NO: 25). Amplification with this primer consistently showed a band corresponding to a DNA fragment of approximately 450 bp for all *Staphylococcus saprophyticus* strains tested but not for any of the four other staphylococcal species tested. This species-specific pattern was confirmed by testing 10 more clinical isolates of *S. saprophyticus* selected from the culture collection of the microbiology laboratory of the CHUL as well as strains selected from the gram-positive bacterial species listed in Table 5.

The band corresponding to the approximately 450 bp amplicon which was specific and ubiquitous for *S*. *saprophyticus* based on AP-PCR was excised from the agarose gel and purified using the QIAquick™ gel extraction kit (QIAGEN Inc.). The gel-purified DNA fragment was cloned into the T/A cloning site of the pCR 2.1™ plasmid vector (Invitrogen Inc.) using T4 DNA ligase (New England BioLabs). Recombinant plasmids were transformed into *E. coli* DH5α competent cells using standard procedures. Plasmid DNA isolation was done by the method of Birnboim and Doly (Nucleic Acids Res. 7:1513-1523) for small-scale preparations. All plasmid DNA preparations were digested with the *EcoRI* restriction endonuclease to ensure the presence of the approximately 450 bp AP-PCR insert into the recombinant plasmids. Subsequently, a large-scale and highly purified plasmid DNA preparation was performed from two selected clones shown to carry the AP-PCR insert by using the QIAGEN plasmid purification kit. These plasmid preparations were used for automated DNA sequencing.

Both strands of the AP-PCR insert from the two selected clones were sequenced by the dideoxynucleotide chain termination sequencing method with SP6 and T7 sequencing primers, by using an Applied Biosystems automated DNA sequencer as described previously. The analysis of the obtained sequences revealed that the DNA sequences for both strands from each clone were 100% complementary. Furthermore, it showed that the entire sequence determined for each clone were both identical. These sequencing data confirm the 100% accuracy for the determined 438 bp sequence (SEQ ID NO: 29). Optimal amplification primers have been selected from the sequenced AP-PCR *Staphylococcus saprophyticus* DNA fragment with the help of the primer analysis software oligo™ 4.0. The selected primer sequences have been tested in PCR assays to verify their specificity and ubiquity (Table 7). These PCR primers were specific since there was no amplification with DNA from bacterial species other than *S. saprophyticus* selected from Tables 4 and 5. Furthermore, this assay was ubiquitous since 245 of 260 strains of *S. saprophylicus* were efficiently amplified with this PCR assay. When used in combination with another *S. saprophyticus-specific* PCR assay, which is an object of our co-pending U.S. (N.S. 08/526,840) and PCT (PCT/CA/95/00528) patent applications, the ubiquity reaches 100% for these 260 strains.

### DNA amplification

For DNA amplification by the widely used PCR (polymerase chain reaction) method, primer pairs were derived from proprietary DNA fragments or from database sequences. Prior to synthesis, the potential primer pairs were analyzed by using the Oligo™ 4.0 software to verify that they are good candidates for PCR amplification.

During DNA amplification by PCR, two oligonucleotide primers binding respectively to each strand of the heat-denatured target DNA from the bacterial genome are used to amplify exponentially *in vitro* the target DNA by successive thermal cycles allowing denaturation of the DNA, annealing of the primers and synthesis of new targets at each cycle (Persing et al, 1993, Diagnostic Molecular Microbiology: Principles and Applications, American Society for Microbiology, Washington, D.C.).

Briefly, the PCR protocols were as follow: Treated clinical specimens or standardized bacterial or fungal suspensions (see below) were amplified in a 20 µL PCR reaction mixture containing 50 mM KCI, 10 mM Tris-HCl (pH 9.0), 2.5 mM MgCl₂, 0.4 µM of each primer, 200 µM of each of the four dNTPs and 0.5 unit of *Taq* DNA polymerase (Promega) combined with the TaqStart™ antibody (Clontech Laboratories Inc., Palo Alto, CA). The TaqStart™ antibody, which is a neutralizing monoclonal antibody to *Taq* DNA polymerase, was added to all PCR reactions to enhance the specificity and the sensitivity of the amplifications (Kellogg et al., 1994, Biotechniques 16:1134-1137). The treatment of the clinical specimens varies with the type of specimen tested, since the composition and the sensitivity level required are different for each specimen type. It consists in a rapid protocol to lyse the bacterial cells and eliminate the PCR inhibitory effects (see example 11 for urine specimen preparation). For amplification from bacterial or fungal cultures, the samples were added directly to the PCR amplification mixture without any pre-treatment step (see example 10). Primer sequences derived from highly conserved regions of the bacterial 16S ribosomal RNA gene were used to provide an internal control for all PCR reactions. Alternatively, the internal control was derived from sequences not found in microorganisms or in the human genome. The internal control was integrated into all amplification reactions to verify the efficiency of the PCR assays and to ensure that significant PCR inhibition was absent. The internal control derived from rRNA was also useful to monitor the efficiency of bacterial lysis protocols.

PCR reactions were then subjected to thermal cycling (3 min at 95°C followed by 30 cycles of 1 second at 95°C for the denaturation step and 30 second at 55°C for the annealing-extension step) using a PTC-200 thermal cycler (MJ Research Inc.) and subsequently analyzed by standard ethidium bromide-stained agarose gel electrophoresis. The number of cycles performed for the PCR assays varies according to the sensitivity level required. For example, the sensitivity level required for microbial detection directly from clinical specimens is higher for blood specimens than for urine specimens because the concentration of microorganisms associated with a septicemia can be much lower than that associated with a urinary tract infection. Consequently, more sensitive PCR assays having more thermal cycles are required for direct detection from blood specimens. Similarly, PCR assays performed directly from bacterial or fungal cultures may be less sensitive than PCR assays performed directly from clinical specimens because the number of target organisms is normally much lower in clinical specimens than in microbial cultures.

It is clear that other methods for the detection of specific amplification products, which may be faster and more practical for routine diagnosis, may be used. Such methods may be based on the detection of fluorescence after amplification (e.g. TaqMan™ system from Perkin Elmer or Amplisensor™ from Biotronics). Methods based on the detection of fluorescence are particularly promising for utilization in routine diagnosis as they are very rapid, quantitative and can be automated (Example 14).

Microbial pathogens detection and identification may also be performed by solid support or liquid hybridization using species-specific internal DNA probes hybridizing to an amplification product. Such probes may be generated from any species-specific or genus-specific DNA amplification products which are objects of the present invention. Alternatively, the internal probes for species or genus detection and identification may be derived from the amplicons produced by the universal amplification assay. The oligonucleotide probes may be labeled with biotin or with digoxigenin or with any other reporter molecules.

To assure PCR efficiency, glycerol, dimethyl sulfoxide (DMSO) or other related solvents can be used to increase the sensitivity of the PCR and to overcome problems associated with the amplification of a target DNA having a high GC content or forming strong secondary structures (Dieffenbach and Dveksler, 1995, PCR Primer : A Laboratory Manual, Cold Spring Harbor Laboratory Press, Plainview, New York). The concentration ranges for glycerol and DMSO are 5-15% (v/v) and 3-10% (v/v), respectively. For the PCR reaction mixture, the concentration ranges for the amplification primers and MgCl₂ are 0.1-1.5 µM and 1.5-3.5 mM, respectively. Modifications of the standard PCR protocol using external and nested primers (i.e. nested PCR) or using more than one primer pair (i.e. multiplex PCR) may also be used (Persing et al., 1993, Diagnostic Molecular Microbiology: Principles and Applications, American Society for Microbiology, Washington, D.C.). For more details about the PCR protocols and amplicon detection methods, see Examples 9 to 14.

The person skilled in the art of DNA amplification knows the existence of other rapid amplification procedures such as ligase chain reaction (LCR), transcription-mediated amplification (TMA), self sustained sequence replication (3SR), nucleic acid sequence-based amplification (NASBA), strand displacement amplification (SDA), branched DNA (bDNA) and cycling probe technology (CPT) (Lee et al., 1997, Nucleic Acid Amplification Technologies: Application to Disease Diagnosis, Eaton Publishing, Boston, MA ; Persing et al., 1993, Diagnostic Molecular Microbiology: Principles and Applications, American Society for Microbiology, Washington, D.C.). The scope of this invention is not limited to the use of amplification by PCR, but rather includes the use of any rapid nucleic acid amplification method or any other procedure which may be used to increase rapidity and sensitivity of the tests. Any oligonucleotide suitable for the amplification of nucleic acids by approaches other than PCR and derived from the species-specific, genus-specific and universal DNA fragments as well as from selected antibiotic resistance gene sequences included in this document are also under the scope of this invention.

### Hybridization assays with oligonucleotide probes

In hybridization experiments, single-stranded oligonucleotides (size less than 100 nucleotides) have some advantages over DNA fragment probes for the detection of bacteria, such as ease of synthesis in large quantities, consistency in results from batch to batch and chemical stability. Briefly, for the hybridizations, oligonucleotides were 5' end-labeled with the radionucleotide Y-³²p(dATP) using T4 polynucleotide kinase (Pharmacia) (Sambrook et al., 1989, Molecular Cloning: A Laboratory Manual, 2nd ed., Cold Spring Harbor Laboratory Press, Cold Spring Harbor, NY). The unincorporated radionucleotide was removed by passing the labeled oligonucleotide through a Sephadex G-50™ column. Alternatively, oligonucleotides were labeled with biotin, either enzymatically at their 3' ends or incorporated directly during synthesis at their 5' ends, or with digoxigenin. It will be appreciated by the person skilled in the art that labeling means other than the three above labels may be used.

Each oligonucleotide probe was then tested for its specificity by hybridization to DNAs from a variety of bacterial and fungal species selected from Tables 4, 5 and 6. All of the bacterial or fungal species tested were likely to be pathogens associated with common infections or potential contaminants which can be isolated from clinical specimens. Each target DNA was released from bacterial cells using standard chemical treatments to lyse the cells (Sambrook e.t al., 1989, Molecular Cloning: A Laboratory Manual, 2 nd ed., Cold Spring Harbor Laboratory Press, Cold Spring Harbor, NY). Subsequently, the DNA was denatured by conventional methods and then irreversibly fixed onto a solid support (e.g. nylon or nitrocellulose membranes) or free in solution. The fixed single-stranded target DNAs were then hybridized with the oligonucleotide probe cells (Sambrook et al., 1989, Molecular Cloning: A Laboratory Manual, 2 nd ed., Cold Spring Harbor Laboratory Press, Cold Spring Harbor, NY). Pre-hybridization conditions were in 1 M NaCl + 10% dextran sulfate +1% SDS + 100 µg/mL salmon sperm DNA at 65°C for 15 min. Hybridization was performed in fresh pre-hybridization solution containing the labeled probe at 65°C overnight. Post-hybridization washing conditions were as follows: twice in 3X SSC containing 1% SDS, twice in 2X SSC containing 1% SDS and twice in 1X SSC containing 1% SDS (all of these washes were at 65°C for 15 min), and a final wash in 0.1X SSC containing 1% SDS at 25°C for 15 min. Autoradiography of washed filters allowed the detection of selectively hybridized probes. Hybridization of the probe to a specific target DNA indicated a high degree of similarity between the nucleotide sequence of these two DNAs because of the high stringency of the washes.

An oligonucleotide probe was considered specific only when it hybridized solely to DNA from the species or genus from which it was isolated. Oligonucleotide probes found to be specific were subsequently tested for their ubiquity (i.e. ubiquitous probes recognized most or all isolates of the target species or genus) by hybridization to microbial DNAs from clinical isolates of the species or genus of interest including ATCC strains. The DNAs from strains of the target species or genus were denatured, fixed onto nylon membranes and hybridized as described above. Probes were considered ubiquitous when they hybridized specifically with the DNA from at least 80% of the isolates of the target species or genus.

### Specificity and ubiquity tests for oligonucleotide primers and probes

The specificity of oligonucleotide primers and probes, derived either from the DNA fragments sequenced by us or selected from databases, was tested by amplification of DNA or by hybridization with bacterial or fungal species selected from those listed in Tables 4, 5 and 6, as described in the two previous sections. Oligonucleotides found to be specific were subsequently tested for their ubiquity by amplification (for primers) or by hybridization (for probes) with bacterial DNAs from isolates of the target species or genus. Results for specificity and ubiquity tests with the oligonucleotide primers are summarized in Table 7. The specificity and ubiquity of the PCR assays using the selected amplification primer pairs were tested directly from cultures (see Examples 9 and 10) of bacterial or fungal species.

The various species-specific and genus-specific PCR assays which are objects of the present invention are all specific. For the PCR assays specific to bacterial species or genus, this means that DNA isolated from a wide variety of bacterial species, other than that from the target species or genus and selected from Tables 4 and 5, could not be amplified. For the PCR assay specific to *Candida albicans,* it means there was no amplification with genomic DNA from the fungal species listed in Table 6 as well as with a variety of bacterial species selected from Tables 4 and 5.

The various species-specific and genus-specific PCR assays which are objects of the present invention are also all ubiquitous (Table 7). (i) The species-specific PCR assays for *E. faecium, L. monocytogenes, S. saprophyticus, S. agalactiae* and *C*. *albicans* amplified genomic DNA from all or most strains of the target species tested, which were obtained from various sources and which are representative of the diversity within each target species (Table7). The species identification of all of these strains was based on classical biochemical methods which are routinely used in clinical microbiology laboratories. (ii) The genus-specific PCR assays specific for *Enterococcus* spp., *Staphylococcus* spp., *Streptococcus* spp. and *Neisseria* spp. amplified genomic DNA from all or most strains of the target genus tested, which represent all clinically important bacterial species for each target genus. These strains were obtained from various sources and are representative of the diversity within each target genus. Again, the species identification of all of these strains was based on classical biochemical methods which are routinely used in clinical microbiology laboratories. More specifically, the four genus-specific PCR assays amplified the following species: (1) The *Enterococcus-specific* assay amplified efficiently DNA from all of the 11 enterococcal species tested including *E. avium, E. casseliflavus, E. dispar, E. durans, E. faecalis, E. faecium, E. flavescens, E. gallinarum, E. hirae, E. mundtii* and *E*. *raffinosus.* (2) The *Neisseria*-specific assay amplified efficiently DNA from all of the 12 neisserial species tested including *N. canis, N. cinerea, N. elongata, N. flavescens, N. gonorrlioeae, N. lactamica, N. meningitidis, N. mucosa, N. polysaccharea, N. sicca, N. subflava* and *N. weaveri.* (3) The *Staphylococcus-specific* assay amplified efficiently DNA from 13 of the 14 staphylococcal species tested including *S. aureus, S. auricularis, S. capitis, S. cohnii, S. epidermidis, S. haemolyticus, S. hominis, S. lugdunensis, S. saprophyticus, S. sclaleiferi, S. simulaus, S. warned* and S. *xylosus.* The staphylococcal species which could not be amplified is *S*. *sciuri.* (4) Finally, the *Streptococcus-specific* assay amplified efficiently DNA from all of the 22 streptococcal species tested including *S. agalactiae, S. anginosus, S. bovis, S. constellatus, S. crista, S. dysgalactiae, S. equi, S. gordonii, S. intermedius, S. milis, S. mutans, S. oralis, S. parasanguis, S. pneumoniae, S. pyogenes, S. salivariu,s, S. sanguis, S. sabrinus, S. suis, S. uberis, S. vestibularis* and *S*. *viridans.* On the other hand, the *Streptococcus-specific* assay did not amplify 3 out of 9 strains of *S*. *mutans* and 1 out of 23 strains of *S*. *salivarius,* thereby showing a slight lack of ubiquity for these two streptococcal species.

All specific and ubiquitous amplification primers for each target microbial species or genus or antibiotic resistance gene investigated are listed in Annex VI. Divergence in the sequenced DNA fragments can occur, insofar as the divergence of these sequences or a part thereof does not affect the specificity of the probes or amplification primers. Variant bacterial DNA is under the scope of this invention.

The PCR amplification primers listed in Annex VI were all tested for their specificity and ubiquity using reference strains as well as clinical isolates from various geographical locations. The 351 reference strains used to test the amplification and hybridization assays (Tables 4, 5 and 6) were obtained from (i) the American Type Culture Collection (ATCC): 85%, (ii) the Laboratoire de santé publique du Québec (LSPQ): 10%, (iii) the Centers for Disease Control and Prevention (CDC): 3% , (iv) the National Culture Type Collection (NCTC): 1% and (v) several other reference laboratories throughout the world: 1%. These reference strains are representative of (i) 90 gram-negative bacterial species (169 strains; Table 4), (ii) 97 gram-positive bacterial species (154 strains; Table 5) and (iii) 12 fungal species (28 strains; Table 6).

### Antibiotic resistance genes

Antimicrobial resistance complicates treatment and often leads to therapeutic failures. Furthermore, overuse of antibiotics inevitably leads to the emergence of bacterial resistance. Our goal is to provide clinicians, in approximately one hour, the needed information to prescribe optimal treatments. Besides the rapid identification of negative clinical specimens with DNA-based tests for universal bacterial detection and the identification of the presence of a specific pathogen in the positive specimens with species-and/or genus-specific DNA-based tests, clinicians also need timely information about the ability of the bacterial pathogen to resist antibiotic treatments. We feel that the most efficient strategy to evaluate rapidly bacterial resistance to antimicrobials is to detect directly from the clinical specimens the most common and clinically important antibiotic resistance genes (i.e. DNA-based tests for the detection of antibiotic resistance genes). Since the sequence from the most important and common bacterial antibiotic resistance genes are available from databases, our strategy was to use the sequence from a portion or from the entire resistance gene to design specific oligonucleotide primers or probes which will be used as a basis for the development of rapid DNA-based tests. The sequence from each of the bacterial antibiotic resistance genes selected on the basis of their clinical relevance (i.e. high incidence and importance) is given in the Sequence Listing. Tables 9 and 10 summarize some characteristics of the selected antibiotic resistance genes. Our approach is unique because the antibiotic resistance genes detection and the bacterial detection and identification are performed simultaneously in multiplex assays under uniform PCR amplification conditions (Example 13).

Annex VI provides a list of all amplification primers selected from 26 clinically important antibiotic resistance genes which were tested in PCR assays. The various PCR assays for antibiotic resistance genes detection and identification were validated by testing several resistant bacterial isolates known to carry the targeted gene and obtained from various countries. The testing of a large number of strains which do not carry the targeted resistance gene was also performed to ensure that all assays were specific. So far, all PCR assays for antibiotic resistance genes are highly specific and have detected all control resistant bacterial strains known to carry the targeted gene. The results of some clinical studies to validate the array of PCR assays for the detection and identification of antibiotic resistance genes and correlate these DNA-based assays with standard antimicrobials susceptibility testing methods are presented in Tables 11 and 12.

### Universal bacterial detection

In the routine microbiology laboratory, a high percentage of clinical specimens sent for bacterial identification are negative by culture (Table 4). Testing clinical samples with universal amplification primers or universal probes to detect the presence of bacteria prior to specific identification and screen out the numerous negative specimens is thus useful as it saves costs and may rapidly orient the clinical management of the patients. Several amplification primers and probes were therefore synthesized from highly conserved portions of bacterial sequences from the *tuf* genes (Table 8). The universal primer selection was based on a multiple sequence alignment constructed with sequences determined by us or selected from available database sequences as described in Example 1 and Annex I.

For the identification of database sequences suitable for the universal detection of bacteria, we took advantage of the fact that the complete genome sequences for two distant microorganisms (i.e. *Mycoplasma genitalium* and *Haemophilus influenzae*) are available. A comparison of the amino acid sequence for all proteins encoded by the genome of these two distant microorganisms led to the identification of highly homologous proteins. An analysis of these homologous proteins allowed to select some promising candidates for the development of universal DNA-based assays for the detection of bacteria. Since the complete nucleotide sequence of several other microbial genomes are presently available in databases, a person skilled in the art could arrive to the same conclusions by comparing genomes sequences other than those of *Mycoplasma genitalium* and *Haemophilus influenzae.* The selected *tuf* gene encodes a protein (EF-Tu) involved in the translation process during protein synthesis. Subsequently, an extensive nucleotide sequence analysis was performed with the *tuf* gene sequences available in databases as well as with novel *tuf* sequences which we have determined as described previously. All computer analysis of amino acid and nucleotide sequences were performed by using the GCG programs. Subsequently, optimal PCR primers for the universal amplification of bacteria were selected with the help of the Oligo™ program. The selected primers are degenerated at several nucleotide positions and contain several inosines in order to allow the amplification of all clinically relevant bacterial species (Annex I). Inosine is a nucleotide analog able to specifically bind to any of the four nucleotides A, C, G or T. Degenerated oligonucleotides consist of an oligonucleotide mix having two or more of the four nucleotides A, C, G or T at the site of mismatches. The inclusion of inosine and/or of degenerescences in the amplification primers allow mismatch tolerance thereby permitting the amplification of a wider array of target nucleotide sequences (Dieffenbach and Dveksler, 1995 PCR Primer: A Laboratory Manual, Cold Spring Harbor Laboratory Press, Plainview, NY).

The amplification conditions with the universal primers were identical to those used for the species- and genus-specific amplification assays except that the annealing temperature was 50°C instead of 55°C. This universal PCR assay was specific and nearly ubiquitous for the detection of bacteria. The specificity for bacteria was verified by amplifying genomic DNA isolated from the 12 fungal species listed in Table 6 as well as genomic DNA from *Leishmania donovarani, Saccharomyces cerevisiae* and human lymphocytes. None of the above eukaryotic DNA preparations could be amplified by the universal assay, thereby suggesting that this test is specific for bacteria. The ubiquity of the universal assay was verified by amplifying genomic DNAs from 116 reference strains which represent 95 of the most clinically relevant bacterial species. These species have been selected from the bacterial species listed in Tables 4 and 5. We found that 104 of these 116 strains could be amplified. The bacterial species which could not be amplified belong to the following genera: *Corynebacterium* (11 species) and *Stenotrophomonas* (1 species). Sequencing of the *tuf* genes from these bacterial species has been recently performed. This sequencing data has been used to select new universal primers which may be more ubiquitous. These primers are in the process of being tested. We also observed that for several species the annealing temperature had to be reduced to 45°C in order to get an efficient amplification. These bacterial species include *Gernella morbilbrum, Listeria* spp. (3 species) and *Gardnerella vaginalis.* It is important to note that the 95 bacterial species selected from Tables 4 and 5 to test the ubiquity of the universal assay include all of the most clinically relevant bacterial species associated with a variety of human infections acquired in the community or in hospitals (nosocomial infections). The most clinically important bacterial and fungal pathogens are listed in Tables 1 and 2.

### EXAMPLES AND ANNEXES

The following examples and annexes are intended to be illustrative of the various methods and compounds of the invention, rather than limiting the scope thereof.

The various annexes show the strategies used for the selection of amplification primers from *tasf* sequences or from the *recA* gene: (i) Annex I illustrates the strategy used for the selection of the universal amplification primers from *luf* sequences. (ii) Annex II shows the strategy used for the selection of the amplification primers specific for the genus *Enterococcus* from *tuf* sequences. (iii) Annex III illustrates the strategy used for the selection of the amplification primers specific for the genus *Staphylococcus* from *tuf* sequences. (iv) Annex IV shows the strategy used for the selection of the amplification primers specific for the species *Candida albicans* from *tuf* sequences. (v) Annex V illustrates the strategy used for the selection of the amplification primers specific for the genus *Streptococcus* from *recA* sequences. (vi) Annex VI gives a list of all selected primer pairs. As shown in these annexes, the selected amplification primers may contain inosines and/or degenerescences. Inosine is a nucleotide analog able to specifically bind to any of the four nucleotides A, C, G or T. Alternatively, degenerated oligonucleotides which consist of an oligonucleotide mix having two or more of the four nucleotides A, C, G or T at the site of mismatches were used. The inclusion of inosine and/or of degenerescences in the amplification primers allow mismatch tolerance thereby permitting the amplification of a wider array of target nucleotide sequences (Dieffenbach and Dveksler, 1995 PCR Primer: A Laboratory Manual, Cold Spring Harbor Laboratory Press, Plainview, New York).

### EXAMPLES

### EXAMPLE 1 :

Selection of universal PCR primers from tuf sequences. As shown in Annex I, the comparison of *tuf* sequences from a variety of bacterial and eukaryotic species allowed the selection of PCR primers which are universal for the detection of bacteria. The strategy used to design the PCR primers was based on the analysis of a multiple sequence alignment of various *tuf* sequences. This multiple sequence alignment includes *tuf* sequences from 38 bacterial species and 3 eukaryotic species either determined by us or selected from databases (Table 13). A careful analysis of this multiple sequence alignment allowed the selection of primer sequences which are conserved within cubacteria but which discriminate sequences from eukaryotes, thereby permitting the universal detection of bacteria. As shown in Annex I, the selected primers contain several inosines and degenerescences. This was necessary because there is a relatively high polymorphism among bacterial *tuf* sequences despite the fact that this gene is highly conserved. In fact, among the *tuf* sequences that we determined, we found many nucleotide variations as well as some deletions and/or insertions of amino acids. The selected universal primers were specific and ubiquitous for bacteria (Table 7). Of the 95 most clinically important bacterial species tested, 12 were not amplified. These species belong to the genera *Corynebacterium* (11 species) and *Stenotrophomonas* (1 species). The universal primers did not amplify DNA of non-bacterial origin, including human and other types of eukaryotic DNA.

### EXAMPLE 2 :

Selection of genus-specific PCR primers from *tuf* sequences. As shown in Annexes 2 and 3, the comparison of *tuf* sequences from a variety of bacterial species allowed the selection of PCR primers specific for *Enterococcus* spp. or for *Staphylococcus* spp. The strategy used to design the PCR primers was based on the analysis of a multiple sequence alignment of various *tuf* sequences. These multiple sequence alignments include the *tuf* sequences of four representative bacterial species selected from each target genus as well as *tuf* sequences from species of other closely related bacterial genera. A careful analysis of those alignments allowed the selection of oligonucleotide sequences which are conserved within the target genus but which discriminate sequences from other closely related genera, thereby permitting the genus-specific and ubiquitous detection and identification of the target bacterial genus.

For the selection of primers specific for *Enterococcus* spp. (Annex II), we have sequenced a portion of approximately 890 bp of the *tuf* genes for *Enterococcus avium, E. faecalis, E. faecium* and *E. gallinarum.* All other *tuf* sequences used in the alignment were either sequenced by us or selected from databases. The analysis of this sequence alignment led to the selection of a primer pair specific and ubiquitous for *Enterococcus* spp. (Table 7). All of the 11 enterococcal species tested were e-fficiently amplified and there was no amplification with genomic DNA from bacterial species of other genera.

For the selection of primers specific for *Staphylococcus* spp. (Annex III), we have also sequenced a portion of approximately 890 bp of the *tuf* genes for *Staphylococcus aurears, S. epidermidis, S. saprophyticus* and *S*. *simulans.* All other *tuf* sequences used in the alignment were either sequenced by us or selected from databases. The analysis of this sequence alignment led to the selection of two primer pairs specific and ubiquitous for *Staphylococcus* spp. (Table 7). Annex III shows the strategy used to select one of these two PCR primer pairs. The same strategy was used to select the other primer pair. Of the 14 staphylococcal species tested, one *(S. sciuri)* could not be amplified by the *Staphylococcaes-*specific PCR assays using either one of these two primer pairs. For PCR assays using either one of these two primer pairs, there was no amplification with DNA from species of other bacterial genera.

### EXAMPLE 3 :

Selection from *tuf* sequences of PCR primers specific for *Candida albicans.* As shown in Annex IV, the comparison of *tuf* sequences from a variety of bacterial and eukaryotic species allowed the selection of PCR primers specific for *Candida albicans.* The strategy used to design the PCR primers was based on the analysis of a multiple sequence alignment of various *tuf* sequences. This multiple sequence alignment includes *tuf* sequences of five representative fungal species selected from the genus *Candida* which were determined by our group (i.e. *C. albicans, C. glabrata, C. krusei, C. parapsilosis* and C. *tropicalis)* as well as *tuf* sequences from other closely related fungal species. *tuf* sequences from various bacterial species were also included. A careful analysis of this sequence alignment allowed the selection of primers from the *C*. *albicans tuf* sequence; these primers discriminate sequences from other closely related *Candida* species and other fungal species, thereby permitting the species-specific and ubiquitous detection and identification of C. *albicans* (Table 7). All of 88 *Candida albicans* strains tested were efficiently amplified and there was no amplification with genomic DNA from other fungal or bacterial species.

### EXAMPLE 4:

Selection of PCR primers specific for *Streptococcus* from *recA.* As shown in Annex V, the comparison of the various bacterial *recA* gene sequences available from databases (GenBank and EMBL) was used as a basis for the selection of PCR primers which are specific and ubiquitous for the bacterial genus *Streptococcus.* Since sequences of the *recA* gene are available for many bacterial species including five species of streptococci, it was possible to choose sequences well conserved within the genus *Streptococcus* but distinct from the *r-ecA* sequences for other bacterial genera. When there were mismatches between the *recA* gene sequences from the five *Streptococcus* species, an inosine residue was incorporated into the primer (Annex V). The selected primers, each containing one inosine and no degenerescence, were specific and ubiquitous for *Streptococcus* species (Table 7). This PCR assay amplified all of the 22 streptococcal species tested. However, the *Streptococcus-specific* assay did not amplify DNA from 3 out of 9 strains of *S. mutants* and 1 out of 3 strains of *S. salivarius.* There was no amplification with genomic DNA from other bacterial genera (Table 7).

### EXAMPLE 5:

Nucleotide sequencing of DNA fragments. The nucleotide sequence of a portion of the *tuf* genes from a variety of bacterial or fungal species was determined by using the dideoxynucleotide chain termination sequencing method (Sanger et al., 1977, Proc. Natl. Acad. Sci. USA. 74:5463-5467). The sequencing was performed by using an Applied Biosystems automated DNA sequencer (model 373A) with their PRISM™ Sequenase@ Terminator Double-stranded DNA Sequencing Kit (Perkin-Elmer Corp., Applied Biosystems Division, Foster City, CA). The sequencing strategy does not discriminate *tufA* and *tufB* genes because the sequencing primers hybridize efficiently to both bacterial *tuf* genes. These DNA sequences are shown in the sequence listing (SEQ ID Nos: 118 to 146). The presence of several degenerated nucleotides in the various *tuf* sequences determined by our group (Table 13) corresponds to sequence variations between *tufA* and *tufB.*

Oligonucleotide primers and probes selection. Oligonucleotide probes and amplification primers were selected from the given proprietary DNA fragments or database sequences using the Oligo™ program and were synthesized with an automated ABI DNA synthesizer (Model 391, Perkin-Elmer Corp., Applied Biosystems Division) using phosphoramidite chemistry.

### EXAMPLE 6 :

Labeling of oligonucleotides for hybridization assays. Each oligonucleotide was 5' end-labeled with y-³²P (dATP) by the T4 polynucleotide kinase (Pharmacia) as described earlier. The label could also be non-radioactive.

Specificity test for oligonucleotide probes. All labeled oligonucleotide probes were tested for their specificity by hybridization to DNAs from a variety of bacterial and fungal species selected from Tables 4, 5 and 6 as described earlier. Species-specific or genus-specific probes were those hybridizing only to DNA from the microbial species or genus from which it was isolated. Oligonucleotide probes found to be specific were submitted to ubiquity tests as follows.

Ubiquity test for oligonucleotide probes. Specific oligonucleotide probes were then used in ubiquity tests with strains of the target species or genus including reference strains and other strains obtained from various countries and which are representative of the diversity within each target species or genus. Chromosomal DNAs from the isolates were transferred onto nylon membranes and hybridized with labeled oligonucleotide probes as described for specificity tests. The batteries of isolates constructed for each target species or genus contain reference ATCC strains as well as a variety of clinical isolates obtained from various sources. Ubiquitous probes were those hybridizing to at least 80% of DNAs from the battery of clinical isolates of the target species or genus.

### EXAMPLE 7:

Same as example 6 except that a pool of specific oligonucleotide probes is used for microbial identification (i) to increase sensitivity and assure 100% ubiquity or (ii) to identify simultaneously more than one microbial species and/or genus. Microbial identification could be performed from microbial cultures or directly from any clinical specimen.

### EXAMPLE 8:

Same as example 6 except that bacteria or fungi were detected directly from clinical samples. Any biological sample was loaded directly onto a dot blot apparatus and cells were lysed *in situ* for bacterial or fungal detection and identification. Blood samples should be heparizined in order to avoid coagulation interfering with their convenient loading on a dot blot apparatus.

### EXAMPLE 9:

PCR amplification. The technique of PCR was used to increase the sensitivity and the rapidity of the assays. The sets of primers were tested in PCR assays performed directly from bacterial colonies or from a standardized bacterial suspension (see Example 10) to determine their specificity and ubiquity (Table 7). Examples of specific and ubiquitous PCR primer pairs are listed in Annex VI.

Specificity and ubiquity tests for amplification primers. The specificity of all selected PCR primer pairs was tested against DNAs from a variety of bacterial and fungal species selected from Tables 4, 5 and 6 as described earlier. Primer pairs found specific for each species or genus were then tested for their ubiquity to ensure that each set of primers could amplify at least 90% of DNAs from a battery of isolates of the target species or genus. The batteries of isolates constructed for each species contain reference ATCC strains and various clinical isolates from around the world which are representative of the diversity within each species or genus.

Standard precautions to avoid false positive PCR results should be taken (Kwok and Higuchi, 1989, Nature, 239:237-238). Methods to inactivate PCR amplification products such as the inactivation by uracil-N-glycosylase may be used to control PCR carryover.

### EXAMPLE 10:

Amplification directly from bacterial or yeast cultures. PCR assays were performed either directly from a bacterial colony or from a bacterial suspension, the latter being adjusted to a standard McFarland 0.5 (corresponds to approximately 1.5 x 10⁸ bacteria/mL). In the case of direct amplification from a colony, a portion of a colony was transferred using a plastic rod directly into a 20 µL PCR reaction mixture containing 50 mM KCl, 10 mM Tris-HCl (pH 9.0), 0.1% Triton X-100, 2.5 mM MgCl₂, 0.4 µM of each primer, 200 µM of each of the four dNTPs and 0.5 unit of *Taq* DNA polymerase (Promega) combined with the TaqStart™ antibody (Clontech Laboratories Inc.). For the bacterial suspension, 1 µL of the cell suspension was added to 19 µL of the same PCR reaction mixture. For the identification from yeast cultures, 1 µL of a standard McFarland 1.0 (corresponds to approximately 3.0 x 10⁸ bacteria/mL) concentrated 100 times by centrifugation was added directly to the PCR reaction. This concentration step for yeast cells was performed because a McFarland 0.5 for yeast cells has approximately 200 times fewer cells than a McFarland 0.5 for bacterial cells.

PCR reactions were then subjected to thermal cycling (3 min at 95°C followed by 30 cycles of 1 second at 95°C for the denaturation step and 30 seconds at 55°C for the annealing-extension step) using a PTC-200 thermal cycler. PCR amplification products were then analyzed by standard agarose gel (2%) electrophoresis. Amplification products were visualized in agarose gels containing 0.25 µg/mL of ethidium bromide under UV at 254 nm. The entire PCR assay can be completed in approximately one hour.

Primer sequences derived from highly conserved regions of the bacterial 16S ribosomal RNA gene were used to provide an internal control for all PCR reactions. Alternatively, the internal control was derived from sequences not found in microorganisms or in the human genome. The internal control was integrated into all amplification reactions to verify the efficiency of the PCR assays and to ensure that significant PCR inhibition was absent. The internal control derived from rRNA was also useful to monitor the efficiency of the bacterial lysis protocols. The internal control and the species-specific or genus-specific amplifications were performed simultaneously in multiplex PCR assays.

### EXAMPLE 11:

Amplification directly from urine specimens. For PCR amplification performed directly from urine specimens, 1 µL of urine was mixed with 4 µL of a lysis solution containing 500 mM KCl, 100 mM tris-HCl (pH 9.0), 1% triton X-100. After incubation for at least 15 minutes at room temperature, 1 µL of the treated urine specimen was added directly to 19 µL of the PCR reaction mixture. The final concentration of the PCR reagents was 50 mM KCl, 10 mM Tris (pH 9.0), 0.1% Triton X-100, 2.5 mM MgCl₂, 0.4 µM of each primer, 200 µM of each of the four dNTPs. In addition, each 20 µL reaction contained 0.5 unit of *Taq* DNA polymerase (Promega) combined with the TaqStart^{™} antibody (Clontech Laboratories Inc.).

Strategies for the internal control, PCR amplification and agarose gel detection of the amplicons are as previously described in example 10.

### EXAMPLE 12:

Detection of antibiotic resistance genes. The presence of specific antibiotic resistance genes which arc frequently encountered and clinically relevant is identified using the PCR amplification or hybridization protocols described previously. Specific oligonucleotides used as a basis for the DNA-based tests are selected from the antibiotic resistance gene sequences. These tests, which allow the rapid evaluation of bacterial resistance to antimicrobial agents, can be performed either directly from clinical specimens, from a standardized bacterial suspension or from a bacterial colony and should complement diagnostic tests for the universal detection of bacteria as well as for the species-specific and genus-specific microbial detection and identification.

### EXAMPLE 13:

Same as examples 10 and 11 except that assays were performed by multiplex PCR (i.e. using several pairs of primers in a single PCR reaction) to reach an ubiquity of 100% for the specific targeted pathogen(s). For more heterogeneous microbial species or genus, a combination of PCR primer pairs may be required to detect and identify all representatives of the target species or genus.

Multiplex PCR assays could also be used to (i) detect simultaneously several microbial species and/or genera or, alternatively, (ii) to simultaneously detect and identify bacterial and/or fungal pathogens and detect specific antibiotic resistance genes either directly from a clinical specimen or from bacterial cultures.

For these applications, amplicon detection methods should be adapted to differentiate the various amplicons produced. Standard agarose gel electrophoresis could be used because it discriminates the amplicons based on their sizes. Another useful strategy for this purpose would be detection using a variety of fluorescent dyes emitting at different wavelengths. The fluorescent dyes can be each coupled with a specific oligonucleotide linked to a fluorescence quencher which is degraded during amplification to release the fluorescent dyes (e.g. TaqMan™, Perkin Elmer).

### EXAMPLE 14:

Detection of amplification products. The person skilled in the art will appreciate that alternatives other than standard agarose gel electrophoresis (Example 10) may be used for the revelation of amplification products. Such methods may be based on fluorescence polarization or on the detection of fluorescence after amplification (e.g. Amplisensor™, Biotronics; TaqMan™, Perkin-Elmer Corp.) or other labels such as biotin (SHARP Signal™ system, Digene Diagnostics). These methods are quantitative and may be automated. One of the amplification primers or an internal oligonucleotide probe specific to the amplicon(s) derived from the species-specific, genus-specific or universal DNA fragments is coupled with the fluorescent dyes or with any other label. Methods based on the detection of fluorescence are particularly suitable for diagnostic tests since they are rapid and flexible as fluorescent dyes emitting at different wavelengths are available.

### EXAMPLE 15:

Species-specific, genus-specific, universal and antibiotic resistance gene amplification primers can be used in other rapid amplification procedures such as the ligase chain reaction (LCR), transcription-mediated amplification (TMA), self-sustained sequence replication (3SR), nucleic acid sequence-based amplification (NASBA), strand displacement amplification (SDA), cycling probe technology (CPT) and branched DNA (bDNA) or any other methods to increase the sensitivity of the test. Amplifications can be performed from isolated bacterial cultures or directly from any clinical specimen. The scope of this invention is therefore not limited to the use of the DNA sequences from the enclosed Sequence Listing for PCR only but rather includes the use of any procedures to specifically detect bacterial DNA and which may be used to increase rapidity and sensitivity of the tests.

### EXAMPLE 16:

A test kit would contain sets of probes specific for each microbial species or genus as well as a set of universal probes. The kit is provided in the form of test components, consisting of the set of universal probes labeled with non-radioactive labels as well as labeled species- or genus-specific probes for the detection of each pathogen of interest in specific types of clinical samples. The kit will also include test reagents necessary to perform the pre-hybridization, hybridization, washing steps and hybrid detection. Finally, test components for the detection of known antibiotic resistance genes (or derivatives therefrom) will be included. Of course, the kit will include standard samples to be used as negative and positive controls for each hybridization test.

Components to be included in the kits will be adapted to each specimen type and to detect pathogens commonly encountered in that type of specimen. Reagents for the universal detection of bacteria will also be included. Based on the sites of infection, the following kits for the specific detection of pathogens may be developed:
- A kit for the universal detection of bacterial or fungal pathogens from all clinical specimens which contains sets of probes specific for highly conserved regions of the microbial genomes.
- A kit for the detection of microbial pathogens retrieved from urine samples, which contains 5 specific test components (sets of probes for the detection of *Enterococcus faecium, Enteroccus* species, *Staphylococcus saprophyticus, Staphylococcus* species and *Candida albicans).*
- A kit for the detection of respiratory pathogens which contains 3 specific test components (sets of probes for the detection of *Staphylococcus* species, *Enterococcus* species and *Candida albicans*).
- A kit for the detection of pathogens retrieved from blood samples, which contains 10 specific test components (sets of probes for the detection of *Streptococcus* species, *Streptococcus agalactiae, Staphylococcus* species, *Staphylococcus saprophyticus, Enterococcus* species, *Enterococcus faecium, Neisseria* species, *Neisseria meningitidis, Listeria monocytogenes* and *Candida albicans*). This kit can also be applied for direct detection and identification from blood cultures.

- A kit for the detection of pathogens causing meningitis, which contains 5 specific test components (sets of probes for the detection of *Streptococcus* species, *Listeria monocytogenes, Neisseria meningitidis, Neisseria* species and *Staphylococcus* species).
- A kit for the detection of clinically important antibiotic resistance genes which contains sets of probes for the specific detection of at least one of the 26 following genes associated with antibiotic resistance: *blaₜₑₘ, bla_{rob}, blaₛₕᵥ, blaₒₓₐ, blaZ, aadB, aacC1, aacC2, aacC3, aacA4, aac6'-IIa, ermA, ermB, ermC, mecA, vanA, vanB, vanC, salA, aac(6')-aph(2"), aad(6'), vat, vga, msrA, sul* and *int.*
- Other kits adapted for the detection of pathogens from skin, abdominal wound or any other clinically relevant infections may also be developed.

### EXAMPLE 17:

Same as example 16 except that the test kits contain all reagents and controls to perform DNA amplification assays. Diagnostic kits will be adapted for amplification by PCR (or other amplification methods) performed directly either from clinical specimens or from microbial cultures. Components required for (i) universal bacterial detection, (ii) species-specific and genus-specific bacterial and/or fungal detection and identification and (iii) detection of antibiotic resistance genes will be included.

Amplification assays could be performed either in tubes or in microtitration plates having multiple wells. For assays in plates, the wells will contain the specific amplification primers and control DNAs and the detection of amplification products will be automated. Reagents and amplification primers for universal bacterial detection will be included in kits for tests performed directly from clinical specimens. Components required for species-specific and genus-specific bacterial and/or fungal detection and identification as well as for the simultaneous antibiotic resistance genes detection will be included in kits for testing directly from bacterial or fungal cultures as well as in kits for testing directly from any type of clinical specimen.

The kits will be adapted for use with each type of specimen as described in example 16 for hybridization-based diagnostic kits.

### EXAMPLE 18:

It is understood that the use of the probes and amplification primers described in this invention for bacterial and/or fungal detection and identification is not limited to clinical microbiology applications. In fact, we feel that other sectors could also benefit from these new technologies. For example, these tests could be used by industries for quality control of food, water, air, pharmaceutical products or other products requiring microbiological control. These tests could also be applied to detect and identify bacteria or fungi in biological samples from organisms other than humans (e.g. other primates, birds, plants, mammals, farm animals, livestock and others). These diagnostic tools could also be very useful for research purposes including clinical trials and epidemiological studies.

This invention has been described herein above, and it is readily apparent that modifications can be made thereto without departing from the spirit of this invention. These modifications are under the scope of this invention, as defined in the appended claims.

**Table 1. Distribution (%) of nosocomial pathogens for various human infections in USA (1990-1992)¹.**

| Pathogen | UTI² | SSI³ | BSI⁴ | Pneumonia | CSF⁵ |
|---|---|---|---|---|---|
| *Escherichia coli* | 27 | 9 | 5 | 4 | 2 |
| *Staphylococcus aureus* | 2 | 21 | 17 | 21 | 2 |
| *Staphylococcus epidennidis* | 2 | 6 | 20 | 0 | 1 |
| *Enterococcus faecalis* | 16 | 12 | 9 | 2 | 0 |
| *Enterococus faecium* | 1 | 1 | 0 | 0 | 0 |
| *Pseudomonas aeruginosa* | 12 | 9 | 3 | 18 | 0 |
| *Klebsiella pneumoniae* | 7 | 3 | 4 | 9 | 0 |
| *Proteus mirabilis* | 5 | 3 | 1 | 2 | 0 |
| *Streptococcus pneumoniae* | 0 | 0 | 3 | 1 | 18 |
| Group B *Streptococci* | 1 | 1 | 2 | 1 | 6 |
| Other *Streptococci* | 3 | 5 | 2 | 1 | 3 |
| *Haemophilus influenzae* | 0 | 0 | 0 | 6 | 45 |
| *Neisseria meningitidis* | 0 | 0 | 0 | 0 | 14 |
| *Listeria monocytogenes* | 0 | 0 | 0 | 0 | 3 |
| Other *Enterococci* | 1 | 1 | 0 | 0 | 0 |
| Other *Staphylococci* | 2 | | 8 | 13 | 20 |
| *Candida albicans* | 9 | 3 | 5 | 5 | 0 |
| Other *Candida* | 2 | | 1 | 3 | 10 |
| *Enterobacter* spp. | 5 | 7 | 4 | 12 | 2 |
| *Acinetobacter* spp. | 1 | 1 | 2 | 4 | 2 |
| *Citrobacter* spp. | 2 | 1 | 1 | 1 | 0 |
| *Serratia marcescens* | 1 | 1 | 1 | 3 | 1 |
| Other *Klebsiella* | 1 | 1 | 1 | 2 | 1 |
| Others | 0 | 6 | 4 | 5 | 0 |

| | | | | | |
|---|---|---|---|---|---|
| ¹ Data recorded by the National Nosocomial Infections Surveillance (NNIS) from 80 hospitals (Emori and Gaynes, 1993, Clin. Microbiol. Rev., 6:428-442). ² Urinary tract infection. ³ Surgical site infection. ⁴ Bloodstream infection. ⁵ Cerebrospinal fluid. | | | | | |

**Table 2. Distribution (%) of bloodstream infection pathogens in Quebec (1995), Canada (1992), UK (1969-1988) and USA (1990-1992).**

| Organism | Quebec¹ | Canada² | UK³ | | USA⁴ |
|---|---|---|---|---|---|
| | | | Community-acquired | Hospital-acquired | Hospital-acquired |
| *E. coli* | 15.6 | 53.8 | 24.8 | 20.3 | 5.0 |
| *S. epidermidis* and other CoNS⁵ | 25.8 | NI⁶ | 0.5 | 7.2 | 31.0 |
| *S. aureus* | 9.6 | NI | 9.7 | 19.4 | 16.0 |
| *S. pneumoniae* | 6.3 | NI | 22.5 | 2.2 | NR⁷ |
| *E. faecalis* | 3.0 | NI | 1.0 | 4.2 | NR |
| *E. faecium* | 2.6 | NI | 0.2 | 0.5 | NR |
| *Enterococcus* spp. | NR | NI | NR | NR | 9.0 |
| *H. influenzae* | 1.5 | NR | 3.4 | 0.4 | NR |
| *P. aeruginosa* | 1.5 | 8.2 | 1.0 | 8.2 | 3.0 |
| *K. pneumoniae* | 3.0 | 11.2 | 3.0 | 9.2 | 4.0 |
| *P. mirabilis* | NR | 3.9 | 2.8 | 5.3 | 1.0 |
| *S. pyogenes* | NR | NI | 1.9 | 0.9 | NR |
| *Enterobacter* spp. | 4.1 | 5.5 | 0.5 | 2.3 | 4.0 |
| *Candida* spp. | 8.5 | NI | NR | 1.0 | 8.0 |
| Others | 18.5 | 17.4⁸ | 28.7 | 18.9 | 19.0 |

| | | | | | |
|---|---|---|---|---|---|
| ¹ Data obtained for 270 isolates collected at the Centre Hospitalier de l'Université Laval (CHUL) during a 5 month period (May to October 1995). ² Data from 10 hospitals throughout Canada representing 941 gram-negative bacterial isolates. (Chamberland et al., 1992, Clin. Infect. Dis., 15:615-628). ³ Data from a 20-year study (1969-1988) for nearly 4000 isolates (Eykyn et al., 1990, J. Antimicrob. Chemother., Suppl. C, 25:41-58). ⁴ Data recorded by the National Nosocomial Infections Surveillance (NNIS) from 80 hospitals (Emori and Gaynes, 1993, Clin. Microbiol. Rev., 6:428-442). ⁵ Coagulase-negative staphylococci. ⁶ NI, not included. This survey included only gram-negative species. ⁷ NR, incidence not reported for these species or genera. ⁸ In this case, 17.4 stands for other gram-negative bacterial species. | | | | | |

**Table 3. Distribution of positive and negative clinical specimens tested at the microbiology laboratory of the CHUL (February 1994 - January 1995).**

| Clinical specimens and/or sites | No. of samples tested (%) | % of positive specimens | % of negative specimens |
|---|---|---|---|
| Urine | 17,981 (54.5) | 19.4 | 80.6 |
| Blood culture/marrow | 10,010 (30.4) | 6.9 | 93.1 |
| Sputum | 1,266 (3.8) | 68.4 | 31.6 |
| Superficial pus | 1,136(3.5) | 72.3 | 27.7 |
| Cerebrospinal fluid | 553 (1.7) | 1.0 | 99.0 |
| Synovial fluid | 523 (1.6) | 2.7 | 97.3 |
| Respiratory tract | 502 (1.5) | 56.6 | 43.4 |
| Deep pus | 473 (1.4) | 56.8 | 43.2 |
| Ears | 289 (0.9) | 47.1 | 52.9 |
| Pleural and pericardial fluid | 132(0.4) | 1.0 | 99.0 |
| Peritoneal fluid | 101(0.3) | 28.6 | 71.4 |
| **Total:** | **32,966 (100.0)** | **20.0** | **80.0** |

**Table 4. Gram-negative bacterial species (90) used to test the specificity of PCR primers and DNA probes (continues on next page).**

| Bacterial species | Number of reference strains tested^{a} | Bacterial species | Number of reference strains tested^{a} |
|---|---|---|---|
| *Acinetobacter baumannii* | 1 | *Moraxella phenylpyruvica* | 1 |
| *Acinetobacter lwoffii* | 3 | *Morganella morganii* | 1 |
| *Actinobacillus lignieresii* | 1 | *Neisseria anima*/*is* | 1 |
| *Alcaligenes faecalis* | 1 | *Neisseria canis* | 1 |
| *Alcaligenes odorans* | 1 | *Neisseria caviae* | 1 |
| *Alcaligenes xylosoxydans* | | *Neisseria cinerea* | 1 |
| subsp. *denitrificans* | 1 | *Neisseria cuniculi* | 1 |
| *Bacteroides distasonis* | 1 | *Neisseria elongata* subsp. *elongata* | 1 |
| *Bacteroides fragilis* | 1 | *Neisseria elongata* subsp. *glycoytica* | 1 |
| *Bacteroides ovatus* | 1 | *Neisseria flavescens* | 1 |
| *Bacteroides thetaiotaomicron* | 1 | *Neisseria flavescens Branham* | 1 |
| *Bacteroides vulgatus* | 1 | *Neisseria gonorrhoeae* | 18 |
| *Bordetella bronchiseptica* | 1 | *Neisseria lactamica* | 1 |
| *Bordetella parapertussis* | 1 | *Neisseria meningitidis* | 4 |
| *Bordetella pertussis* | 2 | *Neisseria mucosa* | 2 |
| *Burkholderia cepacia* | 1 | *Neisseria polysaccharea* | 1 |
| *Citrobacter amalonaticus* | 1 | *Neisseria sicca* | 3 |
| *Citrobacter diversus* subsp. k*oseri* | 2 | *Neisseria subflava* | 3 |
| *Citrobacter freundii* | 1 | *Neisseria weaveri* | 1 |
| *Comamonas acidovorans* | 1 | *Ochrobactrum antropi* | 1 |
| *Enterobacter aerogenes* | 1 | *Pasteurella aerogenes* | 1 |
| *Enterobacter agglomerans* | 1 | *Pasteurella multocida* | 1 |
| *Enterobacter cloacae* | 1 | *Prevotella melaninogenica* | 1 |
| *Escherichia coli* | 9 | *Proteus mirabilis* | 3 |
| *Escherichica fergusonii* | 1 | *Proteus vulgaris* | 1 |
| *Escherichia hermannii* | 1 | *Providencia alcalifaciens* | 1 |
| *Escherichia vulneris* | 1 | *Providencia rettgeri* | 1 |
| *Flavobacterium meningosepticum* | 1 | *Providencia rustigianii* | 1 |
| *Flavobacterium indologenes* | 1 | *Providencia stuartii* | 1 |
| *Flavobacterium odoratum* | 1 | *Pseudomonas aeruginosa* | 14 |
| *Fusobacterium necrophorum* | 2 | *Pseudomonas fluorescens* | 2 |
| *Gardnerella vaginalis* | 1 | *Pseudomonas stutzeri* | 1 |
| *Haemophilus haemolyticus* | 1 | *Salmonella arizonae* | 1 |
| *Haemophilus influenzae* | 12 | *Salmonella choleraesuis* | 1 |
| *Haemophilus parahaemolyticus* | 1 | *Salmonella gallinarum* | 1 |
| *Haemophilus parainfluenzae* | 2 | *Salmonella typhimurium* | 3 |
| *Hafnia alvei* | 1 | *Serratia liquefaciens* | 1 |
| *Kingella indologenes* subsp. *suttonella* | 1 | *Serratia marcescens* | 1 |
| *Kingella kingae* | 1 | *Shewanella putida* | 1 |
| *Klebsiella ornithinolytica* | 1 | *Shigella boydii* | 1 |
| *Klebsiella oxytoca* | 1 | *Shigella dysenteriae* | 1 |
| *Klebsiella pneumoniae* | 8 | *Shigella flexneri* | 1 |
| *Moraxella atlantae* | 1 | *Shigella sonnei* | 1 |
| *Moraxella catarrhalis* | 5 | *Stenotrophomonas maltophilia* | 1 |
| *Moraxella lacunata* | 1 | *Yersinia enterocolitica* | 1 |
| *Moraxella osloensis* | 1 | | |

| | | | |
|---|---|---|---|
| ^{a} Most reference strains were obtained from the American Type Culture Collection (ATCC). The other reference strains were obtained from (i) the Laboratoire de Santé Publique du Québec (LSPQ), (ii) the Center for Disease Control and Prevention (CDC) and (iii) the National Culture Type Collection (NCTC). | | | |

**Table 5. Gram-positive bacterial species (97) used to test the specificity of PCR primers and DNA probes (continues on next page).**

| Bacterial species | Number of reference strains tested^{a} | Bacterial species | Number of reference strains tested^{a} |
|---|---|---|---|
| *Abiotrophia adiacens* | 1 | *Micrococcus kristinae* | 1 |
| *Abiotrophia defectiva* | 1 | *Micrococcus luteus* | 1 |
| *Actinomyces israelii* | 1 | *Micrococcus lylae* | 1 |
| *Clostridium perfringens* | 1 | *Micrococcus roseus* | 1 |
| *Corynebacterium accolens* | 1 | *Micrococcus varians* | 1 |
| *Corynebacterium aquaticum* | 1 | *Peptococcus niger* | 1 |
| *Corynebacterium bovis* | 1 | *Peptostreptococcus anaerobius* | 1 |
| *Corynebacterium cervicis* | 1 | *Peptostreptococcus asaccharolyticus* | 1 |
| *Corynebacterium diphteriae* | 6 | *Staphylococcus aureus* | 10 |
| *Corynebacterium flavescens* | 1 | *Staphylococcus auricularis* | 1 |
| *Corynebacterium genitalium* | 6 | *Staphylococcus capitis* subsp. *urealyticus* | 1 |
| *Corynebacterium jeikeium* | 1 | *Staphylococcuss cohnii* | 1 |
| *Corynebacterium kutcheri* | 1 | *Staphylococcus epidermidis* | 2 |
| *Corynebacterium matruchotii* | 1 | *Staphylococcus haemolyticus* | 2 |
| *Corynebacterium minutissimum* | 1 | *Staphylococcus hominis* | 2 |
| *Corynebacterium mycetoides* | 1 | *Staphylococcus lugdunensis* | 1 |
| *Corynebacterium pseudodiphtheriticum* | 1 | *Staphylococcus saprophyticus* | 3 |
| *Corynebacterium pseudogenitalium* | 6 | *Staphylococcus schleiferi* | 1 |
| *Corynebacterium renale* | 1 | *Staphylococcus sciuri* | 1 |
| *Corynebacterium striatum* | 1 | *Staphylococcus simulans* | 1 |
| *Corynebacterium ulcerans* | 1 | *Staphylococcus warneri* | 1 |
| *Corynebacterium urealyticum* | 1 | *Staphylococcus xylosus* | 1 |
| *Corynebacterium xerosis* | 1 | *Streptococcus agalactiae* | 6 |
| *Enterococcus avium* | 1 | *Streptococcus anginosus* | 2 |
| *Enterococcus casseliflavus* | 1 | *Streptococcus bovis* | 2 |
| *Enterococcus cecorum* | 1 | *Streptococcus constellatus* | 1 |
| *Enterococcus dispar* | 1 | *Streptococcus crista* | 1 |
| *Enterococcus durans* | 1 | *Streptococcus dysgalactiae* | 1 |
| *Enterococcus faecalis* | 6 | *Streptococcus equi* | 1 |
| *Enterococcus faecium* | 3 | *Streptococcus gordonii* | 1 |
| *Enterococcus flavescens* | 1 | *Group C Streptococci* | 1 |
| *Enterococcus gallinarum* | 3 | *Group D Streptococci* | 1 |
| *Enterococcus hirae* | 1 | *Group E Streptococci* | 1 |
| *Enterococcus mundtii* | 1 | *Group F Streptococci* | 1 |
| *Enterococcus pseudoavium* | 1 | *Group G Streptococci* | 1 |
| *Enterococcus raffinosus* | 1 | *Streptococcus intermedius* | 1 |
| *Enterococcus saccharolyticus* | 1 | *Streptococcus mitis* | 2 |
| *Enterococcus solitarius* | 1 | *Streptococcus mutans* | 1 |
| *Eubacterium lentum* | 1 | *Streptococcus oralis* | 1 |
| *Gemella haemolysans* | 1 | *Streptococcus parasanguis* | 1 |
| *Gemella morbillorum* | 1 | *Streptococcus pneumoniae* | 6 |
| *Lactobacillus acidophilus* | 1 | *Streptococcus pyogenes* | 3 |
| *Listeria innocua* | 1 | *Streptococcus salivarius* | 2 |
| *Listeria ivanovii* | 1 | *Streptococcus sanguis* | 2 |
| *Listeria grayi* | 1 | *Streptococcus sobrinus* | 1 |
| *Listeria monocytogenes* | 3 | *Streptococcus suis* | I |
| *Listeria murrayi* | 1 | *Streptococcus uberis* | 1 |
| *Listeria seeligeri* | 1 | *Streptococcus vestibularis* | 1 |
| *Listeria welshimeri* | 1 | | |

| | | | |
|---|---|---|---|
| ^{a} Most reference strains were obtained from the American Type Culture Collection (ATCC). The other reference strains were obtained from (i) the Laboratoire de Santé Publique du Québec (LSPQ), (ii) the Center for Disease Control and Prevention (CDC) and (iii) the National Culture Type Collection (NCTC). | | | |

**Table 6. Fungal species (12) used to test the specificity of PCR primers and DNA probes.**

| Fungal species | Number of reference strains tested^{a} |
|---|---|
| *Candida albicans* | 12 |
| *Candida glabrata* | 1 |
| *Candida guilliermondii* | 1 |
| *Candida kefyr* | 3 |
| *Candida krusei* | 2 |
| *Candida lusitaniae* | 1 |
| *Candida parapsilosis* | 2 |
| *Candida tropicalis* | 3 |
| *Rhodotorula glutinis* | 1 |
| *Rhodotorula minuta* | 1 |
| *Rhodotorula rubra* | 1 |
| *Saccharomyces cerevisiae* | 1 |

| | |
|---|---|
| ^{a} Most reference strains were obtained from (i) the American Type Culture Collection (ATCC) and (ii) the Laboratoire de Santé Publique du Québec (LSPQ). | |

**Table 7. PCR assays developed for several clinically important bacterial and fungal pathogens (continues on next page).**

| Organism | Primer Pair^{a} SEQ ID NO | Amplicon size (bp) | Ubiquity^{b} | DNA amplification from | |
|---|---|---|---|---|---|
| | | | | culture^{c} | specimens^{d} |
| *Enterococcus faecium* | 1-2 | 216 | 79/80 | + | + |
| *Listeria monocytogenes* | 3-4 | 130 | 164/168^{e} | + | + |
| *Neisseria meningitidis* | 5-6 | 177 | 258/258 | + | + |
| *Staphylococcus saprophyticus* | 7-8 | 149 | 245/260 | + | NT |
| Streptococcus agalactiae | 9-10 | 154 | 29/29 | + | + |
| *Candida albicans* | 11-12 | 149 | 88/88 | + | NT |
| *Enterococcus* spp. (11 species)^{f} | 13-14 | 112 | 87/87 | + | NT |
| *Neisseria* spp. (12 species)^{f} | 15-16 | 103 | 321/321 | + | + |
| *Staphylococcus* spp. (14 species) | 17-18 | 192 | 13/14 | + | NT |
| | 19-20 | 221 | 13/14 | + | NT |
| *Streptococcus* spp. (22 species)^{f} | 21-22 | 153 | 210/214^{g} | + | + |
| Universal detection^{h} (95 species)' | 23-24 | 309 | 104/ 116ⁱ | + | + |

| | | | | | |
|---|---|---|---|---|---|
| ^{a} All primer pairs are specific in PCR assays since no amplification was observed with DNA from the bacterial and fungal species other than the species of interest listed in Tables 4, 5 and 6. ^{b} Ubiquity was tested by using reference strains as well as strains from throughout the world, which are representatite of the diversity within each target species or genus. ^{c} For all primer pairs, PCR amplifications performed directly from a standardized ^{d} microbial suspension (MacFarland) or from a colony were all specific and ubiquitous. ^{d} PCR assays performed directly from blood cultures, urine specimens or cerebrospinal fluid. NT, not tested. ^{e} The four *L*. *monocytogenes* strains undetected are not clinical isolates. These strains were isolated from food and are not associated with a human infection. ^{f} The bacterial species tested include all those clinically relevant for each genus (Tables 4 and 5). All of these species were efficiently amplified by their respective genus-specific PCR assay, except for the *Staphylococcus-specific* assay, which does not amplify *S. sciuri.* ^{g} The *Streptococcus-specific* PCR assay did not amplify 3 out of 9 strains of *S*. *mutans* and 1 out of 3 strains of *S. salivarius.* ^{h} The primers selected for universal bacterial detection do not amplify DNA of non-bacterial origin, including human and other types of eukaryotic genomic DNA. ⁱ For the universal amplification, the 95 bacterial species tested represent the most clinically important bacterial species listed in Tables 4 and 5. The 12 strains not amplified are representatives of genera *Corynebacterium* (11 species) and *Stenotrophomonas* (1 species). | | | | | |

**Table 8. Target genes for the various genus-specific, species-specific and universal amplification assays.**

| Microorganisms | Gene | Protein encoded |
|---|---|---|
| *Candida albicans* | *tuf* | translation elongation factor EF-Tu |
| *Enterococcus faecium* | *ddl* | D-alanine:D-alanine ligase |
| *Listeria monocytogenes* | *actA* | actin-assembly inducing protein |
| *Neisseria meningitidis* | *omp* | outer membrane protein |
| *Streptococcus agalactiae* | *cAMP* | cAMP factor |
| *Staphylococcus saprophyticus* | unknown | unknown |
| *Enterococcus* spp. | *tuf* | translation elongation factor EF-Tu |
| *Neisseria* spp. | *asd* | ASA-dehydrogenase |
| *Staphylococcus* spp. | *tuf* | translation elongation factor EF-Tu |
| *Streptococcus* spp. | *rec*A | RecA protein |
| Universal detection | *tuf* | translation elongation factor EF-Tu |

**Table 9. Antibiotic resistance genes selected for diagnostic purposes.**

| Genes | SEQ ID NOs | | Antibiotics | Bacteria^{a} |
|---|---|---|---|---|
| | selected primers | originating fragment | | |
| | | | | |
| *blaₒₓₐ* | 49-50 | 110 | β-lactams | *Enterobacteriaceae,Ps eudomonadaceae* |
| | | | | |
| *blaZ* | 51-52 | 111 | β-lactams | *Enterococcus* spp. |
| *aac6'-IIa* | 61-64 | 112 | Aminoglycosides | *Pseudomonadaceae* |
| *ermA* | 91-92 | 113 | Macrolides | *Staphylococcus* spp. |
| *ermB* | 93-94 | 114 | Macrolides | *Staphylococcus* spp. |
| *ermC* | 95-96 | 115 | Macrolides | *Staphylococcus* spp. |
| *vanB* | 71-74 | 116 | Vancomycin | *Enterococcus* spp. |
| *vanC* | 75-76 | 117 | Vancomycin | *Enterococcus* spp. |
| *aad(6')* | 173-174 | - | Streptomycin | *Enterococcus* spp. |

| | | | | |
|---|---|---|---|---|
| ^{a} Bacteria having high incidence for the specified antibiotic resistance genes. The presence of these antibiotic resistance genes in other bacteria is not excluded. | | | | |

**Table 10. Antibiotic resistance genes from our co-pending US (N.S. 08/526840) and PCT (PCT/CA/95/00528) patent applications for which we have selected PCR primer pairs.**

| Genes | SEQ ID NOs of selected primers | Antibiotics | Bacteria^{a} |
|---|---|---|---|
| | | | |
| *blaₜₑₘ* | 37-40 | β-lactams | *Enterobacteriaceae,Ps eudomonadaceae,Hae mophilus* spp., *Neisseria* spp. |
| *bla_{rob}* | 45-48 | β-lactams | *Haemophilus* spp., *Pasteurella* spp. |
| *blaₛₕᵥ* | 41-44 | β-lactams | *Klebsiella* spp. and other *Enterobacteriaceae* |
| | | | |
| *aadB* | 53-54 | Aminoglycosides | *Enterabacteriaceae,* |
| *aac*C1 | 55-56 | | *Pseudomonadaceae* |
| *aac*C2 | 57-58 | | |
| *aac*C3 | 59-60 | | |
| *aac*A4 | 65-66 | | |
| *mecA* | 97-98 | β-lactams | *Staphylococcus* spp. |
| *van*A | 67-70 | Vancomycin | *Enterococcus* spp. |
| *sat*A | 81-82 | Macrolides | *Enterococcus* spp. |
| *aac*(6')*-aph*(2") | 83-86 | Aminoglycosides | *Enterococcus* spp., |
| | | | *Staphylococcus* spp. |
| *vat* | 87-88 | Macrolides | *Staphylococcus* spp. |
| *vga* | 89-90 | Macrolides | *Staphylococcus* spp. |
| *msr*A | 77-80 | Erythromycin | *Staphylococcus* spp. |
| *int* | 99-102 | β-lactams, | *Enterobacteriaceae,* |
| | | trimethoprim, | |
| *sul* | 103-106 | aminoglycosides, | *Pseudonionadaceae* |
| | | antiseptic, | |
| | | chloramphenicol | |

| | | | |
|---|---|---|---|
| ^{a} Bacteria having high incidence for the specified antibiotic resistance genes. The presence of these antibiotic resistance genes in other bacteria is not excluded. | | | |

**Table 11. Correlation between disk diffusion and PCR amplification of antibiotic resistance genes in Staphylococcus species^{a}.**

| | | | Disk diffusion (Kirby-Bauer)^{b} | | |
|---|---|---|---|---|---|
| Antibiotic | Phenotype | PCR | Resistant | Intermediate | Sensitive |
| Penicillin | *blaZ* | + | 165 | 0 | 0 |
| | | - | 0 | 0 | 31 |
| Oxacillin | *mecA* | + | 51 | 11 | 4 |
| | | - | 2 | 0 | 128 |
| Gentamycin | *aac*(6')*aph*(2") | + | 24 | 18 | 6 |
| | | - | 0 | 0 | 148 |
| Erythromycin | *erm*A | + | 15 | 0 | 0 |
| | *erm*B | + | 0 | 0 | 0 |
| | *erm*C | + | 43 | 0 | 0 |
| | *msr*A | + | 4 | 0 | 0 |
| | | - | 0 | 1 | 136 |

| | | | | | |
|---|---|---|---|---|---|
| ^{a} The *Staphylococcus* strains studied include *S. aureus* (82 strains), *S. epidermidis* (83 strains), *S. hominis* (2 strains), *S. capitis* (3 strains), *S. haemolyticus* (9 strains), *S. simulans* (12 strains) and *S. warneri* (5 strains), for a total of 196 strains. ^{b} Susceptibility testing was performed by the method of Kirby-Bauer according to the protocol reccommended by the National Committee of Clinical Laboratory Standards (NCCLS). | | | | | |

**Table 12. Correlation between disk diffusion profiles and PCR amplification of antibiotic resistance genes in Enterococcus species^{a}.**

| | | | Disk diffusion (Kirby-Bauer)^{b} | |
|---|---|---|---|---|
| Antibiotic | Phenotype | PCR | Resistant | Sensitive |
| Ampicillin | *blaZ* | + | 0 | 2 |
| | | - | 1 | 30 |
| Gentamycin | *aac(6')aph(2")* | + | 51 | 1 |
| | | - | 3 | 38 |
| Streptomycin | *aad(6')* | + | 26 | 15 |
| | | - | 6 | 27 |
| Vancomycin | *vanA* | + | 36 | 0 |
| | *vanB* | + | 26 | 0 |
| | | - | 0 | 40 |

| | | | | |
|---|---|---|---|---|
| ^{a} The *Enterococcus* strains studied include *E. faecalis* (33 strains) and *E. faecium* (69 strains), for a total of 102 strains. ^{b} Susceptibility testing was performed by the method of Kirby-Bauer according to the protocol reccommended by the National Committee of Clinical Laboratory Standards (NCCLS). | | | | |

**Table 13. Origin of tuf sequences in the Sequence Listing (continues on next page).**

| SEQ ID NO | Bacterial or fungal species | Source |
|---|---|---|
| 118 | *Abiotrophia adiacens* | This patent |
| 119 | *Abiotrophia defectiva* | This patent |
| 120 | *Candida albicans* | This patent |
| 121 | *Candida glabrata* | This patent |
| 122 | *Candida krusei* | This patent |
| 123 | *Candida parapsilosis* | This patent |
| 124 | *Candida tropicalis* | This patent |
| 125 | *Corynebacterium accolens* | This patent |
| 126 | *Corynebacterium diphteriae* | This patent |
| 127 | *Corynebacterium genitalium* | This patent |
| 128 | *Corynebacterium jeikeium* | This patent |
| 129 | *Corynebacterium pseudotuberculosis* | This patent |
| 130 | *Corynebacterium striatum* | This patent |
| 131 | *Enterococcus avium* | This patent |
| 132 | *Enterococcus faecalis* | This patent |
| 133 | *Enterococcus faecium* | This patent |
| 134 | *Enterococcus gallinarum* | This patent |
| 135 | *Gardnerella vaginalis* | This patent |
| 136 | *Listeria innocua* | This patent |
| 137 | *Listeria ivanovii* | This patent |
| 138 | *Listeria monocytogenes* | This patent |
| 139 | *Listeria seeligeri* | This patent |
| 140 | *Staphylococcus aureus* | This patent |
| 141 | *Staphylococcus epidermidis* | This patent |
| 142 | *Staphylococcus saprophyticus* | This patent |
| 143 | *Staphylococcus simulans* | This patent |
| 144 | *Streptococcus agalactiae* | This patent |
| 145 | *Streptococcus pneumoniae* | This patent |
| 146 | *Streptococcus salivarius* | This patent |
| 147 | *Agrobactetium tumefaciens* | Database |
| 148 | *Bacillus subtilis* | Database |
| 149 | *Bacteroides fragilis* | Database |
| 150 | *Borrelia burgdorferi* | Database |
| 151 | *Brevibacterium linens* | Database |
| 152 | *Burkholderia cepacia* | Database |
| 153 | *Chlamydia trachomatis* | Database |
| 154 | *Escherichia coli* | Database |
| 155 | *Fibrobacter succinogenes* | Database |
| 156 | *Flavobacterium ferrugineum* | Database |
| 157 | *Haemophilus influenzae* | Database |
| 158 | *Helicobacter pylori* | Database |
| 159 | *Micrococcus luteus* | Database |
| 160 | *Mycobacterium tuberculosis* | Database |
| 161 | *Mycoplasma genitalium* | Database |
| 162 | *Neisseria gonorrhoeae* | Database |
| 163 | *Rickettsia prowazekii* | Database |
| 164 | *Salmonella typhimurium* | Database |
| 165 | *Shewanella putida* | Database |
| 166 | *Stigmatella aurantiaca* | Database |
| 167 | *Streptococcus pyogenes* | Database |
| 168 | *Thiobacillus cuprinus* | Database |
| 169 | *Treponema pallidum* | Database |
| 170 | *Ureaplasma urealyticum* | Database |
| 171 | *Wolinella succinogenes* | Database |

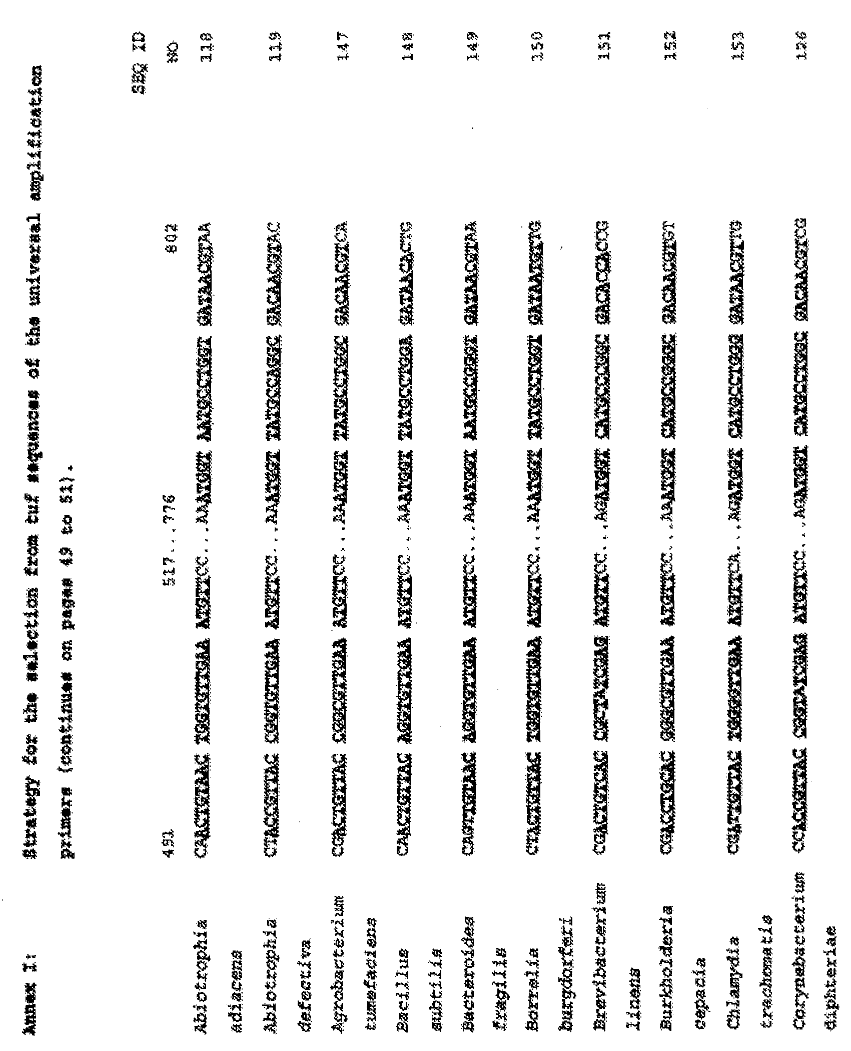

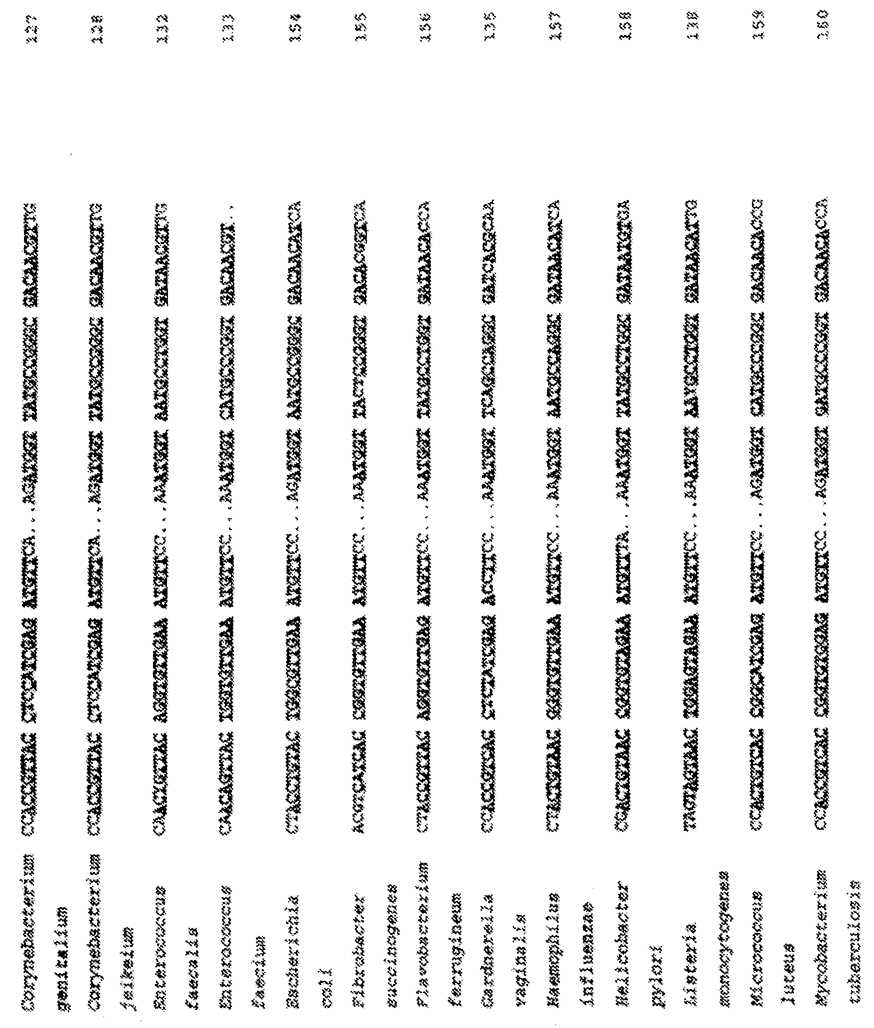

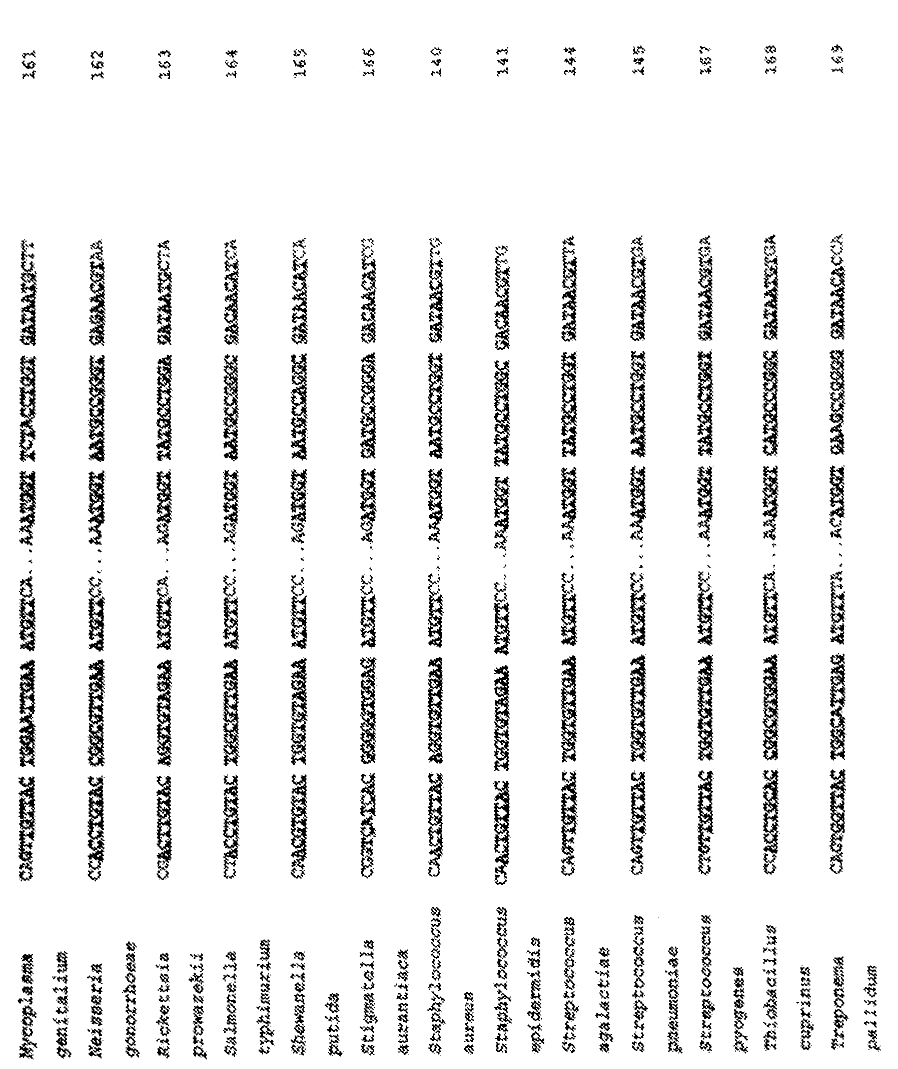

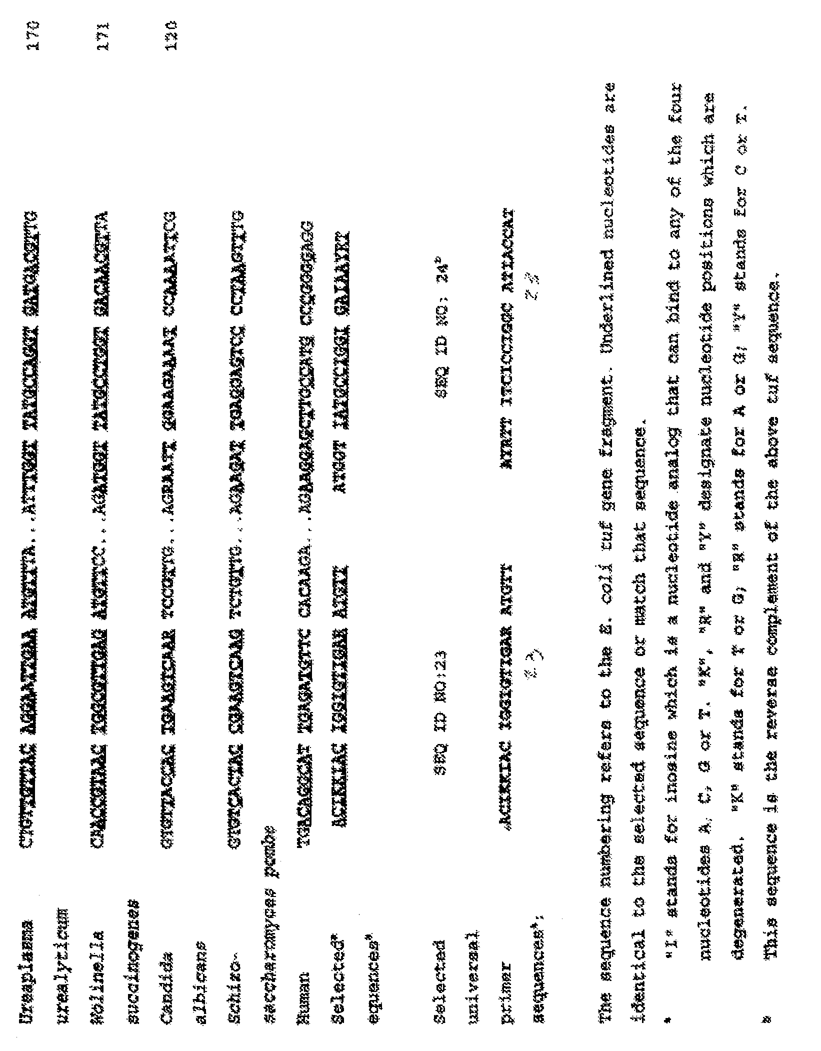

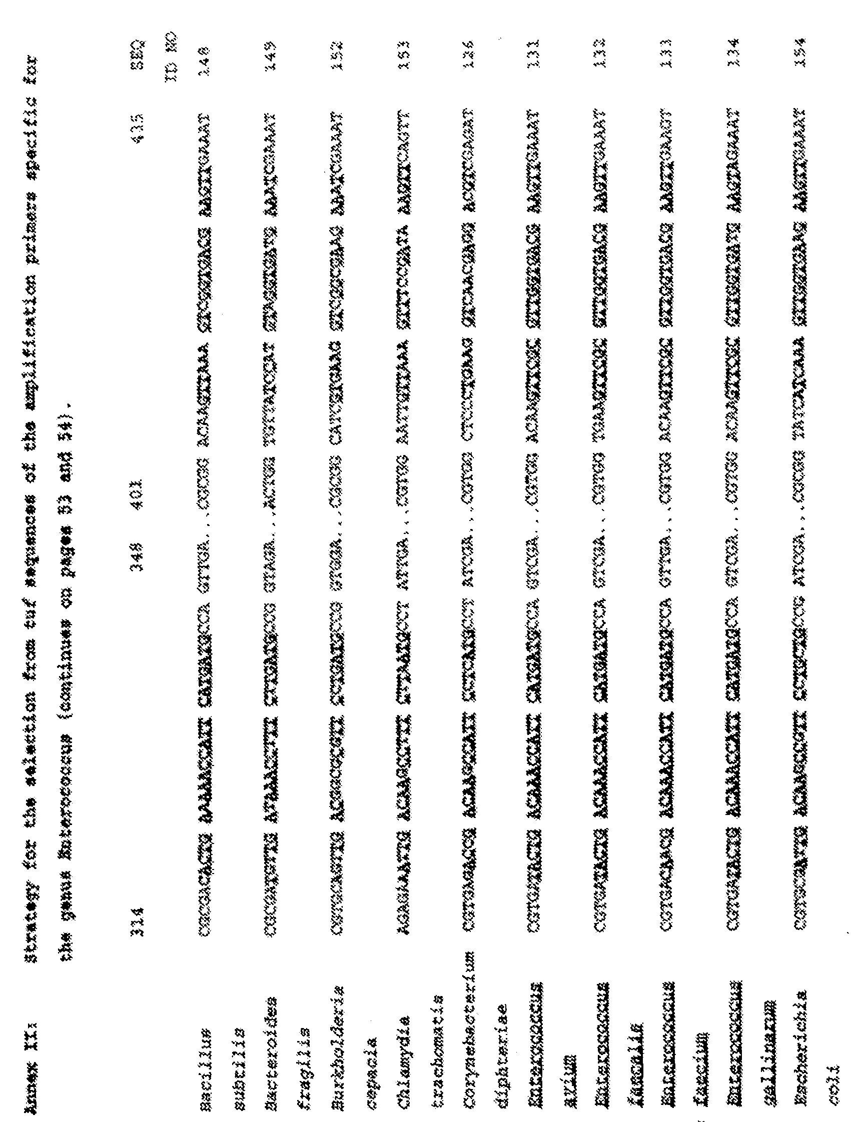

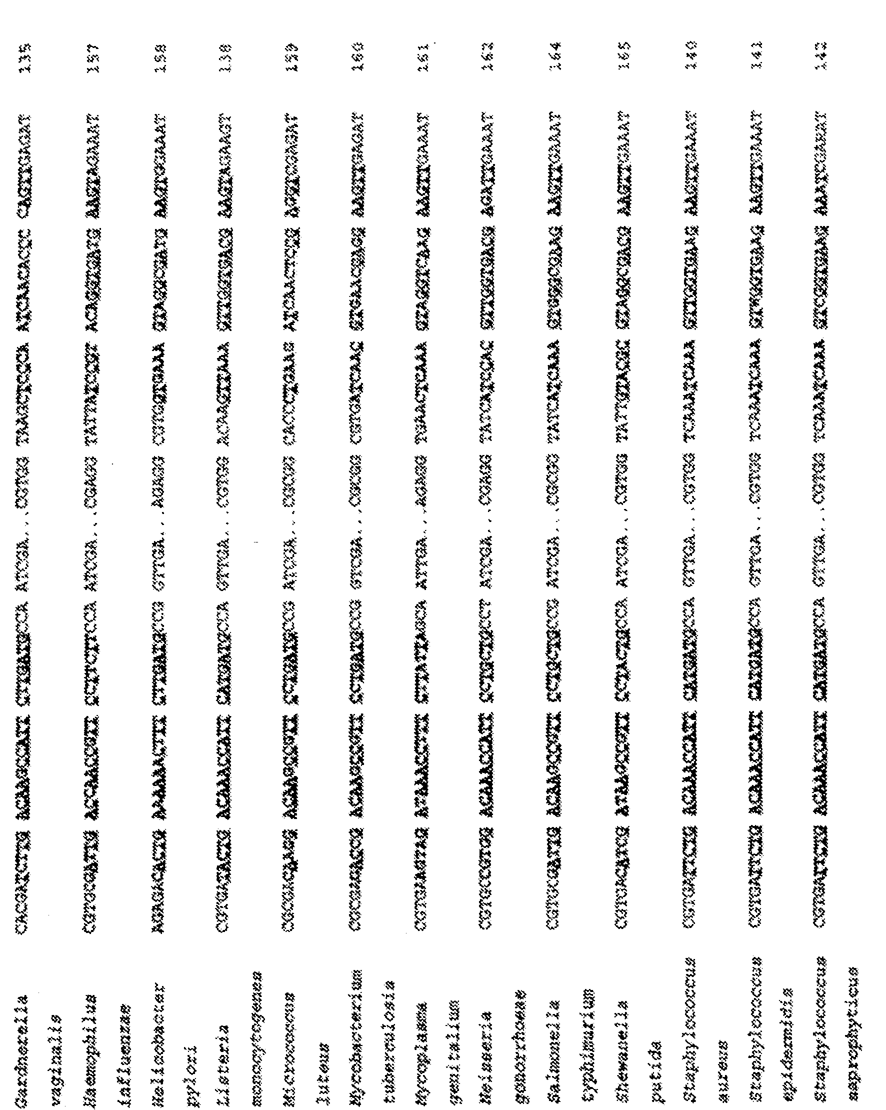

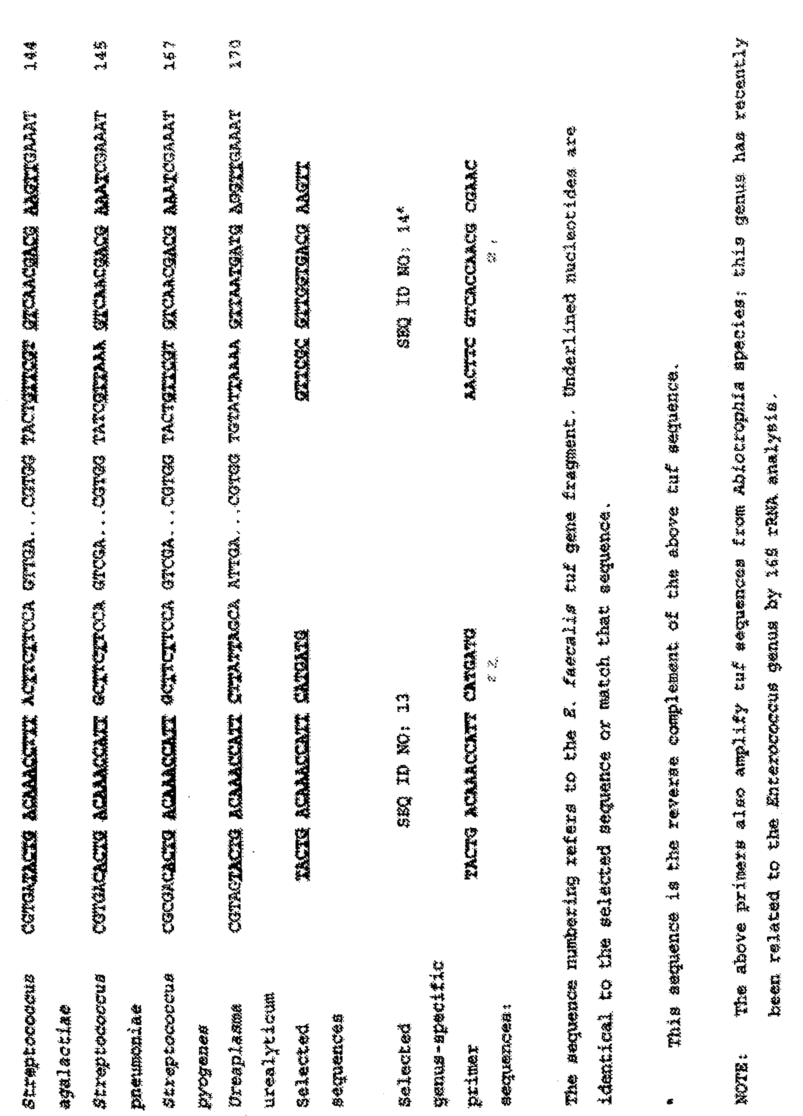

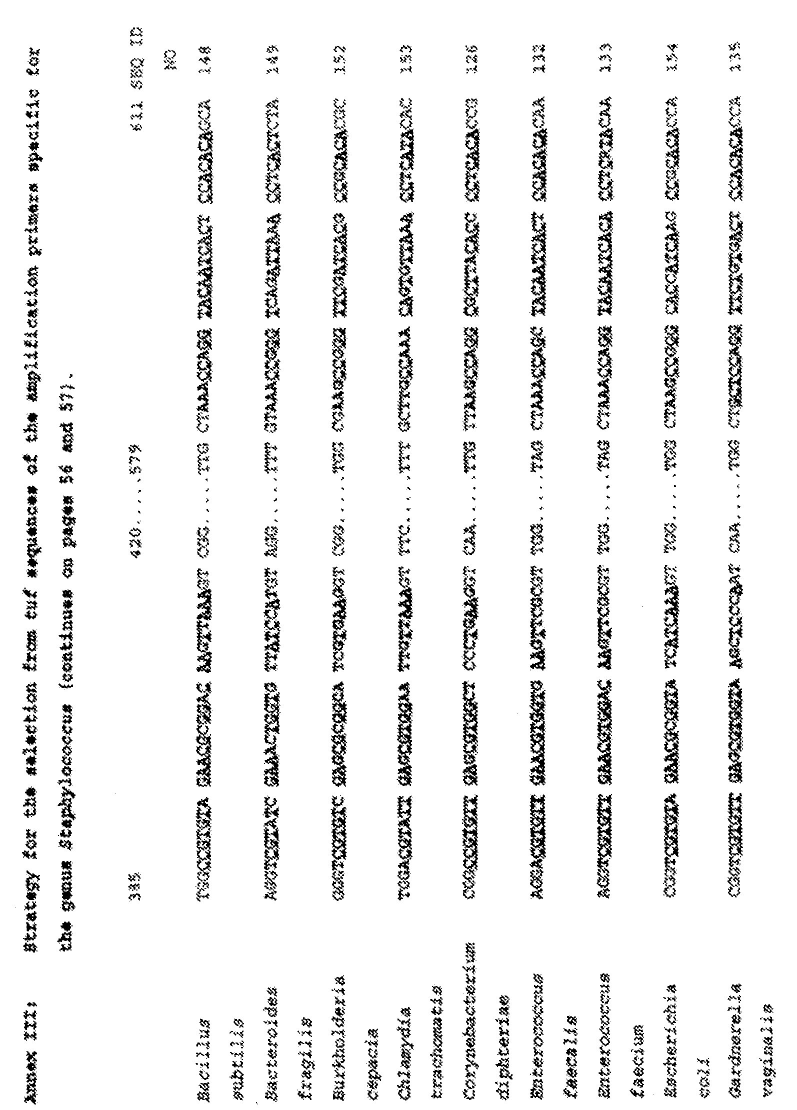

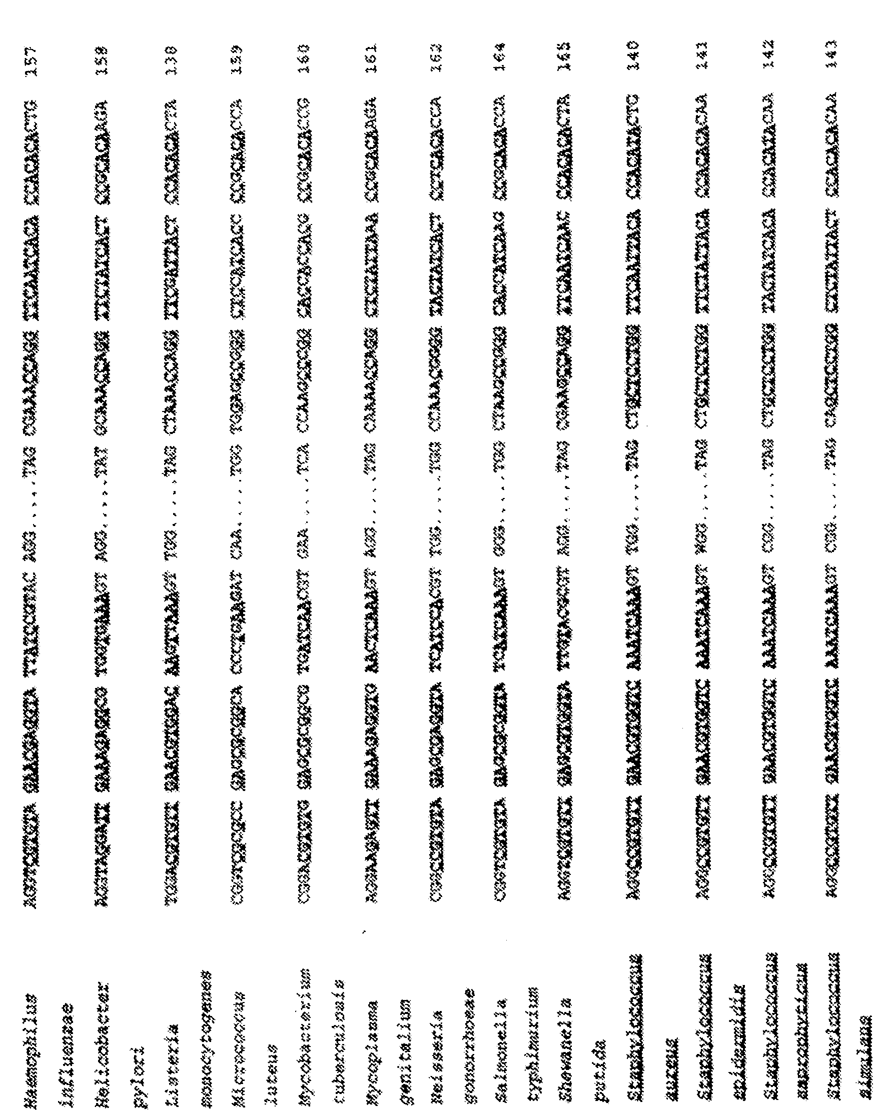

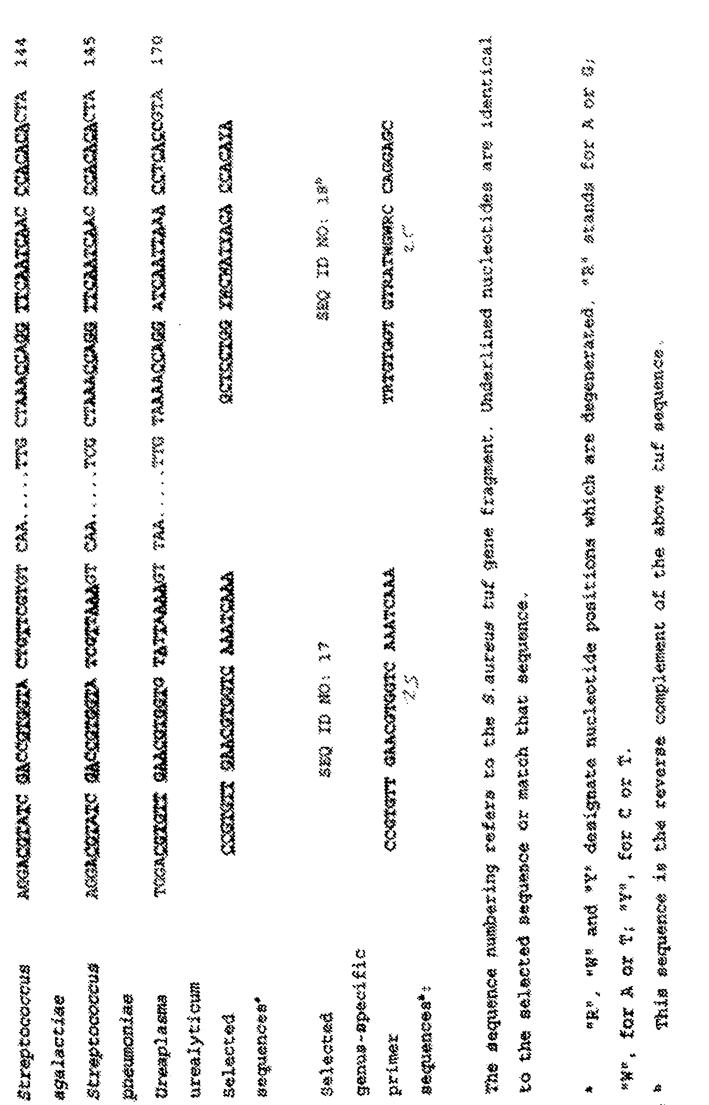

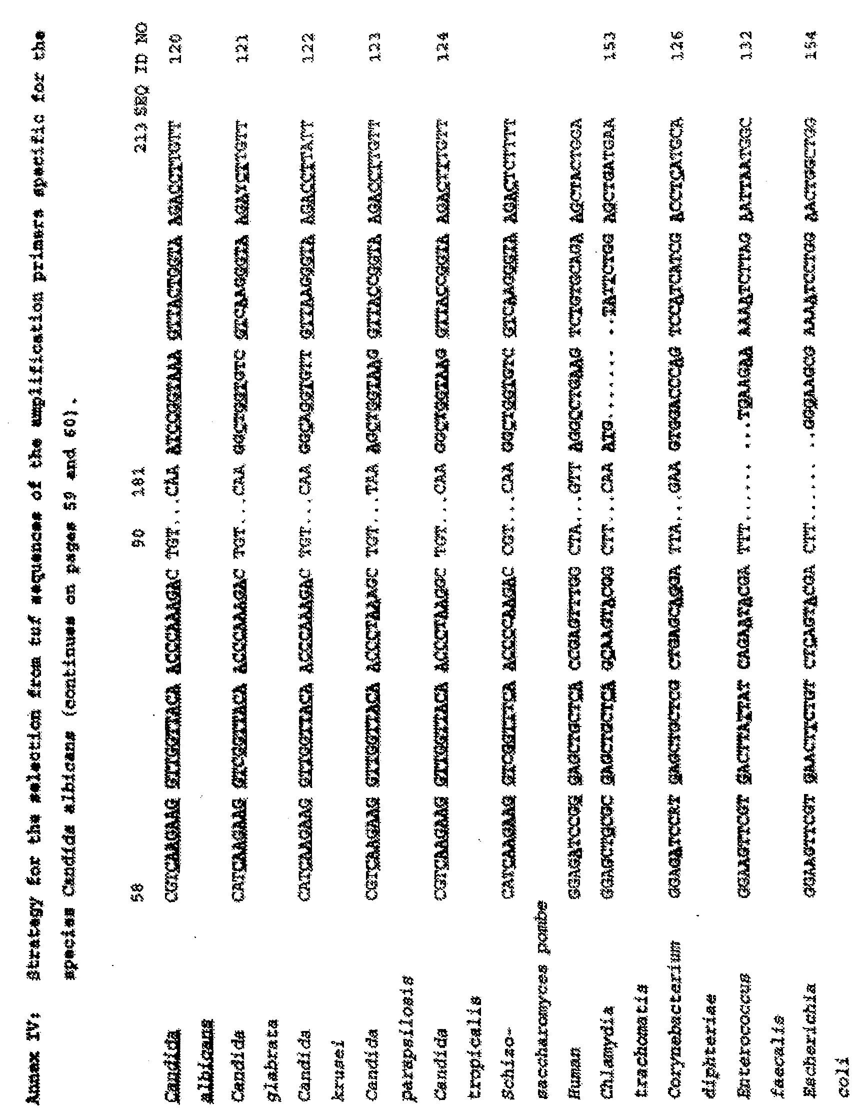

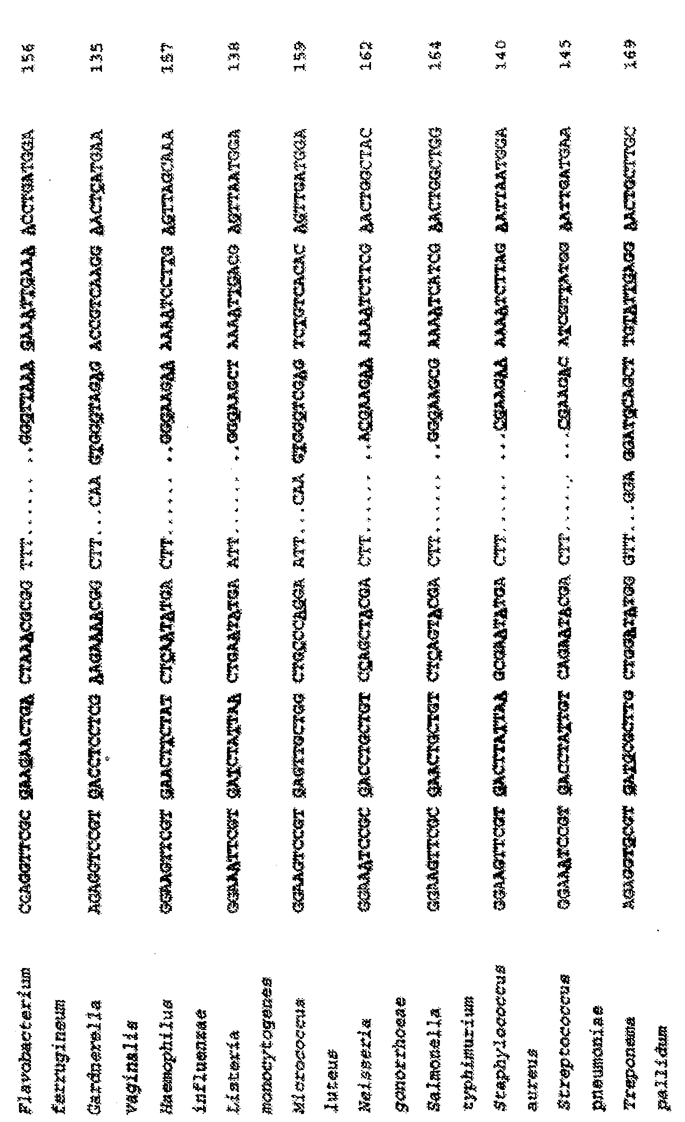

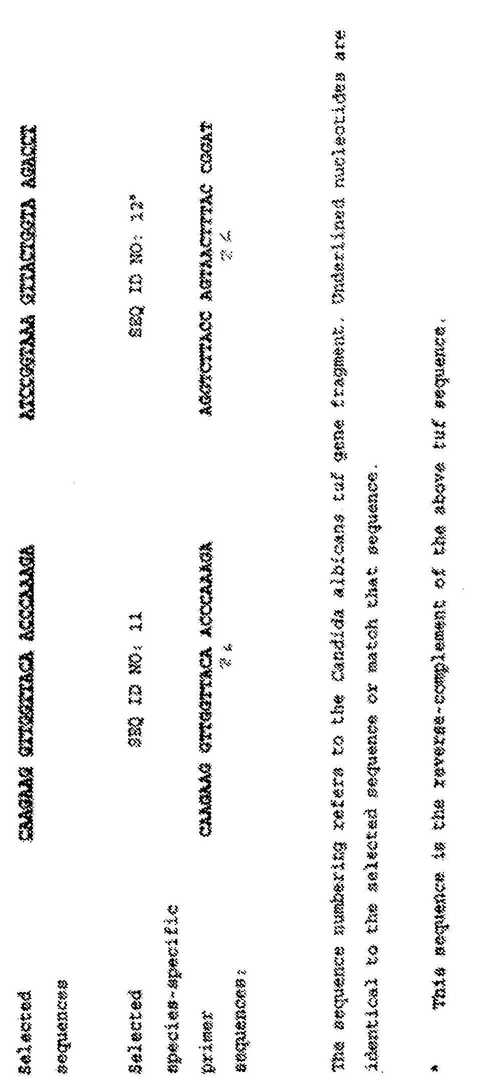

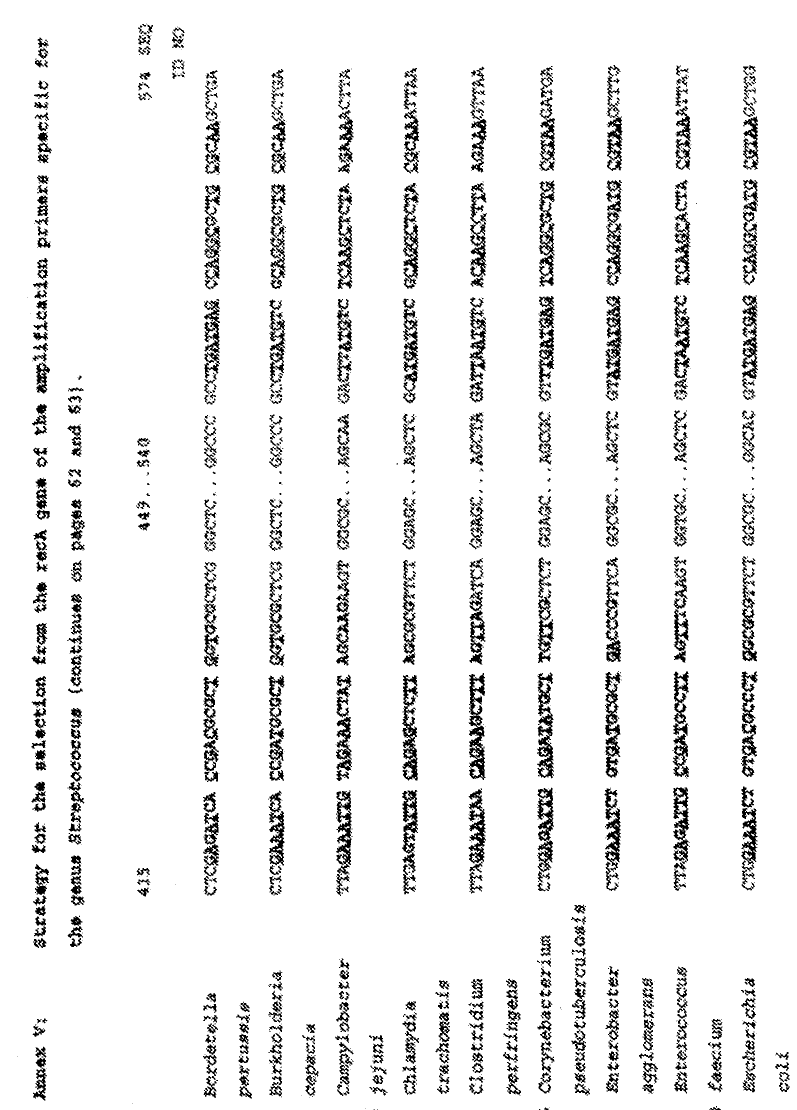

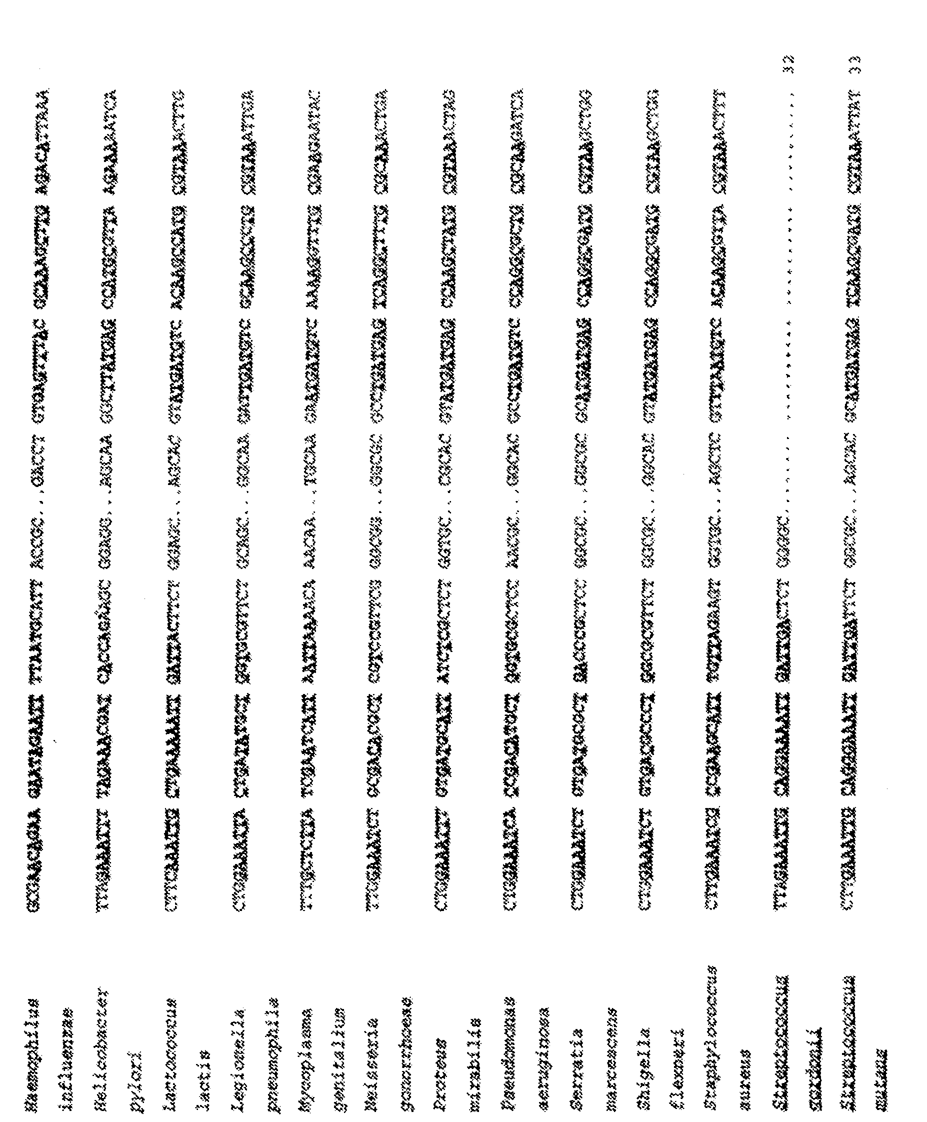

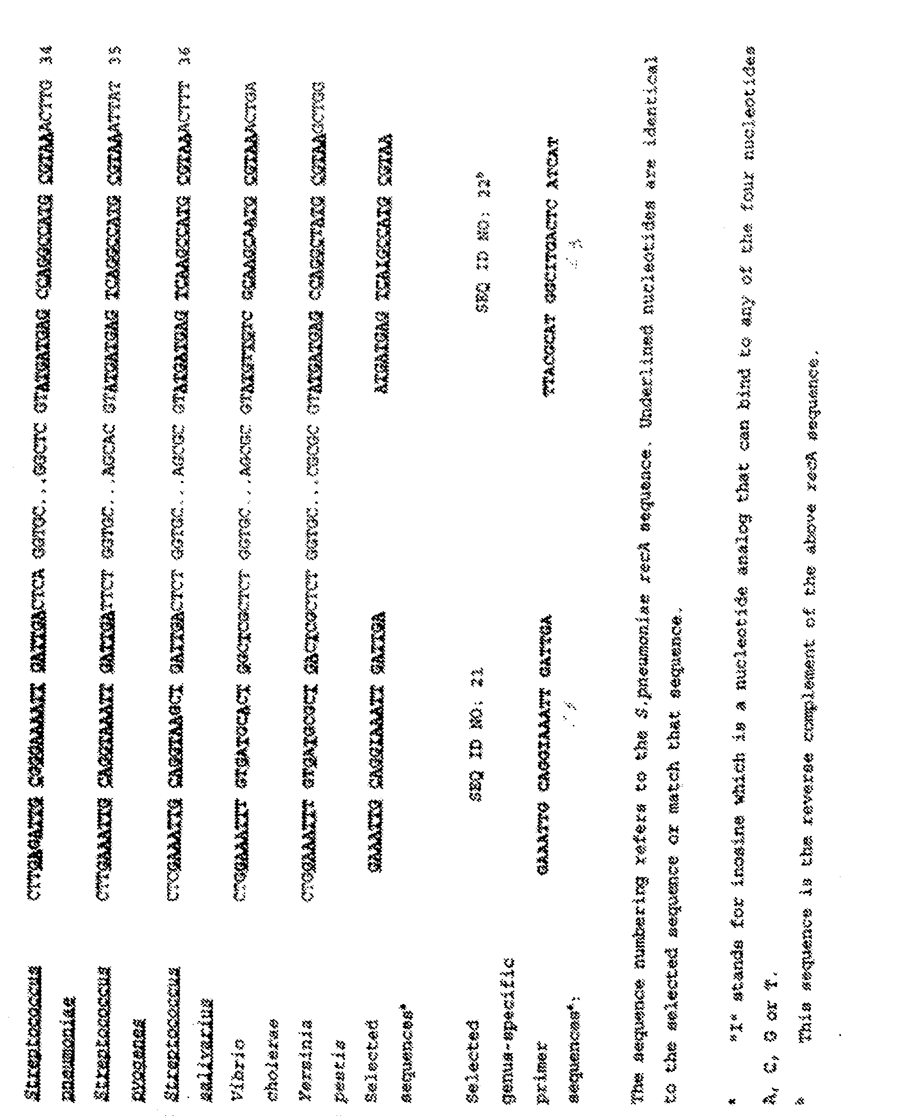

**Annex VI: Specific and ubiquitous primers for DNA amplification**

| SEQ ID NO | Nucleotide sequence | Originating DNA fragment | |
|---|---|---|---|
| | | SEQ ID NO | Nucleotide position |
| Bacterial species: ***Enterococcus faecium*** | | | |
| | | | |
| 1 | 5'-TGC TTT AGC AAC AGC CTA TCA G | 26^{a} | 273-294 |
| 2^{b} | 5'-TAA ACT TCT TCC GGC ACT TCG | 26^{a} | 468-488 |
| | | | |
| Bacterial species: ***Listeria monocytogenes*** | | | |
| | | | |
| 3 | 5'-TGC GGC TAT AAA TGA AGA GGC | 27^{a} | 339-359 |
| 4^{b} | 5'-ATC CGA TGA TGC TAT GGC TTT | 27^{a} | 448-468 |
| | | | |
| Bacterial species: ***Neisseria meningitidis*** | | | |
| | | | |
| 5 | 5'-CCA GCG GTA TTG TTT GGT GGT | 28^{a} | 56-76 |
| 6^{b} | 5'-CAG GCG GCC TTT AAT AAT TTC | 28^{a} | 212-232 |
| | | | |
| Bacterial species: ***Staphylococcus saprophyticus*** | | | |
| | | | |
| 7 | 5'- AGA TCG AAT TCC ACA TGA AGG TTA TTA | TGA 29^{c} | 290-319 |
| 8^{b} | 5'- TCG CTT CTC CCT CAA CAA TCA AAC TAT CCT | 29^{c} | 409-438 |
| | | | |
| Bacterial species: ***Streptococcus agalactiae*** | | | |
| | | | |
| 9 | 5'-TTT CAC CAG CTG TAT TAG AAG TA | 30^{a} | 59-81 |
| 10^{b} | 5'-GTT CCC TGA ACA TTA TCT TTG AT | 30^{a} | 190-212 |
| | | | |
| Fungal species: ***Candida albicans*** | | | |
| | | | |
| 11 | 5'-CAA GAA GGT TGG TTA CAA CCC AAA GA | 120^{c} | 61-86 |
| 12^{b} | 5'-AGG TCT TAC CAG TAA CTT TAC CGG AT | 120^{c} | 184-209 |

| | | | |
|---|---|---|---|
| ^{a} Sequences from databases. ^{b} These sequences are from the opposite DNA strand of the sequence of the originating fragment given in the Sequence Listing. ^{c} Sequences determined by our group. | | | |

**Annex VI: Specific and ubiquitous primers for DNA amplification (continues on next page)**

| SEQ ID NO | Nucleotide sequence | Originating DNA fragment | |
|---|---|---|---|
| | | SEQ ID NO | Nucleotide position |
| Bacterial genus: ***Enterococcus*** | | | |
| 13 | 5'-TAC TGA CAA ACC ATT CAT GAT G | 131-134^{a,b} | 319-340^{c} |
| 14^{d} | 5'-AAC TTC GTC ACC AAC GCG AAC | 131-134^{a,b} | 410-430^{c} |
| | | | |
| Bacterial genus: ***Neisseria*** | | | |
| | | | |
| 15 | 5'-CTG GCG CGG TAT GGT CGG TT | 31^{e} | 21-40^{f} |
| 16^{d} | 5'-GCC GAC GTT GGA AGT GGT AAA G | 31^{e} | 102-123^{f} |
| Bacterial genus: ***Staphylococcus*** | | | |
| | | | |
| 17 | 5'-CCG TGT TGA ACG TGG TCA AAT CAA A | 140-143^{a,b} | 391-415^{g} |
| 18^{d} | 5'-TRT GTG GTG TRA TWG WRC CAG GAG C | 140-143^{a,b} | 584-608^{g} |
| 19 | 5'-ACA ACG TGG WCA AGT WTT AGC WGC T | 140-143^{a,b} | 562-583^{g} |
| 20^{d} | 5'-ACC ATT TCW GTA CCT TCT GGT AAG T | 140-143^{a,b} | 729-753^{g} |
| | | | |
| Bacterial genus: ***Streptococcus*** | | | |
| | | | |
| 21 | 5'-GAA ATT GCA GGI AAA TTG ATT GA | 32-36^{e} | 418-440^{h} |
| 22^{d} | 5'-TTA CGC ATG GCI TGA CTC ATC AT | 32-36^{e} | 547-569^{h} |
| | | | |
| | **Universal primers** | | |
| | | | |
| 23 | 5'-ACI KKI ACI GGI GTI GAR ARG TT | 118-146^{a,b} | 493-515ⁱ |
| | | 147-171^{a,e} | |
| 24^{d} | 5'-AYR TTI TCI CCI GGC ATI ACC AT | 118-146^{a,b} | 778-800ⁱ |
| | | 147-171^{d,e} | |

| | | | |
|---|---|---|---|
| ^{a} These sequences were aligned to derive the corresponding primer. ^{b} *tuf* sequences determined by our group. ^{c} The nucleotide positions refer to the *E. faecalis tuf* gene fragment (SEQ ID NO: 132). ^{d} These sequences arc from the opposite DNA strand of the sequence of the originating fragment given in the Sequence Listing. ^{e} Sequences from databases. ^{t} The nucleotide positions refer to the *N. meningitidis asd* gene fragment (SEQ ID NO: 31). ^{g} The nucleotide positions refer to the *S. aureus tuf* gene fragment (SEQ ID NO: 140). ^{h} The nucleotide positions refer to the *S. pneumoniae recA* gene (SEQ ID NO: 34). ^{l} The nucleotide positions refer to the *E. coli tuf* gene fragment (SEQ ID NO: 154). | | | |

**Annex VI: Specific and ubiquitous primers for DNA amplification**

| SEQ ID NO | Nucleotide sequence | Originating DNA fragment | |
|---|---|---|---|
| | | SEQ ID NO | Nucleotide position |
| Antibiotic resistance gene: ***blaₜₑₘ*** | | | |
| 37 | 5'-CTA TGT GGC GCG GTA TTA TC | - | - |
| 38 | 5'-CGC AGT GTT ATC ACT CAT GG | - | - |
| | | | |
| 39 | 5'-CTG AAT GAA GCC ATA CCA AA | - | - |
| 40 | 5'-ATC AGC AAT AAA CCA GCC AG | - | - |
| | | | |
| Antibiotic resistance gene: ***bla**ₛₕᵥ* | | | |
| | | | |
| 41 | 5'-TTA CCA TGA GCG ATA ACA GC | - | - |
| 42 | 5'-CTC ATT CAG TTC CGT TTC CC | - | - |
| | | | |
| 43 | 5'-CAG CTG CTG CAG TGG ATG GT | - | - |
| 44 | 5'-CGC TCT GCT TTG TTA TTC GG | - | - |
| | | | |
| Antibiotic resistance gene: ***bla**_{rob}* | | | |
| | | | |
| 45 | 5'-TAC GCC AAC ATC GTG GAA AG | - | - |
| 46 | 5'-TTG AAT TTG GCT TCT TCG GT | - | - |
| | | | |
| 47 | 5'-GGG ATA CAG AAA CGG GAC AT | - | - |
| 48 | 5'-TAA ATC TTT TTC AGG CAG CG | - | - |
| | | | |
| Antibiotic resistance gene: ***bla**ₒₓₐ* | | | |
| | | | |
| 49 | 5'-GAT GGT TTG AAG GGT TTA TTA TAA G | 110^{a} | 686-710 |
| 50^{b} | 5'-AAT TTA GTG TGT TTA GAA TGG TGA T | 110^{a} | 802-826 |
| | | | |
| Antibiotic resistance gene: ***blaZ*** | | | |
| | | | |
| 51 | 5'-ACT TCA ACA CCT GCT GCT TTC | 111^{a} | 511-531 |
| 52^{b} | 5'-TGA CCA CTT TTA TCA GCA ACC | 111^{a} | 663-683 |
| | | | |
| Antibiotic resistance gene: ***aadB*** | | | |
| | | | |
| 53 | 5'-GGC AAT AGT TGA AAT GCT CG | - | - |
| 54 | 5'-CAG CTG TTA CAA CGG ACT GG | - | - |
| | | | |
| Antibiotic resistance gene: ***aacC1*** | | | |
| | | | |
| 55 | 5'-TCT ATG ATC TCG CAG TCT CC | - | - |
| 56 | 5'-ATC GTC ACC GTA ATC TGC TT | - | - |

| | | | |
|---|---|---|---|
| ^{a} Sequences from databases. ^{b} These sequences are from the opposite DNA strand of the sequence of the originating fragment given in the Sequence Listing. | | | |

**Annex VI: Specific and ubiquitous primers for DNA amplification**

| SEQ ID NO | Nucleotide sequence | Originating DNA fragment | |
|---|---|---|---|
| | | SEQ ID NO | Nucleotide position |
| Antibiotic resistance gene: ***aacC2*** | | | |
| | | | |
| 57 | 5' -CAT TCT CGA TTG CTT TGC TA | - | - |
| 58 | 5'-CCG AAA TGC TTC TCA AGA TA | - | - |
| | | | |
| Antibiotic resistance gene: ***aacC3*** | | | |
| | | | |
| 59 | 5'-CTG GAT TAT GGC TAC GGA GT | - | - |
| 60 | 5'-AGC AGT GTG ATG GTA TCC AG | - | - |
| | | | |
| Antibiotic resistance gene: ***aac6'-IIa*** | | | |
| | | | |
| 61 | 5'-GAC TCT TGA TGA AGT GCT GG | 112^{a} | 123-142 |
| 62^{b} | 5' -CTG GTC TAT TCC TCG CAC TC | 112^{a} | 284-303 |
| | | | |
| 63 | 5'-TAT GAG AAG GCA GGA TTC GT | 112^{a} | 445-464 |
| 64^{b} | 5'-GCT TTC TCT CGA AGG CTT GT | 112^{a} | 522-541 |
| | | | |
| Antibiotic resistance gene: ***aacA4*** | | | |
| | | | |
| 65 | 5'-GAG TTG CTG TTC AAT GAT CC | - | - |
| 66 | 5'-GTG TTT GAA CCA TGT ACA CG | - | - |
| | | | |
| Antibiotic resistance gene: ***aad(6')*** | | | |
| | | | |
| 173 | 5'-TCT TTA GCA GAA CAG GAT GAA | - | - |
| 174 | 5'-GAA TAA TTC ATA TCC TCC G | - | - |
| | | | |
| Antibiotic resistance gene: ***vanA*** | | | |
| 67 | 5'-TGT AGA GGT CTA GCC CGT GT | - | - |
| 68 | 5'-ACG GGG ATA ACG ACT GTA TG | - | - |
| | | | |
| 69 | 5'-ATA AAG ATG ATA GGC CGG TG | - | - |
| 70 | 5'-TGC TGT CAT ATT GTC TTG CC | - | - |
| | | | |
| Antibiotic resistance gene: ***vanB*** | | | |
| 71 | 5'-ATT ATC TTC GGC GGT TGC TC | 116^{a} | 22-41 |
| 72° | 5'-GAC TAT CGG CTT CCC ATT CC | 116^{a} | 171-190 |
| 73 | 5'-CGA TAG AAG CAG CAG GAC AA | 116^{a} | 575-594 |
| 74^{b} | 5'-CTG ATG GAT GCG GAA GAT AC | 116^{a} | 713-732 |

| | | | |
|---|---|---|---|
| ^{a} Sequences from databases. ^{b} These sequences are from the opposite DNA strand of the sequence of the originating fragment given in the Sequence Listing. | | | |

**Annex VI: Specific and ubiquitous primers for DNA amplification**

| SEQ ID NO | Nucleotide sequence | Originating DNA fragment | |
|---|---|---|---|
| | | SEQ ID NO | Nucleotide position |
| Antibiotic resistance gene: ***vanC*** | | | |
| | | | |
| 75 | 5'-GCC TTA TGT ATG AAC AAA TGG | 117^{a} | 373-393 |
| 76^{b} | 5'-GTG ACT TTW GTG ATC CCT TTT GA | 117^{a} | 541-563 |
| | | | |
| Antibiotic resistance gene: ***msrA*** | | | |
| | | | |
| 77 | 5'-TCCAAT CAT TGC ACA AAA TC | - | - |
| 78 | 5'-AAT TCC CTC TAT TTG GTG GT | - | - |
| | | | |
| 79 | 5'-TCC CAA GCC AGT AAA GCT AA | - | - |
| 80 | 5'-TGG TTT TTC AAC TTC TTC CA | - | - |
| | | | |
| Antibiotic resistance gene: ***satA*** | | | |
| | | | |
| 81 | 5'-TCA TAG AAT GGA TGG CTC AA | - | - |
| 82 | 5'-AGC TAC TAT TGC ACC ATC CC | - | - |
| | | | |
| Antibiotic resistance gene: ***aac(6')-aph(2")*** | | | |
| | | | |
| 83 | 5'-CAA TAA GGG CAT ACC AAA AAT C | - | - |
| 84 | 5'-CCT TAA CAT TTG TGG CAT TAT C | - | - |
| | | | |
| 85 | 5'-TTG GGA AGA TGA AGT TTT TAG A | - | - |
| 86 | 5'-CCT TTA CTC CAA TAA TTT GGC T | - | - |
| | | | |
| Antibiotic resistance gene: ***vat*** | | | |
| | | | |
| 87 | 5'-TTT CAT CTA TTC AGG ATG GG | - | - |
| 88 | 5'-GGA GCA ACA TTC TTT GTG AC | - | - |
| | | | |
| Antibiotic resistance gene: ***vga*** | | | |
| | | | |
| 89 | 5'-TGT GCC TGA AGA AGG TAT TG | - | - |
| 90 | 5'-CGT GTT ACT TCA CCA CCA CT | - | - |
| | | | |
| Antibiotic resistance gene: ***ermA*** | | | |
| | | | |
| 91 | 5'-TAT CTT ATC GTT GAG AAG GGA TT | 113^{a} | 370-392 |
| 92^{b} | 5'-CTA CAC TTG GCT TAG GAT GAA A | 113^{a} | 487-508 |

| | | | |
|---|---|---|---|
| ^{a} Sequences from databases. ^{b} These sequences are from the opposite DNA strand of the sequence of the originating fragment given in the Sequence Listing. | | | |

**Annex VI: Specific and ubiquitous primers for DNA amplification**

| **SEQ ID NO** | **Nucleotide sequence** | **Originating DNA fragment** | |
|---|---|---|---|
| | | **(SEQ ID NO** | **Nucleotide position** |
| Antibiotic resistance gene: ***ermB*** | | | |
| | | | |
| 93 | 5'-CTA TCT GAT TGT TGA AGA AGG ATT | 114^{a} | 366-389 |
| 94^{b} | 5'-GTT TAC TCT TGG TTT AGG ATG AAA | 114^{a} | 484-507 |
| | | | |
| Antibiotic resistance gene: ***ermC*** | | | |
| | | | |
| 95 | 5'-CTT GTT GAT CAC GAT AAT TTC C | 115^{a} | 214-235 |
| 96^{b} | 5'-ATC TTT TAG CAA ACC CGT ATT C | 115^{a} | 382-403 |
| | | | |
| Antibiotic resistance gene: ***mecA*** | | | |
| | | | |
| 97 | 5'-AAC AGG TGA ATT ATT AGC ACT TGT AAG | - | - |
| 98 | 5'-ATT GCT GTT AAT ATT TTT TGA GTT GAA | - | - |
| | | | |
| Antibiotic resistance gene: ***int*** | | | |
| | | | |
| 99 | 5'-GTG ATC GAA ATC CAG ATC C | - | - |
| 100 | 5'-ATC CTC GGT TTT CTG GAA G | - | - |
| | | | |
| 101 | 5'-CTG GTC ATA CAT GTG ATG G | - | - |
| 102 | 5'-GAT GTT ACC CGA GAG CTT G | - | - |
| | | | |
| Antibiotic resistance gene: ***sul*** | | | |
| | | | |
| 103 | 5'-TTA AGC GTG CAT AAT AAG CC | - | - |
| 104 | 5'-TTG CGA TTA CTT CGC CAA CT | - | - |
| | | | |
| 105 | 5'-TTT ACT AAG CTT GCC CCT TC | - | - |
| 106 | 5'-AAA AGG CAG CAA TTA TGA GC | - | - |

| | | | |
|---|---|---|---|
| ^{a} Sequences from databases. ^{b} These sequences are from the opposite DNA strand of the sequence of the originating fragment given in the Sequence Listing. | | | |

In view of the foregoing, it will be appreciated that the invention described herein *inter alia* relates to the following items:
1. A method using probes and/or amplification primers which are specific, ubiquitous and sensitive for determining the presence and/or amount of nucleic acids:
   - from a bacterial antibiotic resistance gene selected from the group consisting of *blaₜₑₘ, blaₛₕᵥ, bla_{rob}, blaₒₓₐ, blaZ, aadB, aacC1, aacC2, aacC3, aac6'-IIa, aacA4, aad(6'), vanA, vanB, vanC, msrA, satA, aac(6')-aph(2"), vat, vga, ermA, ermB, ermC, mecA, int* and *sul,* and
   - from specific bacterial and fungal species selected from the group consisting of *Enterococcus faecium, Listeria monocytogenes, Neisseria meningitidis, Staphylococcus saprophyticus, Streptococcus agalactiae, Candida albicans, Enterococcus* species, *Neisseria* species, *Staphylococcus* species, *Streptococcus* species and *Candida* species,
   in any sample suspected of containing said bacterial and/or fungal nucleic acids,
   wherein each of said nucleic acid or variant or part thereof comprises a selected target region hybridizable with said probes or primers;
   said method comprising the following steps: contacting said sample with said probes or primers and detecting the presence and/or amount of hybridized probes or amplified products as an indication of the presence and/or amount of said specific bacterial and/or fungal species and bacterial antibiotic resistance genes.
2. A method according to item 1, which further makes use of probes and/or primers which are specific, ubiquitous and sensitive for determining the presence and/or amount of nucleic acids from any bacterium or fungus.
3. The method of item 1, which is performed directly from a test sample.
4. The method of item 1, which is performed directly from a test sample consisting of a bacterial and/or fungal culture or suspension.
5. The method of item 1, wherein said nucleic acids are all detected under uniform hybridization or amplification conditions.
6. The method of item 1, wherein said nucleic acids are amplified by a method selected from the group consisting of:
   a) polymerase chain reaction (PCR),
   b) ligase chain reaction (LCR),
   c) nucleic acid sequence-based amplification (NASBA),
   d) self-sustained sequence replication (3SR),
   e) strand displacement amplification (SDA),
   f) branched DNA signal amplification (bDNA),
   g) transcription-mediated amplification (TMA),
   h) cycling probe technology (CPT),
   i) nested PCR, and
   j) multiplex PCR.
7. The method of item 6, wherein said nucleic acids are amplified by PCR.
8. The method of item 7, wherein the PCR protocol achieves within one hour under uniform amplification conditions the determination of the presence of said nucleic acids by performing for each amplification cycle an annealing step of thirty seconds at 45-55°C and a denaturation step of only one second at 95°C without any time specifically allowed to an elongation step.
9. A method for the detection, identification and/or quantification of a microorganism selected from the group consisting of *Enterococcus faecium, Listeria monocytogenes, Neisseria meningitidis, Staphylococcus saprophyticus, Streptococcus agalactiae, Candida albicans, Enterococcus* species, *Neisseria* species, *Staphylococcus* species, *Streptococcus* species and *Candida* species, directly from a test sample or from bacterial and/or fungal cultures, which comprises the following steps:
   a) depositing and fixing on an inert support or leaving in solution the said microorganism DNA of the sample or of a substantially homogeneous population of said microorganism isolated from this sample, or
      inoculating said sample or said substantially homogeneous population of microorganism isolated from this sample on an inert support, and lysing *in situ* said inoculated sample or said isolated microorganism to release the said microorganism DNA,
      said microorganism DNA being made in a substantially single-stranded form;
   b) contacting said single-stranded DNA with a probe, said probe comprising at least one single-stranded nucleic acid which nucleotide sequence is selected from the group consisting of SEQ ID NOs: 26, 27, 28, 29, 30, 120, 131 to 134, 31, 140 to 143, 32 to 36, 120 to 124, a sequence complementary thereof, a part thereof having at least 12 nucleotides in length, and a variant thereof, which specifically and ubiquitously anneals with strains or representatives of *Enterococcus faecium, Listeria monocytogenes, Neisseria meningitidis, Staphylococcus saprophyticus, Streptococcus agalactiae, Candida* albicans, *Enterococcus* species, Neisseria species, *Staphylococcus* species, *Streptococcus* species and *Candida* species, respectively, under conditions such that the nucleic acid of said probe can selectively hybridize with said microorganism DNA, whereby a hybridization complex is formed; and
   c) detecting the presence of said hybridization complex on said inert support or in said solution as an indication of the presence and/or amount of said microorganism, in said test sample.
10. A method for detecting the presence and/or amount of a microorganism selected from the group consisting of *Enterococcus faecium, Listeria monocytogenes, Neisseria meningitidis, Staphylococcus saprophyticus, Streptococcus* agalactiae, *Candida albicans, Enterococcus* species, Neisseria species, *Staphylococcus* species, *Streptococcus* species and *Candida* species, in a test sample which comprises the following steps:
   a) treating said sample with an aqueous solution containing at least one pair of oligonucleotide primers having at least 12 nucleotides in length, one of said primers being capable of hybridizing selectively with one of the two complementary strands of said microorganism DNA that contains a target sequence, and the other of said primers being capable of hybridizing with the other of said strands so as to form an extension product which contains the target sequence as a template, said at least one pair of primers being chosen from a nucleotide sequence within the group consisting of SEQ ID NOs: 26, 27, 28, 29, 30, 120, 131 to 134, 31, 140 to 143, 32 to 36, 120 to 124, respectively with regard to said microorganism, a sequence complementary thereof, and a variant thereof;
   b) synthesizing an extension product of each of said primers, said extension product containing the target sequence, and amplifying said target sequence, if any, to a detectable level; and
   c) detecting the presence and/or amount of said amplified target sequence as an indication of the presence and/or amount of said microorganisms, in said test sample.
11. The method of item 10, wherein said pair of primers is defined in SEQ ID NOs: 1 and 2, 3 and 4, 5 and 6, 7 and 8, 9 and 10, 11 and 12, 13 and 14, 15 and 16, 17 to 20, 21 and 22, respectively, for each of *Enterococcus faecium, Listeria monocytogenes,* Neisseria *meningitidis, Staphylococcus saprophyticus, Streptococcus agalactiae,* Candida *albicans, Enterococcus* species, Neisseria species, Staphylococcus species and *Streptococcus* species.
12. A method for detecting the presence and/or amount of any bacterium directly from a test sample or a bacterial culture, which comprises the following steps:
   a) depositing and fixing on an inert support or leaving in solution the bacterial DNA of the sample or of a substantially homogeneous population of bacteria isolated from this sample, or
      inoculating said sample or said substantially homogeneous population of bacteria isolated from this sample on an inert support, and lysing *in situ* said inoculated sample or isolated bacteria to release the bacterial DNA,
      said bacterial DNA being made in a substantially single-stranded form;
   b) contacting said single-stranded DNA with a probe, said probe comprising at least one single-stranded nucleic acid which nucleotide sequence is selected from the group consisting of SEQ ID NOs: 118, 119, 125 to 171, a sequence complementary thereof, a part thereof having at least 12 nucleotides in length, and a variant thereof, which specifically and ubiquitously anneals with strains or representatives of any bacterial species, under conditions such that the nucleic acid of said probe can selectively hybridize with said bacterial DNA, whereby a hybridization complex is formed; and
   c) detecting the presence of said hybridization complex on said inert support or in said solution as an indication of the presence and/or amount of any bacterium in said test sample.
13. A method for detecting the presence and/or amount of any bacterium in a test sample which comprises the following steps:
   a) treating said sample with an aqueous solution containing at least one pair of oligonucleotide primers having at least 12 nucleotides in length, one of said primers being capable of hybridizing selectively with one of the two complementary strands of any bacterial DNA that contains a target sequence, and the other of said primers being capable of hybridizing with the other of said strands so as to form an extension product which contains the target sequence as a template, said at least one pair of primers being chosen from a nucleotide sequence within the group consisting of SEQ ID NO: 118, 119, 125 to 171, a sequence complementary thereof, and a variant thereof;
   b) synthesizing an extension product of each of said primers, said extension product containing the target sequence, and amplifying said target sequence, if any, to a detectable level; and
   c) detecting the presence and/or amount of said amplified target sequence as an indication of the presence and/or amount of any bacterium in said test sample.
14. The method of item 13, wherein said pair of primers is defined in SEQ ID NOs: 23 and 24.
15. A method for obtaining *tuf* sequences from any bacterium directly from a test sample or a bacterial culture, which comprises the following steps:
   a) treating said sample with an aqueous solution containing a pair of primers having a sequence selected within the nucleotide sequences defined in SEQ ID NOs: 107 and 108, a part thereof having at least 12 nucleotides in length, a sequence complementary thereof, and a variant thereof, one of said primers being capable of hybridizing selectively with one of the two complementary strands of said bacterial *tuf* gene that contains a target sequence, and the other of said primers being capable of hybridizing with the other of said strands so as to form an extension product which contains the target sequence as a template;
   b) synthesizing an extension product of each of said primers, said extension product containing the target sequence, and amplifying said target sequence, if any, to a detectable level; and
   c) detecting the presence and/or amount of said amplified target sequence; and
   d) determining the nucleotide sequence of the said amplified target sequence by using any DNA sequencing method.
16. A method for detecting the presence and/or amount of any fungus directly from a test sample or a fungal culture, which comprises the following steps:
   a) depositing and fixing on an inert support or leaving in solution the fungal DNA of the sample or of a substantially homogeneous population of fungi isolated from this sample, or
      inoculating said sample or said substantially homogeneous population of fungi isolated from this sample on an inert support, and lysing *in situ* said inoculated sample or isolated fungi to release the fungal DNA,
      said fungal DNA being made in a substantially single-stranded form;
   b) contacting said single-stranded DNA with a probe, said probe comprising at least one single-stranded nucleotide sequence selected from the group consisting of SEQ ID NOs: 120 to 124, a sequence complementary thereof, a part thereof having at least 12 nucleotides in length, and a variant thereof, which specifically and ubiquitously anneals with strains or representatives of any fungus, under conditions such that the nucleic acid of said probe can selectively hybridize with said fungal DNA, whereby a hybridization complex is formed; and
   c) detecting the presence of said hybridization complex on said inert support or in said solution as an indication of the presence and/or amount of any fungus in said test sample.
17. A method for detecting the presence and/or amount of any fungus in a test sample which comprises the following steps:
   a) treating said sample with an aqueous solution containing at least one pair of oligonucleotide primers having at least 12 nucleotides in length, one of said primers being capable of hybridizing selectively with one of the two complementary strands of any fungal DNA that contains a target sequence, and the other of said primers being capable of hybridizing with the other of said strands so as to form an extension product which contains the target sequence as a template, said at least one pair of primers being chosen from a nucleotide sequence within the group consisting of SEQ ID NOs: 120 to 124, a sequence complementary thereof, and a variant thereof;
   b) synthesizing an extension product of each of said primers, said extension product containing the target sequence, and amplifying said target sequence, if any, to a detectable level; and
   c) detecting the presence and/or amount of said amplified target sequence as an indication of the presence and/or amount of any fungus in said test sample.
18. A method for obtaining *tuf* sequences from any fungus directly from a test sample or a fungal culture, which comprises the following steps:
   a) treating said sample with an aqueous solution containing a pair of primers having a sequence selected within the nucleotide sequence defined in EQ ID NOs: 109 and 172, a part thereof having at least 12 nucleotides in length, a sequence complementary thereof, and a variant thereof, one of said primers being capable of hybridizing selectively with one of the two complementary strands of said fungal *tuf* gene that contains a target sequence, and the other of said primers being capable of hybridizing with the other of said strands so as to form an extension product which contains the target sequence as a template;
   b) synthesizing an extension product of each of said primers, said extension product containing the target sequence, and amplifying said target sequence, if any, to a detectable level; and
   c) detecting the presence and/or amount of said amplified target sequence; and
   d) determining the nucleotide sequence of the said amplified target sequence by using any DNA sequencing method.
19. A method as defined in item 1, which comprises the evaluation of the presence of a bacterial resistance mediated by a bacterial antibiotic resistance gene selected from the group consisting of *blaₒₓₐ, blaZ, aac6'-IIa, ermA, ermB, ermC, vanB, vanC,* directly from a test sample or a bacterial culture, which comprises the following steps:
   a) depositing and fixing on an inert support or leaving in solution the bacterial DNA of the sample or of a substantially homogeneous population of bacteria isolated from this sample, or
      inoculating said sample or said substantially homogeneous population of bacteria isolated from this sample on an inert support, and lysing *in situ* said inoculated sample or isolated bacteria to release the bacterial DNA,
      said bacterial DNA being made in a substantially single-stranded form;
   b) contacting said single-stranded DNA with a probe, said probe comprising at least one single-stranded nucleotide sequence having at least 12 nucleotide in length is selected from the group consisting of SEQ ID NOs: 110, 111, 112, 113, 114 115, 116, 117, a sequence complementary thereof, and a variant thereof, which specifically hybridizes with said bacterial antibiotic resistance gene, respectively; and
   c) detecting the presence of a hybridization complex as an indication of a bacterial resistance mediated by said one of said bacterial antibiotic resistance genes.
20. A method as defined in item 1, which comprises the evaluation of the presence of a bacterial resistance mediated by a bacterial antibiotic resistance gene selected from the group consisting of blaₒₓₐ, blaZ, *aac6'-IIa, ermA, ermB, ermC, vanB, vanC,* directly from a test sample or a bacterial culture, which comprises the following steps:
   a) treating said sample with an aqueous solution containing at least one pair of primers having at least 12 nucleotides in length, one of said primers being capable of hybridizing selectively with one of the two complementary strands of said bacterial antibiotic resistance gene that contains a target sequence, and the other of said primers being capable of hybridizing with the other of said strands so as to form an extension product which contains the target sequence as a template, said at least one pair of primers being chosen from a nucleotide sequence within the group consisting of SEQ ID NOs: 110, 111, 112, 113, 114, 115, 116, 117, respectively with regard to said bacterial antibiotic resistance gene, a sequence complementary thereof, and a variant thereof;
   b) synthesizing an extension product of each of said primers, said extension product containing the target sequence, and amplifying said target sequence, if any, to a detectable level; and
   c) detecting the presence and/or amount of said amplified target sequence as an indication of a bacterial resistance mediated by one of said bacterial antibiotic resistance genes.
21. A method as defined in item 1, which comprises the evaluation of the presence of a bacterial resistance gene selected from the group consisting of *blaₜₑₘ, blaₛₕᵥ, bla_{rob}, blaₒₓₐ, blaZ, aadB, aacC1, aacC2, aacC3, aac6'-IIa, aacA4, aad(6), vanA, vanB, vanC, msrA, satA, aac(6')-aph(2''), vat, vga, ermA, ermB, ermC, mecA, int* and *sul,* directly from a test sample or a bacterial culture, which comprises the following steps:
   a) treating said sample with an aqueous solution containing at least one pair of primers having a sequence selected in the group consisting of SEQ ID NOs: 37 to 40, 41 to 44, 45 to 48, 49 and 50, 51 and 52, 53 and 54, 55 and 56, 57 and 58, 59 to 60, 61 to 64, 65 and 66, 173 and 174, 67 to 70, 71 to 74, 75 and 76, 77 to 80, 81 and 82, 83 to 86, 87 and 88, 89 and 90, 91 and 92, 93 and 94, 95 and 96, 97 and 98, 99 to 102, 103 to 106, a part thereof having at least 12 nucleotides in length, a sequence complementary thereof, a variant thereof, and mixtures thereof, one of said primers of said pair being capable of hybridizing selectively with one of the two complementary strands of its respective bacterial antibiotic resistance gene that contains a target sequence, and the other of said primers of said pairs being capable of hybridizing with the other of said strands so as to form an extension product which contains the target sequence as a template;
   b) synthesizing an extension product of each of said primers, said extension product containing the target sequence, and amplifying said target sequence, if any, to a detectable level; and
   c) detecting the presence and/or amount of said amplified target sequence as an indication of a bacterial resistance mediated by one of said bacterial antibiotic resistance genes.
22. A nucleic acid having the nucleotide sequence of any one of SEQ ID NOs: 26 to 36, 110 to 171, a part thereof, a sequence complementary thereof, and variant thereof which, when in single-stranded form, ubiquitously and specifically hybridizes with a target bacterial or fungal DNA as a probe or as a primer.
23. An oligonucleotide having the nucleotide sequence of any one of SEQ ID NOs: 1 to 25, 37 to 109, 172 to 174, a part thereof, a sequence complementary thereof, and variant thereof, which ubiquitously and specifically hybridizes with a target bacterial or fungal DNA as a probe or as a primer.
24. A recombinant plasmid comprising a nucleic acid as defined in item 22.
25. A recombinant host which has been transformed by a recombinant plasmid according to item 24.
26. A recombinant host according to item 25 wherein said host is *Escherichia coli.*
27. A diagnostic kit for the detection and/or quantification of the nucleic acids of any combination of the microbial species and/or genera selected from the group consisting of *Enterococcus faecium, Listeria monocytogenes, Neisseria meningitidis, Staphylococcus saprophyticus, Streptococcus agalactiae, Candida albicans, Enterococcus* species, *Neisseria* species, Staphylococcus species, Streptococcus species and *Candida* species, comprising any suitable combination of probes of at least 12 nucleotides in length selected from the group consisting of SEQ ID NOs: 26 to 36, 120 to 124, 131 to 134, 140 to 143, sequences complementary thereof, and variants thereof.
28. A diagnostic kit for the detection and/or quantification of the nucleic acids of any combination of the microbial species and/or genera selected from the group consisting of *Enterococcus faecium, Listeria monocytogenes, Neisseria meningitidis, Staphylococcus saprophyticus, Streptococcus agalactiae, Candida albicans, Enterococcus* species, Neisseria species, *Staphylococcus* species, *Streptococcus* species and *Candida* species, comprising any suitable combination of primers of at least 12 nucleotides in length selected from the group consisting of SEQ ID NOs: 26 to 36, 120 to 124, 131 to 134, 140 to 143, sequences complementary thereof, and variants thereof.
29. A diagnostic kit for the detection and/or quantification of the nucleic acids of any combination of the microbial species and/or genera selected from the group consisting of *Enterococcus faecium, Listeria monocytogenes, Neisseria meningitidis, Staphylococcus saprophyticus, Streptococcus agalactiae, Candida albicans, Enterococcus* species, *Neisseria* species, *Staphylococcus* species and *Streptococcus* species, comprising any suitable combination of primers selected from the group consisting of SEQ ID NOs: 1 to 22, parts thereof having at least 12 nucleotides in length, sequences complementary thereof, and variants thereof.
30. A diagnostic kit for the detection and/or quantification of the nucleic acids of any combination of the bacterial resistance genes selected from the group consisting of *blaₒₓₐ, blaZ, aac6'-IIa, ermA, ermB, ermC, vanB, vanC,* comprising any suitable combination of probes of at least 12 nucleotides in length selected from the group consisting of SEQ ID NOs: 110 to 117, sequences complementary thereof, and variants thereof.
31. A diagnostic kit for the detection and/or quantification of the nucleic acids of any combination of the bacterial resistance genes selected from the group consisting of *blaₒₓₐ, blaZ,* aac6'-IIa, *ermA, ermB, ermC, vanB, vanC,* comprising any suitable combination of primers of at least 12 nucleotides in length selected from the group consisting of SEQ ID NOs: 110 to 117, sequences complementary thereof, and variants thereof.
32. A diagnostic kit for the detection and/or quantification of the nucleic acids of any combination of the bacterial resistance genes selected from the group consisting of *blaₜₑₘ*, *blaₛₕᵥ*, *bla_{rob}*, *blaₒₓₐ, blaZ, aadB, aacC1, aacC2, aacC3*, *aac6'-IIa, aacA4, aad(6'), vanA, vanB, vanC, msrA, satA, aac(6')-aph(2"), vat, vga, ermA, ermB, ermC, mecA, int* and *sul,* comprising any suitable combination of primers selected from the group consisting of SEQ ID NOs: 37 to 106, 173 and 174, a part thereof having at least 12 nucleotides in length, sequences complementary thereof, and variants thereof.
   A diagnostic kit for the detection and/or quantification of the nucleic acids of any bacterium and/or fungus, comprising any combination of probes of at least 12 nucleotides in length selected from the group consisting of SEQ ID NOs: 118 to 171, sequences complementary thereof, and variants thereof.
34. A diagnostic kit for the detection and/or quantification of the nucleic acids of any bacterium and/or fungus, comprising any suitable combination of primers of at least 12 nucleotides in length selected from the group consisting of SEQ ID NOs: 118 to 171, sequences complementary thereof, and variants thereof.
35. A diagnostic kit for the detection and/or quantification of the nucleic acids of any bacterium, comprising a pair of primers having a sequence selected within the nucleotide sequence defined in SEQ ID NOs: 23 and 24, parts thereof having at least 12 nucleotides in length, sequences complementary thereof, and variants thereof.
36. A diagnostic kit, as defined in item 27, further comprising any combination of probes of at least 12 nucleotides in length selected within a nucleotide sequence from the group consisting of SEQ ID NOs: 118 to 171, sequences complementary thereof, and variants thereof, for the simultaneous detection and/or quantification of nucleic acids of any bacterium and/or fungus.
37. A diagnostic kit, as defined in item 28, further comprising any suitable combination of primers of at least 12 nucleotides in length selected within a nucleotide sequence from the group consisting of SEQ ID NOs: 118 to 171, sequences complementary thereof, and variants thereof, for the simultaneous detection and/or quantification of nucleic acids of any bacterium and/or fungus.
38. A diagnostic kit, as defined in item 29, further comprising a pair of primers having a sequence selected within the nucleotide sequence defined in SEQ ID NOs: 23 and 24, parts thereof having at least 12 nucleotides in length, sequences complementary thereof, and variants thereof, for the simultaneous detection and/or quantification of nucleic acids of any bacterium.
39. A diagnostic kit, as defined in item 27, further comprising any combination of probes of at least 12 nucleotides in length selected within a nucleotide sequence from the group consisting of SEQ ID NOs: 110 to 117, sequences complementary thereof, and variants thereof, for the simultaneous detection and/or quantification of nucleic acids of any bacterial antibiotic resistance gene selected from the group consisting of *blaₒₓₐ*, *blaZ, aac6'-IIa*, *ermA, ermB, ermC, vanB, vanC.*
40. A diagnostic kit, as defined in item 28, further comprising any suitable combination of primers of at least 12 nucleotides in length selected within a nucleotide sequence from the group consisting of SEQ ID NOs: 110 to 117, sequences complementary thereof, and variants thereof, for the simultaneous detection and/or quantification of nucleic acids of any bacterial antibiotic resistance gene selected from the group consisting of *blaₒₓₐ*, *blaZ*, *aac6'-IIa*, *ermA, ermB, ermC, vanB, vanC.*
41. A diagnostic kit, as defined in item 29, further comprising any suitable combination of primers of at least 12 nucleotides in length selected within a nucleotide sequence from the group consisting of SEQ ID NOs: 37 to 106, 173 and 174, sequences complementary thereof, and variants thereof, for the simultaneous detection and/or quantification of nucleic acids of any bacterial antibiotic resistance gene selected from the group consisting of *blaₜₑₘ, bla_{rob}*, *blaₛₕᵥ blaₒₓₐ blaZ, aadB*, *aacC1*, *aacC2, aacC3*, *aacA4, aac6'-IIa*, *aad(6'), ermA, ermB, ermC, mecA, vanA, vanB, vanC, satA, aac(6')-aph(2''), vat, vga, msrA, sul* and *int.*
42. A diagnostic kit, as defined in item 30, further comprising any combination of probes of at least 12 nucleotides in length selected within a nucleotide sequence from the group consisting of SEQ ID NOs: 118 to 171, sequences complementary thereof, and variants thereof, for the simultaneous detection and/or quantification of nucleic acids of any bacterium and/or fungus.
43. A diagnostic kit, as defined in item 31, further comprising any suitable combination of primers of at least 12 nucleotides in length selected within a nucleotide sequence from the group consisting of SEQ ID NOs: 118 to 171, sequences complementary thereof, and variants thereof, for the simultaneous detection and/or quantification of nucleic acids of any bacterium and/or fungus.
44. A diagnostic kit, as defined in item 32, further comprising a pair of primers having a sequence selected within the nucleotide sequence defined in SEQ ID NOs: 23 and 24, parts thereof having at least 12 nucleotides in length, sequences complementary thereof, and variants thereof, for the simultaneous detection and/or quantification of nucleic acids of any bacterium.
45. A diagnostic kit, as defined in item 39, further comprising any combination of probes of at least 12 nucleotides in length selected within a nucleotide sequence from the group consisting of SEQ ID NOs: 118 to 171, sequences complementary thereof, and variants thereof, for the simultaneous detection and/or quantification of nucleic acids of any bacterium and/or fungus.
46. A diagnostic kit, as defined in item 40, further comprising any suitable combination of primers of at least 12 nucleotides in length selected within a nucleotide sequence from the group consisting of SEQ ID NOs: 118 to 171, sequences complementary thereof, and variants thereof, for the simultaneous detection and/or quantification of nucleic acids of any bacterium and/or fungus.
47. A diagnostic kit, as defined in item 41, further comprising a pair of primers having a sequence selected within the nucleotide sequence defined in SEQ ID NOs: 23 and 24, parts thereof having at least 12 nucleotides in length, sequences complementary thereof, and variants thereof, for the simultaneous detection and/or quantification of nucleic acids of any bacterium.

## Claims

1. A method using at least one oligonucleotide for determining or detecting the presence and/or amount of one or more nucleic acids from the *Streptococcus agalactiae* bacterial species and/or *Streptococcus* genus in a sample, said method comprising:
a) contacting said sample with said at least one oligonucleotide which specifically hybridizes to:
i) SEQ ID NO: 30, 144 or complementary sequences thereof, for determining or detecting the presence and/or amount of one or more nucleic acids from the *Streptococcus agalactiae* species; and/or
ii) each of the *Streptococcus* nucleotide sequences defined in the group consisting of SEQ ID NOs: 32-36, 144-146 or complementary sequences thereof, for determining or detecting the presence or amount of one or more nucleic acids from members of the *Streptococcus* genus, said genus comprising: *S. agalactiae, S. anginosus, S. bovis, S. constellatus, S. crista, S. dysgalactiae, S. equi, S. gordonii, S. intermedius, S. mitis, S. mutans, S. oralis, S. parasanguis, S. pneumoniae, S. pyogenes, S. salivarius, S. sanguis, S. sobrinus, S. suis, S. uberis, S. vestibularis and S. viridans,*
so as to perform an amplification reaction or an hybridization assay; and
b) detecting the presence, amount or both of hybridized oligonucleotides or amplified products in a) as an indication of the presence, amount or both of said one or more nucleic acids.

2. The method of claim 1, wherein said oligonucleotide hybridizes in ii) to each of the *Streptococcus* nucleotide sequences defined in the group consisting of SEQ ID NOs: 33-36.

3. The method of claim 1 or 2, wherein said at least one oligonucleotide in i) hybridizes to SEQ ID NO: 30 and comprises a nucleotide sequence selected from the group consisting of SEQ ID NOs: 9 and 10, parts thereof or complementary sequences thereof, for determining the presence and/or amount of one or more nucleic acids from the *Streptococcus agalactiae* species.

4. The method of claim 2, wherein said at least one oligonucleotide in ii) comprises a nucleotide sequence selected from the group consisting of SEQ ID NOs: 21 and 22, parts thereof or complementary sequences thereof, for determining the presence and/or amount of one or more nucleic acids from one or more members of the *Streptococcus* genus.

5. The method of any one of claims 1-4, further comprising using at least one oligonucleotide for determining the presence and/or amount of one or more nucleic acids from a bacterial and/or fungal species and/or genuses selected from the group consisting of *Enterococcus faecium, Listeria monocytogenes, Neisseria meningitidis, Staphylococcus saprophyticus, Candida albicans,* one or more members of the *Enterococcus* genus, one or more members of the *Neisseria* genus, one or more members of the *Staphylococcus* genus and one or more members of the *Candida* genus, wherein said at least one oligonucleotide for determining the presence and/or amount of one or more nucleic acids from bacterial and fungal species hybridizes specifically to one and only one of the nucleotide sequences selected from the group consisting of:
SEQ ID NO: 26 or a complementary sequence thereof, for determining the presence or amount of one or more nucleic acids from *Enterococcus faecium;*
SEQ ID NO: 27 or a complementary sequence thereof, for determining the presence or amount of one or more nucleic acids from *Listeria monocytogenes;*
SEQ ID NO: 28 or a complementary sequence thereof, for determining the presence or amount of one or more nucleic acids from *Neisseria meningitidis;*
SEQ ID NO: 29 or a complementary sequence thereof, for determining the presence or amount of one or more nucleic acids from *Staphylococcus saprophyticus;* and
SEQ ID NO: 120 or a complementary sequence thereof, for determining the presence or amount of one or more nucleic acids from *Candida albicans;*
or wherein said at least one oligonucleotide for determining the presence and/or amount of one or more nucleic acids from a bacterial and/or fungal genus hybridizes specifically to at least 12 nucleotides of one and only one of the genus-specific group of nucleotide sequences selected from the group consisting of:
SEQ ID NOs: 131-134 or complementary sequences thereof, for determining the presence or amount of one or more nucleic acids from one or more members of the *Enterococcus* genus;
SEQ ID NO: 31 or complementary sequences thereof, for determining the presence or amount of one or more nucleic acids from one or more members of the *Neisseria* genus;
SEQ ID NO: 140-143 or complementary sequences thereof, for determining the presence or amount of one or more nucleic acids from one or more members of the *Staphylococcus* genus; and
SEQ ID NOs: 120-124 or complementary sequences thereof, for determining the presence or amount of one or more nucleic acids from one or more members of the *Candida* genus.

6. The method of claim 5, wherein the presence and/or amount of said one or more nucleic acids from bacterial and fungal species and/or genuses is determined using at least one oligonucleotide comprising a nucleotide sequence selected from the group consisting of:
SEQ ID NOs: 1 and 2, parts thereof or complementary sequences thereof, for determining the presence or amount of one or more nucleic acids from *Enterococcus faecium;*
SEQ ID NOs: 3 and 4, parts thereof or complementary sequences thereof, for determining the presence or amount of one or more nucleic acids from *Listeria monocytogenes;*
SEQ ID NOs: 5 and 6, parts thereof or complementary sequences thereof, for determining the presence or amount of one or more nucleic acids from *Neisseria meningitidis;*
SEQ ID NOs: 7 and 8, parts thereof or complementary sequences thereof, for determining the presence or amount of one or more nucleic acids from *Staphylococcus saprophyticus;*
SEQ ID NOs: 11 and 12, parts thereof or complementary sequences thereof, for determining the presence or amount of one or more nucleic acids from *Candida albicans;*
SEQ ID NOs: 13 and 14, parts thereof or complementary sequences thereof, for determining the presence or amount of one or more nucleic acids from one or more members of the *Enterococcus* genus;
SEQ ID NOs: 15 and 16, parts thereof or complementary sequences thereof, for determining the presence or amount of one or more nucleic acids from one or more members of the *Neisseria* genus; and
SEQ ID NOs: 17-20, parts thereof or complementary sequences thereof, for determining the presence or amount of one or more nucleic acids from one or more members of the *Staphylococcus* genus.

7. The method of any one of claims 1-6, further comprising using at least one oligonucleotide for determining the presence and/or amount of one or more nucleic acids from one or more bacterial antibiotic resistance genes selected from the group consisting of *blaₜₑₘ, blaₛₕᵥ, bla_{rob}, blaₒₓₐ, blaZ, aadB, aacC1, aacC2*, *aacC3*, *aac6*'-*IIa*, *aacA4, aad(6'), vanA, vanB, vanC, msrA, satA, aac(6')-aph(2"), vat, vga, ermA, ermB, ermC, mecA, int* and *sul.*

8. The method of claim 7, wherein said one or more bacterial antibiotic resistance genes are selected from the group consisting of *blaₒₓₐ, blaZ, aac6'-IIa*, *vanB, vanC, ermA, ermB and ermC,* and wherein said at least one oligonucleotide for determining the presence of a nucleic acid from one or more bacterial antibiotic resistance genes hybridizes to one or more of the nucleotide sequences selected from the group consisting of:
SEQ ID NO: 110 or a complementary sequence thereof for the detection of *blaₒₓₐ*;
SEQ ID NO: 111 or a complementary sequence thereof for the detection of *blaZ;*
SEQ ID NO: 112 or a complementary sequence thereof for the detection of *aac6*'-*IIa*;
SEQ ID NO: 113 or a complementary sequence thereof for the detection of *ermA;*
SEQ ID NO: 114 or a complementary sequence thereof for the detection of *ermB;*
SEQ ID NO: 115 or a complementary sequence thereof for the detection of *ermC;*
SEQ ID NO: 116 or a complementary sequence thereof for the detection of *vanB;* and
SEQ ID NO: 117 or a complementary sequence thereof for the detection of *vanC.*

9. The method of claim 7, wherein said at least one oligonucleotide for determining the presence and/or amount of one or more nucleic acids from one or more bacterial antibiotic resistance genes comprises a nucleotide sequence selected from the group consisting of:
SEQ ID NOs: 37-40, parts thereof or complementary sequences thereof for the detection of *blaₜₑₘ;*
SEQ ID NOs: 41-44, parts thereof or complementary sequences thereof for the detection of *bla*_{*shv*;}
SEQ ID NOs: 45-48, parts thereof or complementary sequences thereof for the detection of *bla_{rob}*;
SEQ ID NOs: 49-50, parts thereof or complementary sequences thereof for the detection of *blaₒₓₐ;*
SEQ ID NOs: 51-52, parts thereof or complementary sequences thereof for the detection of *blaZ;*
SEQ ID NOs: 53-54, parts thereof or complementary sequences thereof for the detection of *aad*B;
SEQ ID NOs: 55-56, parts thereof or complementary sequences thereof for the detection of *aac*C1;
SEQ ID NOs: 57-58, parts thereof or complementary sequences thereof for the *detection of aac*C2;
SEQ ID NOs: 59-60, parts thereof or complementary sequences thereof for the detection of *aac*C3;
SEQ ID NOs: 61-64, parts thereof or complementary sequences thereof for the detection of *aac6'-IIa.;*
SEQ ID NOs: 65-66, parts thereof or complementary sequences thereof for the detection of *aacC4*;
SEQ ID NOs: 67-70, parts thereof or complementary sequences thereof for the detection of *vanA;*
SEQ ID NOs: 71-74, parts thereof or complementary sequences thereof for the detection of *vanB;*
SEQ ID NOs: 75-76, parts thereof or complementary sequences thereof for the detection of *vanC.*
SEQ ID NOs: 77-80, parts thereof or complementary sequences thereof for the detection of *msr*A*;*
SEQ ID NOs: 81-82, parts thereof or complementary sequences thereof for the detection of *satA;*
SEQ ID NOs: 83-86, parts thereof or complementary sequences thereof for the detection of *aac(6')-aph(2");*
SEQ ID NOs: 87-88, parts thereof or complementary sequences thereof for the detection of *vat;*
SEQ ID NOs: 89-90, parts thereof or complementary sequences thereof for the detection of *vga;*
SEQ ID NOs: 91-92, parts thereof or complementary sequences thereof for the detection of *ermA;*
SEQ ID NOs: 93-94, parts thereof or complementary sequences thereof for the detection of *ermB;*
SEQ ID NOs: 95-96, parts thereof or complementary sequences thereof for the detection of *ermC;*
SEQ ID NOs: 97-98, parts thereof or complementary sequences thereof for the detection of *mecA*;
SEQ ID NOs: 99-102, parts thereof or complementary sequences thereof for the detection of *int;* and
SEQ ID NOs: 103-106, parts thereof or complementary sequences thereof for the detection of *sul.*

10. A method according to any one of claims 1-9, wherein said determining is performed simultaneously.

11. The method according to any one of claims 1-10, which is performed directly from a test sample.

12. The method according to any one of claims 1-11, which is performed directly from a test sample consisting of a bacterial culture or suspension.

13. The method according to any one of claims 1-12, wherein said nucleic acids are amplified by a method selected from the group consisting of:
a) polymerase chain reaction (PCR),
b) ligase chain reaction (LCR),
c) nucleic acid sequence-based amplification (NASBA),
d) self-sustained sequence replication (3SR),
e) strand displacement amplification (SDA),
f) branched DNA signal amplification (bDNA),
g) transcription-mediated amplification (TMA),
h) cycling probe technology (CPT),
i) nested PCR, and
j) multiplex PCR.

14. The method of claim 13, wherein said nucleic acids are amplified by PCR.

15. The method of claim 14, wherein the amplification conditions are uniform.

16. The method of any one of claims 1-10, further comprising:
a)
i) depositing and fixing on an inert support or leaving in solution said one or more nucleic acids from bacteria of said sample, or
ii) inoculating said sample on an inert support, and lysing *in situ* said inoculated sample to release said one or more nucleic acids in said sample,
said one or more nucleic acids being in a substantially single-stranded form;
b) contacting said substantially single-stranded one or more nucleic acids with an oligonucleotide probe which specifically hybridizes to:
i) a bacterial nucleotide sequence defined in SEQ ID NOs: 30, 144 or complementary sequences thereof; or
ii) each of the bacterial nucleotide sequences defined in the group consisting of SEQ ID NOs: 32-36, 144-146 or complementary sequences thereof, whereby a hybridization complex is formed; and
c) detecting the presence of said hybridization complex on said inert support or in said solution as an indication of the presence and/or amount of said one or more nucleic acids from bacteria in said sample.

17. The method of any one of claims 1-15, wherein said at least one oligonucleotide comprises a pair of primers consisting of SEQ ID NOs: 9 and 10 or complementary sequences thereof for the specific detection of the *Streptococcus agalactiae* species.

18. The method of any one of claims 1-15, wherein said at least one oligonucleotide comprises a pair of primers consisting of SEQ ID NOs: 21 and 22 or complementary sequences thereof for the specific detection of bacteria of the *Streptococcus* genus.

19. The method of any one of claims 1-15, 17 and 18, further comprising the use of a primer pair for the detection of bacterial or fungal nucleic acids.

20. The method of claim 19, wherein said primer pair is selected from the group consisting of SEQ ID NOs: 23 and 24, 107 and 108, and 109 and 172.

21. An isolated oligonucleotide having the nucleotide sequence defined in:
a) SEQ ID NO: 9 or 10, a part thereof, a complementary sequence thereof, or variant thereof, which specifically hybridizes to SEQ ID NO: 30 or a complementary sequence thereof, for determining the presence and/or amount of one or more nucleic acids from the *Streptococcus agalactiae* species; or
b) SEQ ID NO: 21 or 22, a part thereof, a complementary sequence thereof, or variant thereof, which specifically hybridizes to each of the *Streptococcus* nucleotide sequences defined in the group consisting of SEQ ID NOs: 33-35 , a part thereof or complementary sequences thereof, for determining the presence or amount of one or more nucleic acids from one or more members of the *Streptococcus* genus comprising *S. agalactiae, S. anginosus, S. bovis, S. constellatus, S. crista, S. dysgalactiae, S. equi, S. gordonii, S. intermedius, S. mitis, S. mutans, S. oralis, S. parasanguis, S. pneumoniae, S. pyogenes, S. salivarius, S*. *sanguis, S. sobrinus, S*. *suis, S. uberis, S. vestibularis and S. viridans.*

22. An isolated oligonucleotide which specifically hybridizes to:
a) SEQ ID NO:144, a complementary sequence thereof for determining the presence and/or amount of one or more nucleic acids from *Streptococcus agalactiae;*
b) SEQ ID NO:145, or a complementary sequence thereof, for determining the presence and/or amount of one or more nucleic acids from *Streptococcus pneumoniae; or*
c) SEQ ID NO:146, or a complementary sequence thereof, for determining the presence and/or amount of one or more nucleic acids from *Streptococcus salivarius.*

23. A recombinant plasmid comprising the isolated oligonucleotide of claim 21 or 22.

24. A recombinant host which has been transformed by the recombinant plasmid of claim 23.

25. The recombinant host of claim 24, wherein said host is *Escherichia coli.*

26. A diagnostic kit for determining or detecting the presence and/or amount of one or more nucleic acids from the *Streptococcus agalactiae* bacterial species and/or *Streptococcus* genus in a sample, comprising any suitable combination of genus-specific and/or species-specific probes and/or primers which specifically hybridize to:
a) each of the bacterial nucleotide sequences defined in the group consisting of SEQ ID NOs: 32-36 or complementary sequences thereof, for determining or detecting the presence and/or amount of one or more bacterial nucleic acids from members of the *Streptococcus* genus, said genus comprising: *S*. *agalactiae, S. anginosus, S. bovis, S. constellatus, S. crista, S. dysgalactiae, S. equi, S. gordonii, S. intermedius, S. mitis, S. mutans, S. oralis, S. parasanguis, S. pneumoniae, S. pyogenes, S. salivarius,* S. *sanguis, S. sobrinus, S*. *suis, S. uberis, S. vestibularis and S*. *viridans;*
b) SEQ ID NO: 30 or a complementary sequence thereof for determining or detecting the presence and/or amount of one or more nucleic acids from the *Streptococcus agalactiae* species;
c) SEQ ID NO:144 or a complementary sequence thereof for determining the presence and/or amount of one or more nucleic acids from *Streptococcus agalactiae;*
d) SEQ ID NO:145 or a complementary sequence thereof, for determining the presence and/or amount of one or more nucleic acids from *Streptococcus pneumoniae;* and/or
e) SEQ ID NO:146, or a complementary sequence thereof, for determining the presence and/or amount of one or more nucleic acids from *Streptococcus salivarius.*

27. The diagnostic kit of claim 26, wherein said combination of probes and/or primers which specifically hybridize to the sequences in b) comprises a primer and/or probe consisting of one or more of the nucleotide sequences selected from the group consisting of SEQ ID NOs: 9 and 10, parts thereof, complementary sequences thereof and variants thereof for determining or detecting the presence and/or amount of one or more nucleic acids from the *Streptococcus agalactiae* species.

28. The kit of claim 26, wherein said oligonucleotide hybridizes in a) to each of the *Streptococcus* nucleotide sequences defined in the group consisting of SEQ ID NOs: 33-36.

29. The diagnostic kit of claim 28, wherein said combination of probes and/or primers which specifically hybridize to the sequences in a) comprises a primer and/or probe consisting of one or more of the nucleotide sequences selected from the group consisting of SEQ ID NOs: 21 and 22, parts thereof complementary sequences thereof, and variants thereof for determining or detecting the presence and/or amount of bacteria of the *Streptococcus* genus, said genus comprising: *S. agalactiae, S. anginosus, S. bovis, S. constellatus, S. crista, S. dysgalactiae, S. equi, S. gordonii, S. intermedius, S. mitis, S. mutans, S. oralis, S. parasanguis, S. pneumoniae, S. pyogenes, S. salivarius,* S. *sanguis, S. sobrinus, S. suis, S. uberis, S. vestibularis and S. viridans.*

30. The diagnostic kit of any one of claims 26-29, further comprising any suitable combination of probes and/or primers that hybridize to one or more of the nucleotide sequences selected from the group consisting of SEQ ID NOs: 110-117, parts thereof, complementary sequences thereof, and variants thereof for the detection and/or quantification of the nucleic acids of any combination of the bacterial resistance genes selected from the group consisting of *blaₒₓₐ, blaZ, aac6'-IIa*, *ermA, ermB, ermC, vanC.*

31. The diagnostic kit of any one of claims 26-29, further comprising any suitable combination of primers comprising a nucleotide sequence selected from the group consisting of SEQ ID NOs: 37-106, 173 and 174, parts thereof, complementary sequences thereof, and variants thereof, for the simultaneous detection and/or quantification of nucleic acids of any bacterial antibiotic resistance gene selected from the group consisting of *blaₜₑₘ, bla_{rob}, bfaₛₕᵥ, blaₒₓₐ, blaZ, aadB, aacC1, aacC2*, *aacC3*, *aacA4, aac6'-IIa*, *aad(6'), ermA, ermB, ermC, mecA, vanA, vanB, vanC, satA, aac(6)-aph(2'), vat, vga, msrA, sul* and *int.*

32. The diagnostic kit of any one of claims 26-31, further comprising any suitable combination of primers comprising a nucleotide sequence selected from the group consisting of SEQ ID NOs: 1-8 and 11-20-, parts thereof, complementary sequences thereof, and variants thereof, for the detection and/or quantification of nucleic acids of any bacterial and fungal species and/or genus selected from the group consisting of *Enterococcus faecium, Listeria monocytogenes, Neisseria meningitidis, Staphylococcus saprophyticus, Candida albicans, Enterococcus* species, *Neisseria* species and *Staphylococcus species.*

33. The diagnostic kit of any one of claims 26-32, further comprising any suitable combination of primers selected upon alignment of conserved nucleotides of at least two *tuf* sequences comprising a sequence selected from the group consisting of SEQ ID NOs: 118-171, complementary sequences thereof, and variants thereof, said combination of primers comprising a pair of primers comprising a nucleotide sequence selected from the group consisting of SEQ ID NOs: 23, 24, 107, 108, 109 and 172, complementary sequences thereof, and variants thereof, for the simultaneous universal detection and/or quantification of nucleic acids of bacterium or fungus.

34. The diagnostic kit of claim 33, wherein said pair of primers consists of nucleic acid sequences defined in SEQ ID NOs: 23 and 24; 107 and 108 or 109 and 172.

35. The isolated oligonucleotide of claim 21 or 22, wherein said oligonucleotide consists of 12-30 nucleotides in length.

36. The isolated oligonucleotide of claim 21, wherein said oligonucleotide consists of a nucleic acid sequence as defined in SEQ ID NO: 9, 10, 21 or 22 or a complementary sequence thereof.

37. The diagnostic kit of any one of claims 26-34, wherein said probes and/or primers are from 12-30 nucleotides in length.

38. The method of any one of claims 1-15 and 20, wherein multiplex amplification is used.

39. The method of any one of claims 1-15, 17-20 and 38, wherein said oligonucleotide is capable of specifically amplifying its target nucleic acid using PCR conditions comprising for each amplification cycle a denaturation step of 1 s at 95°C and an annealing-extension step of 30 s at 45-55°C.

40. The method of any one of claims 1-15, 17-20 and 38, wherein said oligonucleotide is capable of specifically amplifying its target nucleic acid using PCR conditions comprising for each amplification cycle a denaturation step of 1 s at 95°C and an annealing-extension step of 30 s at 55°C.

41. The oligonucleotide of any one of claims 21, 22, 35 and 36 or the diagnostic kit of any one of claims 26-33 and 36, wherein said oligonucleotide or said probes and/or primers are capable of specifically amplifying their target nucleic acid(s) using PCR conditions comprising for each amplification cycle a denaturation step of 1 s at 95°C and an annealing-extension step of 30 s at 45-55°C.

42. The oligonucleotide of any one of claims 21, 22, 35 and 36 or the diagnostic kit of any one of claims 26-33 and 36, wherein said oligonucleotide or said probes and/or primers are capable of specifically amplifying their target nucleic acid(s) using PCR conditions comprising for each amplification cycle a denaturation step of 1 s at 95°C and an annealing-extension step of 30 s at 55°C.

43. The method of any one of claims 1-20, and 38 wherein said oligonucleotide is capable of specifically hybridizing to its target nucleic acid under the following conditions:
- hybridization in pre-hybridization solution at 65°C overnight followed by post-hybridization washings:
twice in 3X SSC containing 1% SDS at 65°C for 15 min;
twice in 2X SSC containing 1% SDS at 65°C for 15 min;
twice in 1X SSC containing 1% SDS at 65°C for 15 min; and
a final wash in 0.1X SSC containing 1% SDS at 25°C for 15 min.

44. The oligonucleotide of claims 21, 22, 35, and 36 or the diagnostic kit of any one of claims 26-33 and 36, wherein said oligonucleotide or said probes and/or primers are capable of specifically hybridizing to their target nucleic acid(s) under the following conditions:
- hybridization in pre-hybridization solution at 65°C overnight followed by post-hybridization washings:
twice in 3X SSC containing 1% SDS at 65°C for 15 min;
twice in 2X SSC containing 1% SDS at 65°C for 15 min;
twice in 1X SSC containing 1% SDS at 65°C for 15 min; and
a final wash in 0.1X SSC containing 1% SDS at 25°C for 15 min.

45. The method of any one of claims 1-20, 38-40 and 43 or diagnostic kit of any one of claims 26-33, 36, 40, 41 and 43, for determining or detecting the presence and/or amount of *S. agalactiae, S. anginosus, S. bovis, S. constellatus, S. crista, S. dysgalactiae, S. equi, S. gordonii, S. intermedius, S. mitis, S. oralis, S. parasanguis, S. pneumoniae, S. pyogenes, S*. *sanguis, S. sobrinus, S*. *suis, S*. *uberis, S. vestibularis and S*. *viridans.*

46. The oligonucleotide of claims 21, 22, 35, 36, 37, 42 and 44 for determining the presence or amount of *S. agalactiae, S. anginosus, S. bovis, S. constellatus, S. crista, S. dysgalactiae, S. equi, S. gordonii, S. intermedius, S. mitis, S. oralis, S. parasanguis, S. pneumoniae, S. pyogenes, S. sanguis, S. sobrinus, S. suis, S. uberis, S. vestibularis and S. viridans.*

47. The method of any one of claims 1-20, 38-40, 43 and 45, wherein said oligonucleotide consists of 12-30 nucleotides in length.


SEQUENCE LISTING

<110>  GENEOHM SCIENCES CANADA INC.

<120>  SPECIES-SPECIFIC, GENUS-SPECIFIC AND UNIVERSAL DNA PROBES AND
       AMPLIFICATION PRIMERS TO RAPIDLY DETECT AND IDENTIFY COMMON
       BACTERIAL AND FUNGAL PATHOGENS AND ASSOCIATED ANTIBIOTIC
       RESISTANCE GENES...

<130>  GOH13863EPD4

<140>  Divisional Application based on EP09163281.0
<141>  2009-06-19

<150>  US 08/743,637
<151>  1996-11-04

<160>  174

<170>  PatentIn version 3.3

<210>  1
<211>  22
<212>  DNA
<213>  Artificial Sequence

<220>
<223>  Oligonucleotide that binds to Enterococcus faecium

<400>  1
tgctttagca acagcctatc ag                                                22


<210>  2
<211>  21
<212>  DNA
<213>  Artificial Sequence

<220>
<223>  Oligonucleotide that binds to Enterococcus faecium


<400>  2
taaacttctt ccggcacttc g                                                 21


<210>  3
<211>  21
<212>  DNA
<213>  Artificial Sequence

<220>
<223>  Oligonucleotide that binds to Listeria monocytogenes


<400>  3
tgcggctata aatgaagagg c                                                 21

<210>  4
<211>  21
<212>  DNA
<213>  Artificial Sequence

<220>
<223>  Oligonucleotide that binds to Listeria monocytogenes

<400>  4
atccgatgat gctatggctt t                                                21


<210>  5
<211>  21
<212>  DNA
<213>  Artificial Sequence

<220>
<223>  Oligonucleotide that binds to Neisseria meningitidis

<400>  5
ccagcggtat tgtttggtgg t                                                21


<210>  6
<211>  21
<212>  DNA
<213>  Artificial Sequence

<220>
<223>  Oligonucleotide that binds to Neisseria meningitidis

<400>  6
caggcggcct ttaataattt c                                                21


<210>  7
<211>  30
<212>  DNA
<213>  Artificial Sequence

<220>
<223>  Oligonucleotide that binds to Staphylococcus saprophyticus

<400>  7
agatcgaatt ccacatgaag gttattatga                                       30


<210>  8
<211>  30
<212>  DNA
<213>  Artificial Sequence

<220>
<223>  Oligonucleotide that binds to Staphylococcus saprophyticus

<400>  8
tcgcttctcc ctcaacaatc aaactatcct                                        30


<210>  9
<211>  23
<212>  DNA
<213>  Artificial Sequence

<220>
<223>  Oligonucleotide that binds to Streptococcus agalactiae

<400>  9
tttcaccagc tgtattagaa gta                                               23


<210>  10
<211>  23
<212>  DNA
<213>  Artificial Sequence

<220>
<223>  Oligonucleotide that binds to Streptococcus agalactiae

<400>  10
gttccctgaa cattatcttt gat                                               23


<210>  11
<211>  26
<212>  DNA
<213>  Artificial Sequence

<220>
<223>  Oligonucleotide that binds to Candida albicans

<400>  11
caagaaggtt ggttacaacc caaaga                                            26


<210>  12
<211>  26
<212>  DNA
<213>  Artificial Sequence

<220>
<223>  Oligonucleotide that binds to Candida albicans

<400>  12
aggtcttacc agtaacttta ccggat                                            26


<210>  13
<211>  22
<212>  DNA
<213>  Artificial Sequence

<220>
<223>  Oligonucleotide that binds to Enterococcus spp.

<400>  13
tactgacaaa ccattcatga tg                                                22


<210>  14
<211>  21
<212>  DNA
<213>  Artificial Sequence

<220>
<223>  Oligonucleotide that binds to Enterococcus spp.

<400>  14
aacttcgtca ccaacgcgaa c                                                 21


<210>  15
<211>  20
<212>  DNA
<213>  Artificial Sequence

<220>
<223>  Oligonucleotide that binds to Neisseria spp.

<400>  15
ctggcgcggt atggtcggtt                                                   20


<210>  16
<211>  22
<212>  DNA
<213>  Artificial Sequence

<220>
<223>  Oligonucleotide that binds to Neisseria spp.

<400>  16
gccgacgttg gaagtggtaa ag                                                22


<210>  17
<211>  25
<212>  DNA
<213>  Artificial Sequence

<220>
<223>  Oligonucleotide that binds to Staphylococcus spp.

<400>  17
ccgtgttgaa cgtggtcaaa tcaaa                                             25


<210>  18

<211>  25
<212>  DNA
<213>  Artificial Sequence

<220>
<223>  Oligonucleotide that binds to Staphylococcus spp.

<400>  18
trtgtggtgt ratwgwrcca ggagc                                          25


<210>  19
<211>  25
<212>  DNA
<213>  Artificial Sequence

<220>
<223>  Oligonucleotide that binds to Staphylococcus spp.

<400>  19
acaacgtggw caagtwttag cwgct                                          25


<210>  20
<211>  25
<212>  DNA
<213>  Artificial Sequence

<220>
<223>  Oligonucleotide that binds to Staphylococcus spp.

<400>  20
accatttcwg taccttctgg taagt                                          25


<210>  21
<211>  23
<212>  DNA
<213>  Artificial Sequence

<220>
<223>  Oligonucleotide that binds to Streptococcus spp.

<220>
<221>  modified_base
<222>  (12)..(12)
<223>  n = inosine

<400>  21
gaaattgcag gnaaattgat tga                                            23


<210>  22
<211>  23
<212>  DNA
<213>  Artificial Sequence

<220>
<223>  Oligonucleotide that binds to Streptococcus spp.

<220>
<221>  misc_feature
<222>  (12)..(12)
<223>  n = inosine

<400>  22
ttacgcatgg cntgactcat cat                                              23


<210>  23
<211>  23
<212>  DNA
<213>  Artificial Sequence

<220>
<223>  Oligonucleotide that binds to several species (universal detection)

<220>
<221>  modified_base
<222>  (3)..(3)
<223>  n = inosine

<220>
<221>  modified_base
<222>  (6)..(6)
<223>  n = inosine

<220>
<221>  modified_base
<222>  (9)..(9)
<223>  n = inosine

<220>
<221>  modified_base
<222>  (12)..(12)
<223>  n = inosine

<220>
<221>  modified_base
<222>  (15)..(15)
<223>  n = inosine

<400>  23
acnkknacng gngtngarat gtt                                              23


<210>  24
<211>  23
<212>  DNA
<213>  Artificial Sequence

<220>
<223>  Oligonucleotide that binds to several species (universal detection)

<220>
<221>  misc_feature
<222>  (6)..(6)
<223>  n = inosine

<220>
<221>  misc_feature
<222>  (9)..(9)
<223>  n = inosine

<220>
<221>  misc_feature
<222>  (12)..(12)
<223>  n = inosine

<220>
<221>  misc_feature
<222>  (18)..(18)
<223>  n = inosine

<400>  24
ayrttntcnc cnggcatnac cat                                              23


<210>  25
<211>  10
<212>  DNA
<213>  Artificial Sequence

<220>
<223>  Oligonucleotide that binds to Staphylococcus saprophyticus (AP-PCR primer
OPAD-9)

<400>  25
tcgcttctcc                                                             10


<210>  26
<211>  600
<212>  DNA
<213>  Enterococcus faecium

<400>  26
ttcttagaga cattgaatat gccttatgtc ggcgcaggcg tattgaccag tgcatgtgcc      60

atggataaaa tcatgaccaa gtatatttta caagctgctg gtgtgccgca agttccttat     120

gtaccagtac ttaagaatca atggaaagaa aatcctaaaa aagtatttga tcaatgtgaa     180

ggttctttgc tttatccgat gtttgtcaaa cctgcgaata tgggttctag tgtcggcatt     240

acaaaggcag aaaaccgaga agagctgcaa aatgctttag caacagccta tcagtatgat     300

tctcgagcaa tcgttgaaca aggaattgaa gcgcgcgaaa tcgaagttgc tgtattagga     360

aatgaagatg ttcggacgac tttgcctggc gaagtcgtaa aagacgtagc attctatgat     420

tatgaagcca aatatatcaa taataaaatc gaaatgcaga ttccagccga agtgccggaa 480

gaagtttatc aaaaagcgca agagtacgcg aagttagctt acacgatgtt aggtggaagc 540

ggattgagcc ggtgcgattt ctttttgaca aataaaaatg aattattcct gaatgaatta 600

<210> 27
<211> 1920
<212> DNA
<213> Listeria monocytogenes

<400> 27
gtgggattaa acagatttat gcgtgcgatg atggtggttt tcattactgc caattgcatt 60

acgattaacc ccgacataat atttgcagcg acagatagcg aagattctag tctaaacaca 120

gatgaatggg aagaagaaaa aacagaagag caaccaagcg aggtaaatac gggaccaaga 180

tacgaaactg cacgtgaagt aagttcacgt gatattaaag aactagaaaa atcgaataaa 240

gtgagaaata cgaacaaagc agacctaata gcaatgttga aagaaaaagc agaaaaaggt 300

ccaaatatca ataataacaa cagtgaacaa actgagaatg cggctataaa tgaagaggct 360

tcaggagccg accgaccagc tatacaagtg gagcgtcgtc atccaggatt gccatcggat 420

agcgcagcgg aaattaaaaa aagaaggaaa gccatagcat catcggatag tgagcttgaa 480

agccttactt atccggataa accaacaaaa gtaaataaga aaaaagtggc gaaagagtca 540

gttgcggatg cttctgaaag tgacttagat tctagcatgc agtcagcaga tgagtcttca 600

ccacaacctt taaaagcaaa ccaacaacca tttttcccta aagtatttaa aaaaataaaa 660

gatgcgggga aatgggtacg tgataaaatc gacgaaaatc ctgaagtaaa gaaagcgatt 720

gttgataaaa gtgcagggtt aattgaccaa ttattaacca aaaagaaaag tgaagaggta 780

aatgcttcgg acttcccgcc accacctacg gatgaagagt taagacttgc tttgccagag 840

acaccaatgc ttcttggttt taatgctcct gctacatcag aaccgagctc attcgaattt 900

ccaccaccac ctacggatga agagttaaga cttgctttgc cagagacgcc aatgcttctt 960

ggttttaatg ctcctgctac atcggaaccg agctcgttcg aatttccacc gcctccaaca 1020

gaagatgaac tagaaatcat ccgggaaaca gcatcctcgc tagattctag ttttacaaga 1080

ggggatttag ctagtttgag aaatgctatt aatcgccata gtcaaaattt ctctgatttc 1140

ccaccaatcc caacagaaga agagttgaac gggagaggcg gtagaccaac atctgaagaa 1200

tttagttcgc tgaatagtgg tgattttaca gatgacgaaa acagcgagac aacagaagaa 1260

gaaattgatc gcctagctga tttaagagat agaggaacag gaaaacactc aagaaatgcg 1320

ggttttttac cattaaatcc gtttgctagc agcccggttc cttcgttaag tccaaaggta 1380

tcgaaaataa gcgaccgggc tctgataagt gacataacta aaaaaacgcc atttaagaat 1440

ccatcacagc cattaaatgt gtttaataaa aaaactacaa cgaaaacagt gactaaaaaa 1500

ccaacccctg taaagaccgc accaaagcta gcagaacttc ctgccacaaa accacaagaa 1560

accgtactta gggaaaataa aacacccttt atagaaaaac aagcagaaac aaacaagcag 1620

tcaattaata tgccgagcct accagtaatc caaaaagaag ctacagagag cgataaagag 1680

gaaatgaaac cacaaaccga ggaaaaaatg gtagaggaaa gcgaatcagc taataacgca 1740

aacggaaaaa atcgttctgc tggcattgaa gaaggaaaac taattgctaa aagtgcagaa 1800

gacgaaaaag cgaaggaaga accagggaac catacgacgt taattcttgc aatgttagct 1860

attggcgtgt tctctttagg ggcgtttatc aaaattattc aattaagaaa aaataattaa 1920

<210> 28
<211> 415
<212> DNA
<213> Neisseria meningitidis

<400> 28
taccggtacg ctaaatattg gtgatgtatt ggatattatg atttgggaag cgccgccagc 60

ggtattgttt ggtggtggcc tttcttcgat gggctcgggt agtgcgcaac aaaccaagtt 120

gccggagcaa ctggtgacgg cacgtggtac ggtttctgtg ccgtttgttg gcgatatttc 180

ggtggtcggt aaaacgcctg gtcaggttca ggaaattatt aaaggccgcc tgaaaaaaat 240

ggccaatcag ccgcaagtga tggtgcgctt ggtgcagaat aatgcggcaa atgtatcggt 300

gattcgcgca ggcaatagtg tgcgtatgcc gttgacggca gccggtgagc gtgtgttgga 360

tgcggtggct gcggtaggtg gttcaacggc aaatgtgcag gatacgaatg tgcag 415

<210> 29
<211> 438
<212> DNA
<213> Staphylococcus saprophyticus

<400> 29
tcgcttctcc agaagaaatt ttagaaacat atctagaaaa tcccaaatta gataaaccgt 60

ttatattatg tgaatacgca catgcaatgg gaaattcacc aggagatctt aatgcatatc 120

aaacattaat tgaaaaatat gatagtttta ttggcggttt tgtttgggaa tggtgtgatc 180

atagcattca ggttgggata aaggaaggta aaccaatttt tagatatggt ggagattttg 240

gtgaggcctt acatgacggt aatttttgtg ttgatggtat tgtttcgcca gatcgaattc 300

cacatgaagg ttattatgag tttaaacatg aacatagacc tttgagattg gttaacgaag 360

aggattatcg gtttacattg aagaatcaat ttgattttac aaatgcggag gatagtttga 420

ttgttgaggg agaagcga 438

<210> 30
<211> 768
<212> DNA
<213> Streptococcus agalactiae

<400> 30
atgaacgtta cacatatgat gtatctatct ggaactctag tggctggtgc attgttattt 60

tcaccagctg tattagaagt acatgctgat caagtgacaa ctccacaagt ggtaaatcat 120

gtaaatagta ataatcaagc ccagcaaatg gctcaaaagc ttgatcaaga tagcattcag 180

ttgagaaata tcaaagataa tgttcaggga acagattatg aaaaaccggt taatgaggct 240

attactagcg tggaaaaatt aaagacttca ttgcgtgcca accctgagac agtttatgat 300

ttgaattcta ttggtagtcg tgtagaagcc ttaacagatg tgattgaagc aatcactttt 360

tcaactcaac atttaacaaa taaggttagt caagcaaata ttgatatggg atttgggata 420

actaagctag ttattcgcat tttagatcca tttgcttcag ttgattcaat taaagctcaa 480

gttaacgatg taaaggcatt agaacaaaaa gttttaactt atcctgattt aaaaccaact 540

gatagagcta ccatctatac aaaatcaaaa cttgataagg aaatctggaa tacacgcttt 600

actagagata aaaaagtact taacgtcaaa gaatttaaag tttacaatac tttaaataaa 660

gcaatcacac atgctgttgg agttcagttg aatccaaatg ttacggtaca acaagttgat 720

caagagattg taacattaca agcagcactt caaacagcat taaaataa 768

<210> 31
<211> 421
<212> DNA
<213> Neisseria meningitidis

<400> 31
atgaaagtag gtttcgtcgg ctggcgcggt atggtcggtt cggttttgat gcagcgtatg 60

aaagaagaaa acgacttcgc ccacattccc gaagcgtttt tctttaccac ttccaacgtc 120

ggcggcgcac gccctgattt cggtcaggcg gctaaaacat tattggacgc gaacaacgtt 180

gccgagctgg caaaaatgga catcatcgtt acctgccaag gcggcgacta caccaaatcc 240

gtcttccaag ccctgcgcga cagcggctgg aacggctact ggattgacgc ggcatcctcg 300

ctgcgtatga aagacgacgc gattatcgtc ctcgaccccg tcaaccgcaa cgtcatcgac 360

aacggcctca aaaacggcgt gaaaaactac atcggcggca actgtaccgt ttccctgatg 420

c 421

<210> 32
<211> 213
<212> DNA
<213> Streptococcus gordonii

<400> 32
ttcatagacg ctgagcacgc tttggatcca tcttacgcgg ctgctctagg tgtaaatatt 60

gatgagctgt tgctatctca accagattct ggtgagcaag gtttagaaat tgcaggaaaa 120

ttgattgact ctggggcagt tgatttagtt gtcatcgact ctgttgcagc tcttgtacca 180

cgtgcggaaa tcgatggaga tatcggtgat agc 213

<210> 33
<211> 692
<212> DNA
<213> Streptococcus mutans

<400> 33
gggccggaat cttctggtaa gacaactgtc gctcttcatg ctgctgctca ggcgcaaaaa 60

gatggcggta ttgccgcttt cattgatgca gaacatgccc ttgatccagc ctatgctgct 120

gctcttggcg ttaatattga tgagcttttg ctttcacaac cagattcagg agaacagggt 180

cttgaaattg cagggaaatt gattgattct ggcgctgttg atttagttgt tgttgactca 240

gtggcagctt tagtaccacg tgcggagatt gacggagata ttggtaatag tcatgttggc 300

ttacaagcac gcatgatgag tcaagcgatg cgtaaattat cagcttcaat caataaaaca 360

aaaaccattg ctatttttat taatcaattg cgggaaaaag ttggtattat gtttggtaat 420

ccagaaacaa cccctggcgg gcgtgccttg aagttttatt cttctgtgcg tcttgatgtc 480

cgcggcaata ctcaaattaa aggaaccggg gaacaaaaag acagcaatat tggtaaagag 540

accaaaatta aagttgttaa aaataaagtt gctccaccat ttaaggaagc ttttgtagaa 600

attatatatg gtgaaggcat ttctcgtaca ggtgaattag ttaagattgc cagtgatttg 660

ggaattatcc aaaaagctgg agcttggtac tc 692

<210> 34
<211> 1204
<212> DNA
<213> Streptococcus pneumoniae

<400>  34
atggcgaaaa aaccaaaaaa attagaagaa atttcaaaaa aatttggggc agaacgtgaa      60

aaggccttga atgacgctct taaattgatt gagaaagact ttggtaaagg atcaatcatg     120

cgtttgggtg aacgtgcgga gcaaaaggtg caagtgatga gctcaggttc tttagctctt     180

gacattgccc ttggctcagg tggttatcct aagggacgta tcatcgaaat ctatggccca     240

gagtcatctg gtaagacaac ggttgccctt catgcagttg cacaagcgca aaaagaaggt     300

gggattgctg cctttatcga tgcggaacat gcccttgatc cagcttatgc tgcggccctt     360

ggtgtcaata ttgacgaatt gctcttgtct caaccagact caggagagca aggtcttgag     420

attgcgggaa aattgattga ctcaggtgca gttgatcttg tcgtagtcga ctcagttgct     480

gcccttgttc ctcgtgcgga aattgatgga gatatcggag atagccatgt tggtttgcag     540

gctcgtatga tgagccaggc catgcgtaaa cttggcgcct ctatcaataa aaccaaaaca     600

attgccattt ttatcaacca attgcgtgaa aaagttggag tgatgtttgg aaatccagaa     660

acaacaccgg gcggacgtgc tttgaaattc tatgcttcag tccgcttgga tgttcgtggt     720

aatacacaaa ttaagggaac tggtgatcaa aaagaaacca atgtcggtaa agaaactaag     780

attaaggttg taaaaaataa ggtagctcca ccgtttaagg aagccgtagt tgaaattatg     840

tacggagaag gaatttctaa gactggtgag cttttgaaga ttgcaagcga tttggatatt     900

atcaaaaaag caggggcttg gtattcttac aaagatgaaa aaattgggca aggttctgag     960

aatgctaaga aatacttggc agagcaccca gaaatctttg atgaaattga taagcaagtc    1020

cgttctaaat ttggcttgat tgatggagaa gaagtttcag aacaagatac tgaaaacaaa    1080

aaagatgagc caaagaaaga agaagcagtg aatgaagaag ttccgcttga cttaggcgat    1140

gaacttgaaa tcgaaattga agaataagct gttaaagcag tggagaaatc cgctactttt    1200

tcga                                                                 1204


<210>  35
<211>  981
<212>  DNA
<213>  Streptococcus pyogenes

<400>  35
atgcgttcag gaagtctagc tcttgatatt gcttggatag ctggtggtta tcctaaagga      60

cgtatcatcg aaatctatgg tccagagtct tccggtaaaa cgactgtggc tttacatgct     120

gtagcacaag ctcaaaaaga aggtggaatc gcagccttta tcgatgccga gcatgcgctt     180

gatccagctt atgctgctgc gcttggggtt aatattgatg aacttctctt gtctcaacca    240

gattctggag aacaaggact tgaaattgca ggtaaattga ttgattctgg tgcggttgac    300

ctggttgttg tcgattcagt agcagcttta gtgccacgtg ctgaaattga tggtgatatt    360

ggcgatagcc atgtcggatt gcaagcacgt atgatgagtc aggccatgcg taaattatca    420

gcttctatta ataaaacaaa aactatcgca atctttatca accaattgcg tgaaaaagtt    480

ggtgtgatgt ttggaaatcc tgaaacaaca ccaggtggtc gagctttgaa attctatgct    540

tctgttcggc tggatgtgcg tggaaacaac caaattaaag gaactggtga ccaaaagata    600

gccagcattg gtaaggagac caaaatcaag gttgttaaaa acaaggtcgc tccgccattt    660

aaggtagcag aagttgaaat catgtatggg gaaggtattt ctcgtacagg ggagcttgtg    720

aaaattgctt ctgatttgga cattatccaa aaagcaggtg cttggttctc ttataatggt    780

gagaagattg gccaaggttc tgaaaatgct aagcgttatt tggccgatca tccacaattg    840

tttgatgaaa tcgaccgtaa agtacgtgtt aaatttggtt tgcttgaaga aagcgaagaa    900

gaatctgcta tggcagtagc atcagaagaa accgatgatc ttgctttaga tttagataat    960

ggtattgaaa ttgaagatta a                                              981

<210>  36
<211>  312
<212>  DNA
<213>  Streptococcus salivarius

<400>  36
gcgtatgcac gagctctagg tgttaatatc gatgagcttc ttttgtcgca gcctgattct     60

ggtgagcaag gtctcgaaat tgcaggtaag ctgattgact ctggtgcagt ggatttagtt    120

gttgttgact cagttgcggc cttcgtacca cgtgcagaaa ttgatggaga tagtggtgac    180

agtcatgtag gacttcaagc gcgtatgatg agtcaagcca tgcgtaaact ttctgcatct    240

attaataaaa caaaaacgat tgctatcttt attaaccagt tgcgtgaaaa agttggtatc    300

atgtttggta ac                                                        312

<210>  37
<211>  20
<212>  DNA
<213>  Artificial Sequence

<220>
<223>  Oligonucleotide that binds to antibiotic resistance gene of
Enterobacteriaceae,Pseudomonadaceae,Haemophilus spp.,Neisseria spp.

<400>  37
ctatgtggcg cggtattatc                                                   20


<210>  38
<211>  20
<212>  DNA
<213>  Artificial Sequence

<220>
<223>  Oligonucleotide that binds to antibiotic resistance gene of
Enterobacteriaceae,Pseudomonadaceae,Haemophilus spp.,Neisseria spp.

<400>  38
cgcagtgtta tcactcatgg                                                   20


<210>  39
<211>  20
<212>  DNA
<213>  Artificial Sequence

<220>
<223>  Oligonucleotide that binds to antibiotic resistance gene of
Enterobacteriaceae,Pseudomonadaceae,Haemophilus spp.,Neisseria spp.

<400>  39
ctgaatgaag ccataccaaa                                                   20


<210>  40
<211>  20
<212>  DNA
<213>  Artificial Sequence

<220>
<223>  Oligonucleotide that binds to antibiotic resistance gene of
Enterobacteriaceae, Pseudomonadaceae,Haemophilus spp.,Neisseria spp.

<400>  40
atcagcaata aaccagccag                                                   20


<210>  41
<211>  20
<212>  DNA
<213>  Artificial Sequence

<220>
<223>  Oligonucleotide that binds to antibiotic resistance gene of Klebsiella
spp.,and other Enterobacteriaceae

<400>  41
ttaccatgag cgataacagc                                                   20


<210>  42

<211>  20
<212>  DNA
<213>  Artificial Sequence

<220>
<223>  Oligonucleotide that binds to antibiotic resistance gene of Klebsiella
spp.,and other Enterobacteriaceae

<400>  42
ctcattcagt tccgtttccc                                                    20


<210>  43
<211>  20
<212>  DNA
<213>  Artificial Sequence

<220>
<223>  Oligonucleotide that binds to antibiotic resistance gene of Klebsiella
spp.,and other Enterobacteriaceae

<400>  43
cagctgctgc agtggatggt                                                    20


<210>  44
<211>  20
<212>  DNA
<213>  Artificial Sequence

<220>
<223>  Oligonucleotide that binds to antibiotic resistance gene of Klebsiella
spp.,and other Enterobacteriaceae

<400>  44
cgctctgctt tgttattcgg                                                    20


<210>  45
<211>  20
<212>  DNA
<213>  Artificial Sequence

<220>
<223>  Oligonucleotide that binds to antibiotic resistance gene of Haemophilus
spp.,Pasteurella spp.

<400>  45
tacgccaaca tcgtggaaag                                                    20


<210>  46
<211>  20
<212>  DNA
<213>  Artificial Sequence

<220>

<223>  Oligonucleotide that binds to antibiotic resistance gene of Haemophilus
spp.,Pasteurella spp.

<400>  46
ttgaatttgg cttcttcggt                                                    20


<210>  47
<211>  20
<212>  DNA
<213>  Artificial Sequence

<220>
<223>  Oligonucleotide that binds to antibiotic resistance gene of Haemophilus
spp.,Pasteurella spp.

<400>  47
gggatacaga aacgggacat                                                    20


<210>  48
<211>  20
<212>  DNA
<213>  Artificial Sequence

<220>
<223>  Oligonucleotide that binds to antibiotic resistance gene of Haemophilus
spp.,Pasteurella spp.

<400>  48
taaatctttt tcaggcagcg                                                    20


<210>  49
<211>  25
<212>  DNA
<213>  Artificial Sequence

<220>
<223>  Oligonucleotide that binds to antibiotic resistance gene of
Enterobacteriaceae,Pseudomonadaceae

<400>  49
gatggtttga agggtttatt ataag                                              25


<210>  50
<211>  25
<212>  DNA
<213>  Artificial Sequence

<220>
<223>  Oligonucleotide that binds to antibiotic resistance gene of
Enterobacteriaceae,Pseudomonadaceae

<400>  50
aatttagtgt gtttagaatg gtgat                                              25

<210>  51
<211>  21
<212>  DNA
<213>  Artificial Sequence

<220>
<223>  Oligonucleotide that binds to antibiotic resistance gene of Enterococcus
spp.

<400>  51
acttcaacac ctgctgcttt c                                                 21


<210>  52
<211>  21
<212>  DNA
<213>  Artificial Sequence

<220>
<223>  Oligonucleotide that binds to antibiotic resistance gene of Enterococcus
spp.

<400>  52
tgaccacttt tatcagcaac c                                                 21


<210>  53
<211>  20
<212>  DNA
<213>  Artificial Sequence

<220>
<223>  Oligonucleotide that binds to antibiotic resistance gene of
Enterobacteriaceae,Pseudomonadaceae

<400>  53
ggcaatagtt gaaatgctcg                                                   20


<210>  54
<211>  20
<212>  DNA
<213>  Artificial Sequence

<220>
<223>  Oligonucleotide that binds to antibiotic resistance gene of
Enterobacteriaceae,Pseudomonadaceae

<400>  54
cagctgttac aacggactgg                                                   20


<210>  55
<211>  20
<212>  DNA

<213>  Artificial Sequence

<220>
<223>  Oligonucleotide that binds to antibiotic resistance gene of
Enterobacteriaceae,Pseudomonadaceae

<400>  55
tctatgatct cgcagtctcc                                                   20


<210>  56
<211>  20
<212>  DNA
<213>  Artificial Sequence

<220>
<223>  Oligonucleotide that binds to antibiotic resistance gene of
Enterobacteriaceae,Pseudomonadaceae

<400>  56
atcgtcaccg taatctgctt                                                   20


<210>  57
<211>  20
<212>  DNA
<213>  Artificial Sequence

<220>
<223>  Oligonucleotide that binds to antibiotic resistance gene of
Enterobacteriaceae,Pseudomonadaceae

<400>  57
cattctcgat tgctttgcta                                                   20


<210>  58
<211>  20
<212>  DNA
<213>  Artificial Sequence

<220>
<223>  Oligonucleotide that binds to antibiotic resistance gene of bacteria

<400>  58
ccgaaatgct tctcaagata                                                   20


<210>  59
<211>  20
<212>  DNA
<213>  Artificial Sequence

<220>
<223>  Oligonucleotide that binds to antibiotic resistance gene of
Enterobacteriaceae,Pseudomonadaceae

<400>  59
ctggattatg gctacggagt                                                   20

<210>  60
<211>  20
<212>  DNA
<213>  Artificial Sequence

<220>
<223>  Oligonucleotide that binds to antibiotic resistance gene of
Enterobacteriaceae,Pseudomonadaceae

<400>  60
agcagtgtga tggtatccag                                                   20

<210>  61
<211>  20
<212>  DNA
<213>  Artificial Sequence

<220>
<223>  Oligonucleotide that binds to antibiotic resistance gene of
Pseudomonadaceae

<400>  61
gactcttgat gaagtgctgg                                                   20

<210>  62
<211>  20
<212>  DNA
<213>  Artificial Sequence

<220>
<223>  Oligonucleotide that binds to antibiotic resistance gene of
Pseudomonadaceae

<400>  62
ctggtctatt cctcgcactc                                                   20

<210>  63
<211>  20
<212>  DNA
<213>  Artificial Sequence

<220>
<223>  Oligonucleotide that binds to antibiotic resistance gene of
Pseudomonadaceae

<400>  63
tatgagaagg caggattcgt                                                   20

<210>  64

<211>  20
<212>  DNA
<213>  Artificial Sequence

<220>
<223>  Oligonucleotide that binds to antibiotic resistance gene of
Pseudomonadaceae

<400>  64
gctttctctc gaaggcttgt                                                   20


<210>  65
<211>  20
<212>  DNA
<213>  Artificial Sequence

<220>
<223>  Oligonucleotide that binds to antibiotic resistance gene of
Enterobacteriaceae,Pseudomonadaceae

<400>  65
gagttgctgt tcaatgatcc                                                   20


<210>  66
<211>  20
<212>  DNA
<213>  Artificial Sequence

<220>
<223>  Oligonucleotide that binds to antibiotic resistance gene of
Enterobacteriaceae,Pseudomonadaceae

<400>  66
gtgtttgaac catgtacacg                                                   20


<210>  67
<211>  20
<212>  DNA
<213>  Artificial Sequence

<220>
<223>  Oligonucleotide that binds to antibiotic resistance gene of Enterococcus
spp.

<400>  67
tgtagaggtc tagcccgtgt                                                   20


<210>  68
<211>  20
<212>  DNA
<213>  Artificial Sequence

<220>

<223>  Oligonucleotide that binds to antibiotic resistance gene of Enterococcus
spp.

<400>  68
acggggataa cgactgtatg                                                    20


<210>  69
<211>  20
<212>  DNA
<213>  Artificial Sequence

<220>
<223>  Oligonucleotide that binds to antibiotic resistance gene of Enterococcus
spp.

<400>  69
ataaagatga taggccggtg                                                    20


<210>  70
<211>  20
<212>  DNA
<213>  Artificial Sequence

<220>
<223>  Oligonucleotide that binds to antibiotic resistance gene of Enterococcus
spp.

<400>  70
tgctgtcata ttgtcttgcc                                                    20


<210>  71
<211>  20
<212>  DNA
<213>  Artificial Sequence

<220>
<223>  Oligonucleotide that binds to antibiotic resistance gene of Enterococcus
spp.

<400>  71
attatcttcg gcggttgctc                                                    20


<210>  72
<211>  20
<212>  DNA
<213>  Artificial Sequence

<220>
<223>  Oligonucleotide that binds to antibiotic resistance gene of Enterococcus
spp.

<400>  72
gactatcggc ttcccattcc                                                    20

<210>  73
<211>  20
<212>  DNA
<213>  Artificial Sequence

<220>
<223>  Oligonucleotide that binds to antibiotic resistance gene of Enterococcus
spp.

<400>  73
cgatagaagc agcaggacaa                                                    20


<210>  74
<211>  20
<212>  DNA
<213>  Artificial Sequence

<220>
<223>  Oligonucleotide that binds to antibiotic resistance gene of Enterococcus
spp.

<400>  74
ctgatggatg cggaagatac                                                    20


<210>  75
<211>  21
<212>  DNA
<213>  Artificial Sequence

<220>
<223>  Oligonucleotide that binds to antibiotic resistance gene of Enterococcus
spp.

<400>  75
gccttatgta tgaacaaatg g                                                  21


<210>  76
<211>  23
<212>  DNA
<213>  Artificial Sequence

<220>
<223>  Oligonucleotide that binds to antibiotic resistance gene of Enterococcus
spp.

<400>  76
gtgactttwg tgatcccttt tga                                                23


<210>  77
<211>  20
<212>  DNA

<213>  Artificial Sequence

<220>
<223>  Oligonucleotide that binds to antibiotic resistance gene of
Staphylococcus spp.

<400>  77
tccaatcatt gcacaaaatc                                                    20


<210>  78
<211>  20
<212>  DNA
<213>  Artificial Sequence

<220>
<223>  Oligonucleotide that binds to antibiotic resistance gene of
Staphylococcus spp.

<400>  78
aattccctct atttggtggt                                                    20


<210>  79
<211>  20
<212>  DNA
<213>  Artificial Sequence

<220>
<223>  Oligonucleotide that binds to antibiotic resistance gene of
Staphylococcus spp.

<400>  79
tcccaagcca gtaaagctaa                                                    20


<210>  80
<211>  20
<212>  DNA
<213>  Artificial Sequence

<220>
<223>  Oligonucleotide that binds to antibiotic resistance gene of
Staphylococcus spp.

<400>  80
tggtttttca acttcttcca                                                    20


<210>  81
<211>  20
<212>  DNA
<213>  Artificial Sequence

<220>
<223>  Oligonucleotide that binds to antibiotic resistance gene of Enterococcus
spp.

<400>  81
tcatagaatg gatggctcaa                                                   20


<210>  82
<211>  20
<212>  DNA
<213>  Artificial Sequence

<220>
<223>  Oligonucleotide that binds to antibiotic resistance gene of Enterococcus
spp.

<400>  82
agctactatt gcaccatccc                                                   20


<210>  83
<211>  22
<212>  DNA
<213>  Artificial Sequence

<220>
<223>  Oligonucleotide that binds to antibiotic resistance gene of Enterococcus
spp.,Staphylococcus spp.

<400>  83
caataagggc ataccaaaaa tc                                                22


<210>  84
<211>  22
<212>  DNA
<213>  Artificial Sequence

<220>
<223>  Oligonucleotide that binds to antibiotic resistance gene of Enterococcus
spp.,Staphylococcus spp.

<400>  84
ccttaacatt tgtggcatta tc                                                22


<210>  85
<211>  22
<212>  DNA
<213>  Artificial Sequence

<220>
<223>  Oligonucleotide that binds to antibiotic resistance gene of Enterococcus
spp.,Staphylococcus spp.

<400>  85
ttgggaagat gaagttttta ga                                                22

<210>  86
<211>  22
<212>  DNA
<213>  Artificial Sequence

<220>
<223>  Oligonucleotide that binds to antibiotic resistance gene of Enterococcus
spp.,Staphylococcus spp.

<400>  86
cctttactcc aataatttgg ct                                                22


<210>  87
<211>  20
<212>  DNA
<213>  Artificial Sequence

<220>
<223>  Oligonucleotide that binds to antibiotic resistance gene of
Staphylococcus spp.

<400>  87
tttcatctat tcaggatggg                                                   20


<210>  88
<211>  20
<212>  DNA
<213>  Artificial Sequence

<220>
<223>  Oligonucleotide that binds to antibiotic resistance gene of
Staphylococcus spp.

<400>  88
ggagcaacat tctttgtgac                                                   20


<210>  89
<211>  20
<212>  DNA
<213>  Artificial Sequence

<220>
<223>  Oligonucleotide that binds to antibiotic resistance gene of
Staphylococcus spp.

<400>  89
tgtgcctgaa gaaggtattg                                                   20


<210>  90
<211>  20
<212>  DNA
<213>  Artificial Sequence

<220>
<223>  Oligonucleotide that binds to antibiotic resistance gene of
Staphylococcus spp.

<400>  90
cgtgttactt caccaccact                                                   20


<210>  91
<211>  23
<212>  DNA
<213>  Artificial Sequence

<220>
<223>  Oligonucleotide that binds to antibiotic resistance gene of
Staphylococcus spp.

<400>  91
tatcttatcg ttgagaaggg att                                               23


<210>  92
<211>  22
<212>  DNA
<213>  Artificial Sequence

<220>
<223>  Oligonucleotide that binds to antibiotic resistance gene of
Staphylococcus spp.

<400>  92
ctacacttgg cttaggatga aa                                                22


<210>  93
<211>  24
<212>  DNA
<213>  Artificial Sequence

<220>
<223>  Oligonucleotide that binds to antibiotic resistance gene of
Staphylococcus spp.

<400>  93
ctatctgatt gttgaagaag gatt                                              24


<210>  94
<211>  24
<212>  DNA
<213>  Artificial Sequence

<220>
<223>  Oligonucleotide that binds to antibiotic resistance gene of
Staphylococcus spp.

<400>  94

gtttactctt ggtttaggat gaaa                                          24

<210>  95
<211>  22
<212>  DNA
<213>  Artificial Sequence

<220>
<223>  Oligonucleotide that binds to antibiotic resistance gene of
Staphylococcus spp.

<400>  95
cttgttgatc acgataattt cc                                            22

<210>  96
<211>  22
<212>  DNA
<213>  Artificial Sequence

<220>
<223>  Oligonucleotide that binds to antibiotic resistance gene of
Staphylococcus spp.

<400>  96
atcttttagc aaacccgtat tc                                            22

<210>  97
<211>  27
<212>  DNA
<213>  Artificial Sequence

<220>
<223>  Oligonucleotide that binds to antibiotic resistance gene of
Staphylococcus spp.

<400>  97
aacaggtgaa ttattagcac ttgtaag                                       27

<210>  98
<211>  27
<212>  DNA
<213>  Artificial Sequence

<220>
<223>  Oligonucleotide that binds to antibiotic resistance gene of
Staphylococcus spp.

<400>  98
   attgctgtta atattttttg agttgaa                                        27

<210>  99
<211>  19

<212>  DNA
<213>  Artificial Sequence

<220>
<223>  Oligonucleotide that binds to antibiotic resistance gene of
Enterobacteriaceae,Pseudomonadaceae

<400>  99
gtgatcgaaa tccagatcc                                                    19


<210>  100
<211>  19
<212>  DNA
<213>  Artificial Sequence

<220>
<223>  Oligonucleotide that binds to antibiotic resistance gene of
Enterobacteriaceae,Pseudomonadaceae

<400>  100
atcctcggtt ttctggaag                                                    19


<210>  101
<211>  19
<212>  DNA
<<213> Artificial Sequence

<220>
<223>  Oligonucleotide that binds to antibiotic resistance gene of
Enterobacteriaceae,Pseudomonadaceae

<400>  101
ctggtcatac atgtgatgg                                                    19


<210>  102
<211>  19
<212>  DNA
<213>  Artificial Sequence

<220>
<223>  Oligonucleotide that binds to antibiotic resistance gene of
Enterobacteriaceae,Pseudomonadaceae

<400>  102
gatgttaccc gagagcttg                                                    19


<210>  103
<211>  20
<212>  DNA
<213>  Artificial Sequence

<220>

<223>  Oligonucleotide that binds to antibiotic resistance gene of
Enterobacteriaceae,Pseudomonadaceae

<400>  103
ttaagcgtgc ataataagcc                                                   20


<210>  104
<211>  20
<212>  DNA
<213>  Artificial Sequence

<220>
<223>  Oligonucleotide that binds to antibiotic resistance gene of
Enterobacteriaceae,Pseudomonadaceae

<400>  104
ttgcgattac ttcgccaact                                                   20


<210>  105
<211>  20
<212>  DNA
<213>  Artificial Sequence

<220>
<223>  Oligonucleotide that binds to antibiotic resistance gene of
Enterobacteriaceae,Pseudomonadaceae

<400>  105
tttactaagc ttgccccttc                                                   20


<210>  106
<211>  20
<212>  DNA
<213>  Artificial Sequence

<220>
<223>  Oligonucleotide that binds to antibiotic resistance gene of
Enterobacteriaceae,Pseudomonadaceae

<400>  106
aaaaggcagc aattatgagc                                                   20


<210>  107
<211>  29
<212>  DNA
<213>  Artificial Sequence

<220>
<223>  Oligonucleotide that binds to any bacterial species (tuf gene)

<220>
<221>  misc_feature
<222>  (9)..(9)

<223>  n = inosine

<220>
<221>  misc_feature
<222>  (12)..(12)
<223>  n = inosine

<220>
<221>  misc_feature
<222>  (15)..(15)
<223>  n = inosine

<220>
<221>  misc_feature
<222>  (18)..(18)
<223>  n = inosine

<220>
<221>  misc_feature
<222>  (21)..(21)
<223>  n = inosine

<400>  107
aayatgatna cnggngcngc ncaratgga                                         29


<210>  108
<211>  23
<212>  DNA
<213>  Artificial Sequence

<220>
<223>  Oligonucleotide that binds to any bacterial species (tuf gene)

<220>
<221>  misc_feature
<222>  (3)..(3)
<223>  n = inosine

<220>
<221>  misc_feature
<222>  (6)..(6)
<223>  n = inosine

<220>
<221>  misc_feature
<222>  (9)..(9)
<223>  n = inosine

<220>
<221>  misc_feature
<222>  (12)..(12)
<223>  n = inosine

<400>  108
ccnacngtnc knccrccytc rcg                                               23

<210>  109
<211>  29
<212>  DNA
<213>  Artificial Sequence

<220>
<223>  Oligonucleotide that binds to any fungal species (tuf gene)

<220>
<221>  misc_feature
<222>  (6)..(6)
<223>  n = inosine

<220>
<221>  misc_feature
<222>  (12)..(12)
<223>  n = inosine

<220>
<221>  misc_feature
<222>  (15)..(15)
<223>  n = inosine

<220>
<221>  misc_feature
<222>  (18)..(18)
<223>  n = inosine

<400>  109
carytnathg tngcngtnaa yaaratgga                                         29


<210>  110
<211>  831
<212>  DNA
<213>  Enterobacteriaceae,Pseudomonadaceae

<400>  110
atgaaaaaca caatacatat caacttcgct atttttttaa taattgcaaa tattatctac      60

agcagcgcca gtgcatcaac agatatctct actgttgcat ctccattatt tgaaggaact     120

gaaggttgtt ttttacttta cgatgcatcc acaaacgctg aaattgctca attcaataaa     180

gcaaagtgtg caacgcaaat ggcaccagat tcaactttca agatcgcatt atcacttatg     240

gcatttgatg cggaaataat agatcagaaa accatattca aatgggataa aacccccaaa     300

ggaatggaga tctggaacag caatcataca ccaaagacgt ggatgcaatt ttctgttgtt     360

tgggtttcgc aagaaataac ccaaaaaatt agattaaata aaatcaagaa ttatctcaaa     420

gattttgatt atggaaatca agacttctct ggagataaag aaagaaacaa cggattaaca     480

gaagcatggc tcgaaagtag cttaaaaatt tcaccagaag aacaaattca attcctgcgt     540

aaaattatta atcacaatct cccagttaaa aactcagcca tagaaaacac catagagaac 600

atgtatctac aagatctgga taatagtaca aaactgtatg ggaaaactgg tgcaggattc 660

acagcaaata gaaccttaca aaacggatgg tttgaagggt ttattataag caaatcagga 720

cataaatatg tttttgtgtc cgcacttaca ggaaacttgg ggtcgaattt aacatcaagc 780

ataaaagcca agaaaaatgc gatcaccatt ctaaacacac taaatttata a 831

<210> 111
<211> 846
<212> DNA
<213> Enterococcus spp.

<400> 111
ttgaaaaagt taatattttt aattgtaatt gctttagttt taagtgcatg taattcaaac 60

agttcacatg ccaaagagtt aaatgattta gaaaaaaaat ataatgctca tattggtgtt 120

tatgctttag atactaaaag tggtaaggaa gtaaaattta attcagataa gagatttgcc 180

tatgcttcaa cttcaaaagc gataaatagt gctattttgt tagaacaagt accttataat 240

aagttaaata aaaaagtaca tattaacaaa gatgatatag ttgcttattc tcctatttta 300

gaaaaatatg taggaaaaga tatcacttta aaagcactta ttgaggcttc aatgacatat 360

agtgataata cagcaaacaa taaaattata aaagaaatcg gtggaatcaa aaaagttaaa 420

caacgtctaa aagaactagg agataaagta acaaatccag ttagatatga gatagaatta 480

aattactatt caccaaagag caaaaaagat acttcaacac ctgctgcttt cggtaagact 540

ttaaataaac ttatcgcaaa tggaaaatta agcaaagaaa acaaaaaatt cttacttgat 600

ttaatgttaa ataataaaag cggagatact ttaattaaag acggtgttcc aaaagactat 660

aaggttgctg ataaaagtgg tcaagcaata acatatgctt ctagaaatga tgttgctttt 720

gtttatccta agggccaatc tgaacctatt gttttagtca tttttacgaa taaagacaat 780

aaaagtgata agccaaatga taagttgata agtgaaaccg ccaagagtgt aatgaaggaa 840

ttttaa 846

<210> 112
<211> 555
<212> DNA
<213> Pseudomonadaceae

<400> 112
atgtccgcga gcaccccccc cataactctt cgcctcatga ccgagcgcga cctgccgatg 60

ctccatgact ggctcaaccg gccgcacatc gttgagtggt ggggtggcga cgaagagcga    120

ccgactcttg atgaagtgct ggaacactac ctgcccagag cgatggcgga agagtccgta    180

acaccgtaca tcgcaatgct gggcgaggaa ccgatcggct atgctcagtc gtacgtcgcg    240

ctcggaagcg gtgatggctg gtgggaagat gaaactgatc caggagtgcg aggaatagac    300

cagtctctgg ctgacccgac acagttgaac aaaggcctag gaacaaggct tgtccgcgct    360

ctcgttgaac tactgttctc ggaccccacc gtgacgaaga ttcagaccga cccgactccg    420

aacaaccatc gagccatacg ctgctatgag aaggcaggat tcgtgcggga gaagatcatc    480

accacgcctg acgggccggc ggtttacatg gttcaaacac gacaagcctt cgagagaaag    540

cgcggtgttg cctaa                                                      555

<210>  113
<211>  732
<212>  DNA
<213>  Staphylococcus spp.

<400>  113
atgaaccaga aaaaccctaa agacacgcaa aattttatta cttctaaaaa gcatgtaaaa     60

gaaatattga atcacacgaa tatcagtaaa caagacaacg taatagaaat cggatcagga    120

aaaggacatt ttaccaaaga gctagtcaaa atgagtcgat cagttactgc tatagaaatt    180

gatggaggct tatgtcaagt gactaaagaa gcggtaaacc cctctgagaa tataaaagtg    240

attcaaacgg atattctaaa attttccttc ccaaaacata taaactataa gatatatggt    300

aatattcctt ataacatcag tacggatatt gtcaaaagaa ttacctttga aagtcaggct    360

aaatatagct atcttatcgt tgagaaggga tttgcgaaaa gattgcaaaa tctgcaacga    420

gctttgggtt tactattaat ggtggagatg gatataaaaa tgctcaaaaa agtaccacca    480

ctatattttc atcctaagcc aagtgtagac tctgtattga ttgttcttga acgacatcaa    540

ccattgattt caaagaagga ctacaaaaag tatcgatctt ttgtttataa gtgggtaaac    600

cgtgaatatc gtgttctttt cactaaaaac caattccgac aggctttgaa gcatgcaaat    660

gtcactaata ttaataaact atcgaaggaa caatttcttt ctattttcaa tagttacaaa    720

ttgtttcact aa                                                         732

<210>  114
<211>  738
<212>  DNA
<213>  Staphylococcus spp.

<400>  114
atgaacaaaa atataaaata ttctcaaaac tttttaacga gtgaaaaagt actcaaccaa      60

ataataaaac aattgaattt aaaagaaacc gataccgttt acgaaattgg aacaggtaaa     120

gggcatttaa cgacgaaact ggctaaaata agtaaacagg taacgtctat tgaattagac     180

agtcatctat tcaacttatc gtcagaaaaa ttaaaatcga atactcgtgt cactttaatt     240

caccaagata ttctacagtt tcaattccct aacaaacaga ggtataaaat tgttgggaat     300

attccttacc atttaagcac acaaattatt aaaaaagtgg tttttgaaag ccatgcgtct     360

gacatctatc tgattgttga agaaggattc tacaagcgta ccttggatat tcaccgaaca     420

ctagggttgc tcttgcacac tcaagtctcg attcagcaat tgcttaagct gccagcggaa     480

tgctttcatc ctaaaccaag agtaaacagt gtcttaataa aacttacccg ccataccaca     540

gatgttccag ataaatattg gaagctatat acgtactttg tttcaaaatg ggtcaatcga     600

gaatatcgtc aactgtttac taaaaatcag tttcatcaag caatgaaaca cgccaaagta     660

aacaatttaa gtaccgttac ttatgagcaa gtattgtcta tttttaatag ttatctatta     720

tttaacggga ggaaataa                                                   738


<210>  115
<211>  735
<212>  DNA
<213>  Staphylococcus spp.

<400>  115
atgaacgaga aaaatataaa acacagtcaa aactttatta cttcaaaaca taatatagat      60

aaaataatga caaatataag attaaatgaa catgataata tctttgaaat cggctcagga     120

aaagggcatt ttacccttga attagtacag aggtgtaatt tcgtaactgc cattgaaata     180

gaccataaat tatgcaaaac tacagaaaat aaacttgttg atcacgataa tttccaagtt     240

ttaaacaagg atatattgca gtttaaattt cctaaaaacc aatcctataa aatatttggt     300

aatatacctt ataacataag tacggatata atacgcaaaa ttgtttttga tagtatagct     360

gatgagattt atttaatcgt ggaatacggg tttgctaaaa gattattaaa tacaaaacgc     420

tcattggcat tatttttaat ggcagaagtt gatatttcta tattaagtat ggttccaaga     480

gaatattttc atcctaaacc tagagtgaat agctcactta tcagattaaa tagaaaaaaa     540

tcaagaatat cacacaaaga taaacagaag tataattatt tcgttatgaa atgggttaac     600

aaagaataca agaaaatatt tacaaaaaat caatttaaca attccttaaa acatgcagga     660

attgacgatt taaacaatat tagctttgaa caattcttat ctcttttcaa tagctataaa     720

ttatttaata agtaa 735

<210> 116
<211> 1029
<212> DNA
<213> Enterococcus spp.

<400> 116
atgaataaaa taaaagtcgc aattatcttc ggcggttgct cggaggaaca tgatgtgtcg 60

gtaaaatccg caatagaaat tgctgcgaac attaatactg aaaaattcga tccgcactac 120

atcggaatta caaaaaacgg cgtatggaag ctatgcaaga agccatgtac ggaatgggaa 180

gccgatagtc tccccgccat attctccccg gataggaaaa cgcatggtct gcttgtcatg 240

aaagaaagag aatacgaaac tcggcgtatt gacgtggctt tcccggtttt gcatggcaaa 300

tgcggggagg atggtgcgat acagggtctg tttgaattgt ctggtatccc ctatgtaggc 360

tgcgatattc aaagctccgc agcttgcatg gacaaatcac tggcctacat tcttacaaaa 420

aatgcgggca tcgccgtccc cgaatttcaa atgattgaaa aaggtgacaa accggaggcg 480

aggacgctta cctaccctgt ctttgtgaag ccggcacggt caggttcgtc ctttggcgta 540

accaaagtaa acagtacgga agaactaaac gctgcgatag aagcagcagg acaatatgat 600

ggaaaaatct taattgagca agcgatttcg ggctgtgagg tcggctgcgc ggtcatggga 660

aacgaggatg atttgattgt cggcgaagtg gatcaaatcc ggttgagcca cggtatcttc 720

cgcatccatc aggaaaacga gccggaaaaa ggctcagaga atgcgatgat tatcgttcca 780

gcagacattc cggtcgagga acgaaatcgg gtgcaagaaa cggcaaagaa agtatatcgg 840

gtgcttggat gcagagggct tgctcgtgtt gatctttttt tgcaggagga tggcggcatc 900

gttctaaacg aggtcaatac cctgcccggt tttacatcgt acagccgcta tccacgcatg 960

gcggctgccg caggaatcac gcttcccgca ctaattgaca gcctgattac attggcgata 1020

gagaggtga 1029

<210> 117
<211> 1031
<212> DNA
<213> Enterococcus spp.

<400> 117
atgaaaaaaa ttgccgtttt atttggaggg aattctccag aatactcagt gtcactaacc 60

tcagcagcaa gtgtgatcca agctattgac ccgctgaaat atgaagtaat gaccattggc 120

atcgcaccaa caatggattg gtattggtat caaggaaacc tcgcgaatgt tcgcaatgat 180

acttggctag aagatcacaa aaactgtcac cagctgactt tttctagcca aggatttata 240

ttaggagaaa aacgaatcgt ccctgatgtc ctctttccag tcttgcatgg gaagtatggc 300

gaggatggct gtatccaagg actgcttgaa ctaatgaacc tgccttatgt tggttgccat 360

gtcgctgcct ccgcattatg tatgaacaaa tggctcttgc atcaacttgc tgataccatg 420

ggaatcgcta gtgctcccac tttgctttta tcccgctatg aaaacgatcc tgccacaatc 480

gatcgtttta ttcaagacca tggattcccg atctttatca agccgaatga agccggttct 540

tcaaaaggga tcacaaaagt aactgacaaa acagcgctcc aatctgcatt aacgactgct 600

tttgcttacg gttctactgt gttgatccaa aaggcgatag cgggtattga aattggctgc 660

ggcatcttag gaaatgagca attgacgatt ggtgcttgtg atgcgatttc tcttgtcgac 720

ggtttttttg attttgaaga gaaataccaa ttaatcagcg ccacgatcac tgtcccagca 780

ccattgcctc tcgcgcttga atcacagatc aaggagcagg cacagctgct ttatcgaaac 840

ttgggattga cgggtctggc tcgaatcgat tttttcgtca ccaatcaagg agcgatttat 900

ttaaacgaaa tcaacaccat gccgggattt actgggcact cccgctaccc agctatgatg 960

gcggaagtcg ggttatccta cgaaatatta gtagagcaat tgattgcact ggcagaggag 1020

gacaaacgat g 1031

<210> 118
<211> 809
<212> DNA
<213> Abiotrophia adiacens

<400> 118
tggtgctatc ttagtagtat ctgcagctga tggtccaatg cctcaaacac gtgaacacat 60

cttattatca cgtcaagtag gtgttcctta catcgttgta ttcttaaaca aagttgacat 120

ggttgacgat gaagaattat tagaattagt agaaatggaa gttcgtgact tattatcaga 180

atacgatttc ccaggcgatg acactccagt tgttgcaggt tctgctttac gcgctttaga 240

aggcgacgct tcatacraag aaaaaatctt agaattaatg gctgctgttg acgaatacat 300

tccaactcca gaacgygacg ttgacaaacc attcatgatg ccagttgaag acgtgttctc 360

aatcacaggt cgtggtactg ttgctacagg tcgtgttgaa cgtggacaag ttcgtgttgg 420

tgacgaagtt gaaatcgttg gtatttcaga agaaacttca aaaacaactg taactggtgt 480

tgaaatgttc cgtaaattgt tagactacgc tgaagcaggg gataacattg gtacattatt 540

acgtggtgtt acacgtgaca acatcgaacg tggacaagtt cttgctaaac caggaacaat 600

cactccacat actaaattca aagctgaagt ttacgtatta actaaagaag aaggtggacg 660

tcatactcca ttcttctcta actaccgtcc tcaattctac ttccgtacaa cagacatcac 720

tggtgtttgt gtgttaccag aaggcgttga aatggtaatg cctggtgata acgtaactat 780

ggaagttgaa ttaattcacc cagtagcga 809

<210> 119
<211> 817
<212> DNA
<213> Abiotrophia defectiva

<400> 119
cggcgcgatc ctcgttgtat ctgctgctga cggcccaatg ccacaaactc gtgaacacat 60

cctcttgtct cgtcaagttg gtgttcctta catcgtagta ttcttgaaca aagttgacat 120

ggttgacgac gaagaattgc tcgaattagt tgaaatggaa gttcgtgacc tcttgtctga 180

atacgacttc ccaggcgacg acactccagt tatcgctggt tcagctttga aagctttaga 240

aggcgacgct aactacgaag ctaaagtttt agaattgatg gaacaagttg atgcttacat 300

tccagaacca gaacgtgaca ctgacaagcc attcatgatg ccagtcgaag acgtattctc 360

tatcactggt cgtggtactg ttgcaactgg tcgtgttgaa cgtggtcaag ttcgcgttgg 420

tgacgaagtt gaaatcgttg gtatcgaaga agaaacttct aagactaccg ttaccggtgt 480

tgaaatgttc cgtaagttat tggattacgc tgaagctggg gacaacgttg gtaccttgtt 540

acgtggtgta actcgtgacc aaatccaacg tggtcaagta ttatctaaac caggttcaat 600

cactccgyac actaagttcg aagctgaagt gtacgtattg tctaaagaag aaggtggtcg 660

tcacactcca ttcttctcta actaccgtcc acaattctac ttccgtacaa ctgacgtaac 720

tggtgttgtt actttaccag aaggtactga aatggttatg ccaggcgaca acgtacaaat 780

ggttgttgaa ttgatccacc caatcgcgat cgaagaa 817

<210> 120
<211> 754
<212> DNA
<213> Candida albicans

<400> 120
ctctgtcaaa tgggacaaaa acagatttga agaaatcatc aaggaaacct ccaacttcgt 60

caagaaggtt ggttacaacc caaagactgt tccattcgtt ccaatctctg gttggaatgg 120

tgacaacwtg attgaascat ccaccaactg tccatggtac aagggttggg aaaaggaaac 180

caaatccggt aaagttactg gtaagacctt gttagaagct attgacgcta ttgaaccacc 240

aaccagacca accgacaaac cattgagatt gccattrcaa gatgtttaca agatcggtgg 300

tattggtact gtgccagtcg gtagagttga aactggtatc atcaaagccg gtatggtwgt 360

tactttcgcc ccagctggtg ttaccactga agtcaartcc gttgaaatgc atcacgaaca 420

attggctgaa ggtgttccag gtgacaatgt trgtttcaac gttaagaacr tttccgttaa 480

agaaattaga agaggtaacg tttgtggtga ctccaagaac gatccaccaa agggttgtga 540

ctctttcaat gcccaagtca ttgttttgaa ccatccaggt caaatctctg ctggttactc 600

tccagtcttg gattgtcacr ctgcccacat tgcttgtaaa ttcgacrctt tggttgaaaa 660

gattgacaga agaactggta agraattgga agaaaatcca aaattcgtca aatccggtga 720

tgctgctatc gtcaagatgg tcccaaccaa acca 754

<210> 121
<211> 753
<212> DNA
<213> Candida glabrata

<400> 121
tctgtcaagt gggatgaatc cagattcgct gaaatcgtta aggaaacctc caacttcatc 60

aagaaggtcg gttacaaccc aaagactgtt ccattcgtcc caatctctgg ttggaacggt 120

gacaacatga ttgaagccac caccaacgct tcctggtaca agggttggga aaaggaaacc 180

aaggctggtg tcgtcaaggg taagaccttg ttggaagcca ttgacgctat cgaaccacca 240

accagaccaa ctgacaagcc attgagattg ccattgcaag atgtctacaa gatcggtggt 300

atcggtacgg tgccagtcgg tagagtcgaa accggtgtca tcaagccagg tatggttgtt 360

accttcgccc cagctggtgt taccactgaa gtcaagtccg ttgaaatgca ccacgaacaa 420

ttgactgaag gtttgccagg tgacaacgtt ggtttcaacg ttaagaacgt ttccgttaag 480

gaaatcagaa gaggtaatgt ctgtggtgac tccaagaacg acccaccaaa ggctgctgct 540

tctttcaacg ctaccgtcat tgtcttgaac cacccaggtc aaatctctgc tggttactct 600

ccagttttgg actgtcacac cgcccacatt gcttgtaagt tcgaagaatt gttggaaaag 660

aacgacagaa gatccggtaa gaagttggaa gactctccaa agttcttgaa gtccggtgac 720

gctgctttgg ttaagttcgt tccatccaag cca 753

<210> 122

<211>  752
<212>  DNA
<213>  Candida krusei

<400>  122
ccgttaagtg ggatgaaaac agatttgaag aaattgtcaa ggaaacccaa aacttcatca      60

agaaggttgg ttacaaccca aagactgttc cattcgttcc aatctctggt tggaatggtg     120

acaacatgat tgaagcatcc accaactgtc catggtacaa gggttggact aaggaaacca     180

aggcaggtgt tgttaagggt aagaccttat tagaagcaat cgatgctatt gaaccacctg     240

tcagaccaac cgaaaagcca ttaagattac cattacaaga tgtttacaag attggtggta     300

ttggtactgt gccagtcggt agagtcgaaa ccggtgtcat taagccaggt atggttgtca     360

cttttgctcc agcaggtgtc accaccgaag tcaaatccgt tgaaatgcac catgaacaat     420

tagaacaagg tgttccaggt gataacgttg gtttcaacgt taagaacgty tctgtcaagg     480

atatcaagag aggtaacgtt tgtggtgact ccaagaacga cccaccaatg ggtgcagctt     540

ctttcaatgc tcaagtcatt gtcttgaacc accctggtca aatttccgct ggttactctc     600

cagtcttgga ttgtcacact gcccacattg catgtaagtt cgacgaatta atcgaaaaga     660

ttgacagaag aactggtaag tctgttgaag accatccaaa gtcygtcaag tctggtgatg     720

cagctatcgt caagatggtc ccaaccaagc ca                                   752


<210>  123
<211>  754
<212>  DNA
<213>  Candida parapsilosis

<400>  123
ctcagtcaaa tgggacaaga rcagatacga agaaattgtc aaggaaactt ccaacttcgt      60

caagaaggtt ggttacaacc ctaaagctgt cccattcgtc ccaatctctg gttggaacgg     120

tgacaatatg attgaaccat caaccaactg tccatggtac aagggttggg aaaaggaaac     180

taaagctggt aaggttaccg gtaagacctt gttggaagct atcgatgcta tcgarccacc     240

aaccagacca actgacaagc cattgagatt gccattgcaa gatgtctaca agattggtgg     300

tattggaact gtgccagttg gtagagttga aaccggtatc atcaaggctg gtatggttgt     360

tacttttgcc ccagctggtg ttaccactga agtcaagtcc gttgaaatgc accacgaaca     420

attgactgaa ggtgtcccag gtgacaatgt tggtttcaac gtcaagaacg tttcagttaa     480

ggaaatcaga agaggtaacg tytgtggtga ctccaagaac gatccaccaa agggatgtga     540

ytccttcaat gctcaagtta ttgtcttgaa ccacccaggt caaatctctg ctggttactc     600

accagtcttg gattgtcaca ctgcccacat tgcttgtaaa ttcgacactt tgattgaaaa 660

gattgacaga agaaccggta agaaattgga agwtgaacca aaattcatca agtccggtga 720

tgctgcyatc gtcaagatgg tcccaaccaa gcca 754

<210> 124
<211> 753
<212> DNA
<213> Candida tropicalis

<400> 124
tctgttaaat gggacaaraa cagatttgaa gaaattatca aggaaacytc taacttcgtc 60

aagaaggttg gttacaaccc taaggctgtt ccattcgttc caatctcwgg ttggaatggt 120

gacaacatga ttgaagcttc taccaactgt ccatggtaca agggttggga aaaagaaacc 180

aaggctggta aggttaccgg taagactttg ttggaagcca ttgatgctat tgaaccacct 240

tcaagaccaa ctgacaagcc attgagattg ccattgcaag atgtttacaa gattggtggt 300

attggtactg tgccagtcgg tagagttgaa actggtgtca tcaaagccgg tatggttgtt 360

actttygccc cagctggtgt taccactgaa gtcaaatccg tygaaatgca ccacgaacaa 420

ttggctgaag gtgtcccagg tgacaatgtt ggtttcaacg ttaagaacgt ttctgttaaa 480

gaaattagaa gaggtaacgt ttgtggtgac tccaagaacg atccaccaaa gggttgtgac 540

tctttcaacg ctcaagttat tgtcttgaac cacccaggtc aaatytctgc tggttactct 600

ccagtcttgg attgtcacac tgctcatatt gcttgtaaat tcgacacctt ggttgaaaag 660

attgacagaa gaactggtaa gaaattggaa gaaaatccaa aattcgtcaa atccggtgat 720

gctgctattg tcaagatggt tccaaccaaa cca 753

<210> 125
<211> 814
<212> DNA
<213> Corynebacterium accolens

<400> 125
cggcgctatc ctggttgttg ctgcaaccga tggcccgatg ccgcagaccc gcgagcacgt 60

tctgcttgct cgccaggttg gcgttcctta catcctcgtt gcactgaaca agtgcgacat 120

ggttgatgat gaggaaatca tcgagctcgt ggagatggag atctccgagc tgctcgcaga 180

gcaggactac gatgaggaag ctcctatcgt tcacatctcc gctctgaagg cactcgaggg 240

tgacgagaag tgggtacagt ccatcgttga cctgatggat gcctgcgaca actccatccc 300

tgatccggag cgcgctaccg atcagccgtt cttgatgcct atcgaggaca tcttcaccat 360

taccggccgc ggtaccgttg ttaccggccg tgttgagcgt ggtcgtctga acgtcaacga 420

ggacgttgag atcatcggta tccaggagaa gtcccagaac accaccgtta ccggtatcga 480

gatgttccgc aagatgatgg actacaccga ggctggcgac aactgtggtc tgcttctgcg 540

tggtaccaag cgtgaggacg ttgagcgtgg ccaggttgtt atcaagccgg gcgcttacac 600

ccctcacacc aagttcgagg gttccgtcta cgtcctgaag aaggaagagg gcggccgcca 660

caccccgytc atgaacaact accgtcctca gttctacttc cgcaccaccg acgttaccgg 720

tgttgtgaac ctgcctgagg gcaccgagat ggttatgcct ggcgacaacg ttgagatgtc 780

tgttgagctc atccagcctg ttgctatgga cgag 814

<210> 126
<211> 814
<212> DNA
<213> Corynebacterium diphtheriae

<400> 126
cggcgcaatc ctcgttgttg ctgccaccga cggcccaatg cctcagaccc gtgagcacgt 60

tctgctcgct cgccaggtcg gcgttcctta catcctcgtt gctctgaaca agtgcgacat 120

ggttgatgat gaggaaatca tcgagctcgt cgagatggag atccrtgagc tgctcgctga 180

gcaggattac gacgaagagg ctccaatcat ccacatctcc gcactgaagg ctcttgaggg 240

cgacgagaag tggacccagt ccatcatcga cctcatgcag gcttgckatg attccatccc 300

agacccagag cgtgagaccg acaagccatt cctcatgcct atcgaggaca tcttcaccat 360

caccggccgc ggtaccgttg ttaccggccg tgttgagcgt ggctccctga aggtcaacga 420

ggacgtcgag atcatcggta tccgcgagaa kgctaccacc accaccgtta ccggtatcga 480

gatgttccgt aagcttctcg actacaccga ggctggcgac aactgtggtc tgcttctccg 540

tggcgttaag cgcgaagacg ttgagcgtgg ccaggttgtt gttaagccag gcgcttacac 600

ccctcacacc gagttcgagg gctctgtcta cgttctgtcc aaggacgagg gtggccgcca 660

caccccattc ttcgacaact accgcccaca gttctacttc cgcaccaccg acgttaccgg 720

tgttgtgaag cttcctgagg gcaccgagat ggtcatgcct ggcgacaacg tcgacatgtc 780

cgtcaccctg atccagcctg tcgctatgga tgag 814

<210> 127
<211> 814
<212> DNA

<213>  Corynebacterium genitalium

<400>  127
cggcgccatc ctggttgttg ctgcaaccga tggcccgatg ccgcagaccc gtgagcacgt      60

tctgctggct cgccaggttg gcgttccgta catcctagtt gcactgaaca agtgcgacat     120

ggttgatgat gaggagctgc tggagctcgt cgagatggag gtccgcgagc tgctggctga     180

gcaggacttc gacgaggaag cacctgttgt tcacatctcc gcactgaagg ccctggaggg     240

cgacgagaag tgggctaagc agatcctgga gctcatggag gcttgcgaca actccatccc     300

ggatccggag cgcgagaccg acaagccgtt cctgatgccg gttgrggaca tcttcaccat     360

taccggccgc ggtaccgttg ttaccggccg tgttgagcgt ggcgtcctga acctgaacga     420

cgaggtcgag atcctgggca tccgcgagaa gtccaccaag accaccgtta cctccatcga     480

gatgttcaac aagctgctgg acaccgcaga ggctggcgac aacgccgcac tgctgctgcg     540

tggcctgaag cgcgaagatg ttgagcgtgg tcagatcgtt gctaagccgg gcgagtacac     600

cccgcacacc gagttcgagg gctccgtcta cgttctgtcc aaggacgagg gtggccgcca     660

caccccgttc ttcgacaact accgtccgca gttctatttc cgcaccaccg acgttaccgg     720

tgttgtgaag ctgccggagg gcaccgagat ggttatgccg ggcgacaacg ttgacatgtc     780

cgtcaccctg atccagccgg ttgctatgga cgag                                 814


<210>  128
<211>  814
<212>  DNA
<213>  Corynebacterium jeikeium

<400>  128
cggcgccatc ctggttgttg ccgcaaccga tggcccgatg ccgcagaccc gcgagcacgt      60

tctgctggcy cgccaggttg gcgttccgta catcctggtt gcactgaaca agtgtgacat     120

ggttgacgat gaggagctgc tggagctcgt cgagatggag gtccgcgagc tgctggctga     180

gcaggacttc gacgaggaag ctccggttgt tcacatctcc gcactgaagg ccctggaggg     240

cgacgagaag tgggctaacc agattctcga gctgatgcag gcttgcgacg agtctatccc     300

ggatccggag cgcgagaccg acaagccgtt cctgatgccg gttgwggaca tcttcaccat     360

taccggtcgc ggtaccgttg ttaccggccg tgttgagcgt ggcatcctga acctgaacga     420

cgaggttgag atcctgggta tccgcgagaa gtcccagaag accaccgtta cctccatcga     480

gatgttcaac aagctgctgg acaccgcaga ggctggcrac aacgctgcac tgctgctgcg     540

tggtctgaag cgcgaggacg ttgagcgtgg ccagatcatc gctaagccgg gcgagtacac 600

cccgcacacc gagttcgagg gctccgtcta cgttctgtcc aaggacgagg gcggccgcca 660

caccccgttc ttcgacaact accgtccgca gttctacttc cgcaccaccg acgttaccgg 720

tgttgtgaag ctgcctgagg gcaccgagat ggttatgccg ggcgacaacg tygacatgtc 780

cgtcaccctg atccagccgg ttgctatgga cgag 814

<210> 129
<211> 748
<212> DNA
<213> Corynebacterium pseudodiphtheriticum

<400> 129
cggcgctatc ttggttgttg cagctaccga cggcccaatg ccacagactc gcgagcacgt 60

tctgctggct cgccaggttg gcgttcctta catcctggtt gcactaaaca agtgcgacat 120

ggttgacgac gaggaaatcc tcgagctcgt cgagatggag atccgcgaat tgctggctga 180

ccaggaattc gacgaagaag ctccaatcgt tcacatctcc gcagtcggcg ccttggaagg 240

cgaagagagg tgggttaacg ccatcgttga actgatggat gcttgtgacg agtcgatccc 300

tgatccagac cgtgctaccg acaagccatt cctgatgcct atcgaggaca tcttcaccat 360

taccggtcgt ggcaccgttg ttacgggtcg tgttgagcgt ggttccctga aggtcaacga 420

agaagtcgag atcatcggca tcaaggaaaa gtcccagaag accaccatca ccggtatcga 480

aatgttccgc aagatgctgg actacaccga ggccggcgac aacgctggtc tgctgcttcg 540

cggtaccaag cgtgaagacg ttgagcgtgg acaggttatc gttgctccag gtgcttacag 600

cacccacaag aagttcgaag gttccgtcta cgttctttcc aaggacgagg gcggccgcca 660

caccccgttc ttcgacaact accgtcctca gttctacttc cgcaccaccg acgttaccgg 720

tgttgttacc ctgcctgagg gcaccgag 748

<210> 130
<211> 813
<212> DNA
<213> Corynebacterium striatum

<400> 130
ggcgctatct tggttgttgc tgcaaccgat ggcccgrtgc cgcagacccg cgagcacgtt 60

cttctggctc gccaggttgg cgttccttac atcctcgttg cactgaacaa gtgcgacatg 120

gttgacgacg aggaaattat cgagctcgtc gagatggaga tccgcgaact gctcgcagag 180

caggactacg atgaggaagc tccgatcgtt cacatctctg ctctgaaggc tcttgagggc 240

grcgagaagt gggtacaggc tatcgttgac ctgatgcagg cttgcgatga ctccatcccg 300

gatccggagc gcgagctgga caagccgttc ctgatgccaa tcgaggacat cttcaccatc 360

accggccgcg gtaccgttgt tactggccgt gttgagcgtg gctccctgaa cgtcaacgag 420

gacgttgaga tcatcggtat ccaggacarg tccatctcca ccaccgttac cggtatcgag 480

atgytccgca agatgatgga ctacaccgag gctggcgaca actgtggtct gcttctgcgt 540

ggtaccaagc gtgaagaggt tgagcgcggc caggttgtta ttaagccggg cgcttacacc 600

cctcacaccc agttcgaggg ttccgtctac gtcctgaaga aggaagaggg cggccgccac 660

accccgttca tggacaacta ccgtccgcag ttctacttcc gcaccaccga cgttaccggc 720

gtcatcaagc tgcctgaggg caccgagatg gttatgcctg gcgacaacgt cgagatgtcy 780

gtcgagctga tccagccggt cgctatggac gag 813

<210> 131
<211> 817
<212> DNA
<213> Enterococcus avium

<400> 131
cggagctatc ttagtagtat ctgctgctga tggccctatg cctcaaactc gtgaacacat 60

cttgttatct cgtaacgttg gtgttcctta catcgttgta ttcttaaaca aaatggatat 120

ggttgacgat gaagaattac ttgaattagt tgaaatggaa gttcgtgact tattaactga 180

atacgacttc ccaggcgacg acactccagt tatcgcaggt tcagcgttga aagctttaga 240

aggcgacgct tcatacgaag aaaaaatctt agaattaatg gctgctgttg acgaatatat 300

cccaacacca gttcgtgata ctgacaaacc attcatgatg ccagtcgaag acgtattctc 360

aatcactggt cgtggtactg ttgcaactgg tcgtgttgaa cgtggacaag ttcgcgttgg 420

tgacgaagtt gaaatcgtag gtatcgctga cgaaactgct aaaacaactg ttacaggtgt 480

tgaaatgttc cgtaaattgt tagactacgc tgaagcaggt gacaacatcg gtgctttgtt 540

acgtggtgtt gcacgtgaag atatccaacg tggacaagta ttggctaaac cagcttcaat 600

cactccacat acaaaattct ctgcagaagt ttatgttcta actaaagaag aaggtggacg 660

tcatactcca ttcttcacta actaccgtcc tcagttctac ttccgtacaa ctgacgtaac 720

tggtgtagtt gatctaccag aaggtactga aatggtwatg cctggggata acgtaactat 780

ggaagttgaa ttgatycacc caatygcggt agaagac 817

<210>  132
<211>  817
<212>  DNA
<213>  Enterococcus faecalis

<400>  132
cggagctatc ttagtagttt ctgctgctga tggtcctatg cctcaaacac gtgaacatat      60

cttattatca cgtaacgttg gtgtaccata catcgttgta ttcttaaaca aaatggatat     120

ggttgatgac gaagaattat tagaattagt agaaatggaa gttcgtgact tattatcaga     180

atacgatttc ccaggcgatg atgttccagt tatcgcaggt tctgctttga aagctttaga     240

aggcgacgag tcttatgaag aaaaaatctt agaattaatg gctgcagttg acgaatatat     300

cccaactcca gaacgtgata ctgacaaacc attcatgatg ccagtcgaag acgtattctc     360

aatcactgga cgtggtactg ttgctacagg acgtgttgaa cgtggtgaag ttcgcgttgg     420

tgacgaagtt gaaatcgttg gtattaaaga cgaaacatct aaaacaacyg ttacaggtgt     480

tgaaatgttc cgtaaattat tagactacgc tgaagcaggc gacaacmtcg gtgctttatt     540

acgtggtgta gcacgtgaag atatcgaacg tggacaagta ttagctaaac cagctacaat     600

cactccacac acaaaattca aagctgaagt atacgtatta tcaaaagaag aaggcggacg     660

tcacactcca ttcttcacta actaccgtcc tcaattctac ttccgtacaa cagacgttac     720

tggtgttgta gaattgccag aaggtactga aatggtaatg cctggtgata acgttgctat     780

ggacgttgaa ttaattcacc caatcgctat cgaagac                              817

<210>  133
<211>  774
<212>  DNA
<213>  Enterococcus faecium

<400>  133
cggagctatc ttggtagttt ctgctgctga cggcccaatg cctcaaactc gtgaacacat      60

cctattgtct cgtcaagttg gtgttcctta catcgttgta ttcttgaaca aagtagacat     120

ggttgatgac gaagaattac tagaattagt tgaaatggaa gttcgtgacc tattaacaga     180

atacraattc cctggtgrcg atgttcctgt agttgctgga tcagctttga aagctctaga     240

aggcgacgct tcatacgaag aaaaaattct tgaattaatg gctgcagttg acgaatacat     300

cccaactcca gaacgtgaca acgacaaacc attcatgatg ccagttgaag acgtgttctc     360

aattactgga cgtggtactg ttgctacagg tcgtgttgaa cgtggacaag ttcgcgttgg     420

tgacgaagtt gaagttgttg gtattgctga agaaacttca aaaacaacag ttactggtgt     480

tgaaatgttc cgtaaattgt tagacyacgc tgaagctgga gacracattg gtgctttact 540

acgtggtgtt gcacgtgaag acatccaacg tggacaagtt ttagctaaac caggtacaat 600

cacacctcrt acaaaattct ctgcagaagt atacgtgttg acaaaagaag aaggtggacg 660

tcatactcca ttcttcacta actaccgtcc acaattctac ttccgtacaa ctgacgtaac 720

aggtgttgtt gaattaccag aaggaactga aatggtcatg cccggtgaca acgt 774

<210> 134
<211> 809
<212> DNA
<213> Enterococcus gallinarum

<400> 134
cggtgcgatc ttagtagtat ctgctgctga cggtcctatg cctcaaactc gtgaacacat 60

cttgttatca cgtaacgttg gcgtaccata catcgttgtt ttcttgaaca aaatggatat 120

ggttgaygac gaagaattgc tagaattagt tgaaatggaa gttcgtgacc tattgtctga 180

atatgacttc ccaggcgacg atgttcctgt aatcgccggt tctgctttga aagctcttga 240

aggagatcct tcatacgaag aaaaaatcat ggaattgatg gctgcagttg acgaatacgt 300

tccaactcca gaacgtgata ctgacaaacc attcatgatg ccagtcgaag acgtattctc 360

aatcactgga cgtggtactg ttgctacagg ccgtgttgaa cgtggacaag ttcgcgttgg 420

tgatgaagta gaaatcgttg gtattgctga cgaaactgct aaaacaactg taacaggtgt 480

tgaaatgttc cgtaaattgt tagactatgc tgaagcaggg gataacattg gtgcattgct 540

acgtggggtt gctcgtgaag acatccaacg tggacaagta ttggctaaag ctggtacaat 600

cacacctcat acaaaattca aagctgaagt ttatgttttg acaaaagaag aaggtggacg 660

tcacactcca ttcttcacta actaccgtcc tcagttctac ttccgtacaa ctgacgtaac 720

tggtgttgtt gaattaccag aaggaactga aatggtgatg cctggcgaca acgtgaccat 780

cgacgttgaa ttgatrcacc caatcgctc 809

<210> 135
<211> 823
<212> DNA
<213> Gardnerella vaginalis

<400> 135
tggcgcaatc ctcgtggttg ctgctaccga cggtccaatg gctcagaccc gtgaacacgt 60

cttgcttgct aagcaggtcg gcgttccaaa aattcttgtt gctttgaaca agtgcgatat 120

ggttgacgac gaagagctta tcgatctcgt tgaagaagag gtccgtgacc tcctcgaaga 180

aaacggcttc gatcgcgatt gcccagtcyt ccgtacttcc gcttacggcg ctttgcatga 240

tgacgctcca gaccacgaca agtgggtaga gaccgtcaag gaactcatga aggctgttga 300

cgagtacatc ccaaccccaa ctcacgatct tgacaagcca ttcttgatgc caatcgaaga 360

tgtgttcacc atctccggtc gtggtyccgt tgtcaccggt cgtgttgagc gtggtaagct 420

cccaatcaac accccagttg agatcgttgg tttgcgcgat acccagacca ccaccgtcac 480

ctctatcgag accttccaca agcagatgga tgaggcagag gctggcgata acactggtct 540

tcttctccgc ggtatcaacc gtaccgacgt tgagcgtggt caggttgtgg ctgctccagg 600

ttctgtgact ccacacacca agttcgaagg cgaagtttac gtcttgacca aggacgaagg 660

tggccgtcac tcgccattct tctccaacta ccgtccacag ttctacttcc gtaccaccga 720

tgttactggc gttatcacct tgccagacgg catcgaaatg gttcagccag gcgatcacgc 780

aaccttcact gttgagttga tccaggctat cgcaatggaa gag 823

<210> 136
<211> 817
<212> DNA
<213> Listeria innocua

<400> 136
cggagctatc ttagtagtat ctgctgctga tggcccaatg ccacaaactc gtgaacatat 60

cttactttca cgtcaagttg gtgttccata catcgttgta ttcatgaaca aatgtgacat 120

ggttgacgat gaagaattac tagaattagt tgaaatggaa attcgtgatc tattaactga 180

atatgaattc cctggcgatg acattcctgt aatcaaaggt tcagctctta aagcacttca 240

aggtgaagct gactgggaag ctaaaattga cgagttaatg gaagctgtag attcttacat 300

tccaactcca gaacgtgata ctgacaaacc attcatgatg ccagttgagg atgtattctc 360

aatcactggt cgtggaacag ttgcaactgg acgtgttgaa cgtggacaag ttaaagttgg 420

tgacgaagta gaagttatcg gtattgaaga agaaagcaaa aaagtagtag taactggagt 480

agaaatgttc cgtaaattac tagactacgc tgaagctggc gacaacattg gcgcacttct 540

acgtggtgtt gctcgtgaag atatccaacg tggtcaagta ttagctaaac caggttcgat 600

tactccacac actaacttca aagctgaaac ttatgtttta actaaagaag aaggtggacg 660

tcacactcca ttcttcaaca actaccgccc acaattctat ttccgtacta ctgacgtaac 720

tggtattgtt acacttccag aaggtactga aatggtaatg cctggtgata acattgagct 780

tgcagttgaa ctaattgcac caatcgctat cgaagac 817

<210> 137
<211> 818
<212> DNA
<213> Listeria ivanovii

<400> 137
cggagctatc ttagtagtat ctgctgctga tggtccaatg ccacaaactc gtgaacatat 60

tcttactttc acgtcaagtt ggtgttccat acatcgttgt attcatgaac aaatgtgaca 120

tggttgacga tgaagaatta cttgaattag ttgaaatgga aattcgtgat ctattaactg 180

aatatgaatt ccctggcgac gacattcctg taatcaaagg ttcagctctt aaagcacttc 240

aaggtgaagc tgattgggaa gctaaaattg acgagttaat ggaagctgta gattcttaca 300

ttccaactcc agaacgtgat actgacaaac cattcatgat gccagttgag gatgtattct 360

caatcactgg tcgtggaaca gttgcaactg gacgtgttga acgtggacaa gttaaagttg 420

gtgacgaagt agaagttatc ggtattgaag aagaaagcaa aaaagtagta gtaactggag 480

tagaaatgtt ccgtaaatta ctagactacg ctgaagctgg cgacaacatt ggcgcacttc 540

tacgtggtgt tgctcgtgaa gatatccaac gtggtcaagt attagctaaa ccaggttcga 600

ttactccaca tactaacttc aaagctgaaa cttatgtttt aactaaagaa gaaggtggac 660

gtcatactcc attcttcaac aactaccgcc cacaattcta tttccgtact actgacgtaa 720

ctggtattgt tacacttcca gaaggtactg aaatggtaat gcctggtgat aacattgagc 780

ttgcagttga actaattgca ccaatcgcta tcgaagac 818

<210> 138
<211> 817
<212> DNA
<213> Listeria monocytogenes

<400> 138
cggagctatc ttagtagtat ctgctgctga tggcccaatg ccacaaactc gtgaacatat 60

cttactttca cgtcaagttg gtgttccata catcgttgta ttcatgaaca aatgtgacat 120

ggttgacgat gaagaattac tagaattagt tgaaatggaa attcgtgatc tattaactga 180

atatgaattc cctggcgatg acattcctgt aatcaaaggt tcagctctta aagcacttca 240

aggtgaagct gactgggaag ctaaaattga cgagttaatg gaagctgtag attcttacat 300

tccaactccw gaacgtgata ctgacaaacc attcatgatg ccagttgagg atgtattctc 360

aatcactggt cgtggaacag ttgcaactgg acgtgttgaa cgtggacaag ttaaagttgg 420

tgacgaagta gaagttatcg gtatcgaaga agaaagcaaa aaagtagtag taactggagt 480

agaaatgttc cgtaaattac tagactacgc tgaagctggc gacaacattg gcgcacttct 540

acgtggtgtt gctcgtgaag atatccaacr tggtcaagta ttagctaaac caggttcgat 600

tactccacac actaacttca aagctgaaac ttatgtttta actaaagaag aaggtggacg 660

tcacactcca ttcttcaaca actaccgccc acaattctat ttccgtacta ctgacgtaac 720

tggtattgtt acacttccag aaggtactga aatggtaayg cctggtgata acattgagct 780

tgcagttgaa ctaattgcac caatcgctat cgaagac 817

<210> 139
<211> 817
<212> DNA
<213> Listeria seeligeri

<400> 139
cggagctatc ttagtagtat ctgctgctga tggcccaatg ccacaaactc gtgaacatat 60

cttactttca cgtcaagttg gtgttccata catcgttgta ttcatgaaca aatgtgacat 120

ggttgacgat gaagaattac ttgaattagt tgaaatggaa attcgtgatc tattaactga 180

atatgaattc cctggtgatg acattcctgt aatcaaaggt tcagctctta aagcacttca 240

aggtgaagct gactgggaag ctaaaattga cgagttaatg gaagctgtag attcttacat 300

tccaactcca gaacgtgata ctgacaaacc attcatgatg ccagttgagg atgtattctc 360

aatcactggt cgtggaactg ttgcaactgg acgtgttgaa cgtggacaag ttaaagttgg 420

tgacgaagta gaagttatcg gtattgaaga agaaagcaaa aaagtaatag taactggagt 480

agaaatgttc cgtaaattac tagactacgc tgaagctggc gacaacattg gcgcacttct 540

acgtggtgtt gctcgtgaag atatccaacg tggtcaagta ttagctaaac caggttcgat 600

tactccacat actaacttca aagctgaaac ttatgtttta actaaagaag aaggtggacg 660

tcacactcca ttcttcaaca actaccgccc acaattctat ttccgtacta ctgacgtaac 720

tggtattgtt acacttccag aaggtactga aatggtaatg cctggtgata acattgagct 780

tgcagttgaa ctaattgcac caatcgctat cgaagac 817

<210> 140
<211> 814
<212> DNA
<213> Staphylococcus aureus

<400> 140

cggtggtatc ttagtagtat ctgctgctga cggtccaatg ccacaaactc gtgaacacat 60

tcttttatca cgtaacgttg gtgtaccagc attagtagta ttcttaaaca aagttgacat 120

ggttgacgat gaagaattat tagaattagt agaaatggaa gttcgtgact tattaagcga 180

atatgacttc ccaggtgacg atgtacctgt aatcgctggt tcagcattar aagctttaga 240

aggcgatgct caatacgaag aaaaaatctt agaattartg gaagctgtag atacttacat 300

tccaactcca gaacgtgatt ctgacaaacc attcatgatg ccagttgagg acgtattctc 360

aatcactggt cgtggtactg ttgctacagg ccgtgttgaa cgtggtcaaa tcaaagttgg 420

tgaagaagtt gaaatcatcg gtttacatga cacatctaaa acaactgtta caggtgttga 480

aatgttccgt aaattattag actacgctga agctggtgac aacattggtg cattattacg 540

tggtgttgct cgtgaagacg tacaacgtgg tcaagtatta gctgctcctg gttcaattac 600

accacatact gaattcaaag cagaagtata cgtattatca aaagacgaag gtggacgtca 660

cactccattc ttctcaaact atcgtccaca attctatttc cgtactactg acgtaactgg 720

tgttgttcac ttaccagaag gtactgaaat ggtaatgcct ggtgataacg ttgaaatgac 780

agtagaatta atcgctccaa tcgcgattga agac 814

<210> 141
<211> 814
<212> DNA
<213> Staphylococcus epidermidis

<400> 141
cggcggtatc ttagttgtat ctgctgctga cggtccaatg ccacaaactc gtgaacacat 60

cttattatca cgtaacgttg gtgtaccagc attagttgta ttcttaaaca aagttgacat 120

ggtagacgac gaagaattat tagaattagt tgaaatggaa gttcgtgact tattaagcga 180

atatgacttc ccaggtgacg atgtacctgt aatcgctggt tctgcattaa aagcattaga 240

aggcgatgct gaatacgaac aaaaaatctt agacttaatg caagcagttg atgattacat 300

tccaactcca gaacgtgatt ctgacaaacc attcatgatg ccagttgagg acgtattctc 360

aatcactggt cgtggtactg ttgctacagg ccgtgttgaa cgtggtcaaa tcaaagtwgg 420

tgaagaagtt gaaatcatcg gtatgcacga aacttctaaa acaactgtta ctggtgtaga 480

aatgttccgt aaattattag actacgctga agctggtgac aacatcggtg ctttattacg 540

tggtgttgca cgtgaagacg tacaacgtgg tcaagtatta gctgctcctg gttctattac 600

accacacaca aaattcaaag ctgaagtata cgtattatct aaagatgaag gtggacgtca 660

cactccattc ttcactaact atcgcccaca attctatttc crtactactg acgtaactgg 720

tgttgtaaac ttaccagaag gtacagaaat ggttatgcct ggcgacaacg ttgaaatgac 780

agttgaatta atcgctccaa tcgctatcga agac 814

<210> 142
<211> 817
<212> DNA
<213> Staphylococcus saprophyticus

<400> 142
cggagctatc ttagtagtat ctgctgctga tggcccaatg ccacaaactc gtgaacacat 60

tcttttatca cgtracgttg gtgytccagc attagttgta ttcttaaaca aagttgacat 120

ggttgacgay gaagaattat tagaattrgt agaaatggaa gttcgtgrct tattaagcga 180

atatgacttc ccaggtgacg atgtacctgt aatctctggt tctgcattaa aagctttaga 240

aggcgacgct gactatgagc aaaaaatctt agacttaatg caagctgttg atgactycat 300

tccaacacca gaacgtgatt ctgacaaacc attcatgatg ccagttgagg acgtattctc 360

aatcactggt cgtggtactg ttgctacagg ccgtgttgaa cgtggtcaaa tcaaagtcgg 420

tgaagaaatc garatcatcg gtatgcaaga agaatcaagc aaaacaactg ttactggtgt 480

agaaatgttc cgtaaattat tagactacgc tgaagctggt gacaacattg gtgcattatt 540

acgtggtgtt tcacgtgatg atgtacaacg tggtcaagtt ttagctgctc ctggtactat 600

cacaccacat acaaaattca aagcggatgt ttacgtttta tctaaagatg aaggtggtcg 660

tcatacgcca ttcttcacta actaccgccc acaattctat ttccgtacta ctgacgtaac 720

tggtgttgtt aacttaccag aaggtactga aatggttatg cctggcgata acgttgaaat 780

ggatgttgaa ttaatttctc caatcgctat tgaagac 817

<210> 143
<211> 817
<212> DNA
<213> Staphylococcus simulans

<400> 143
cggcggtatc ttagtagtat ctgctgcaga tggtccaatg ccacaaactc gtgaacacat 60

cttattatca cgtaacgttg gtgtaccagc tttagttgta ttcttaaaca aagctgacat 120

ggttgacgac gaagaattat tagaattagt tgaaatggaa gttcgtgact tattatctga 180

atacgacttc cctggtgacg atgtaccagt tatcgttggt tctgcattaa aagctttaga 240

aggcgaccca gaatacgaac aaaaaatctt agacttaatg caagctgtag atgactacat 300

cccaactcca gaacgtgact ctgataaacc attcatgatg ccagttgagg acgtattctc 360

aatcactggt cgtggtactg tagcaacagg ccgtgttgaa cgtggtcaaa tcaaagtcgg 420

tgaagaagtt gaaatcatcg gtatcactga agaaagcaag aaaacaacag ttacaggtgt 480

agaaatgttc cgtaaattat tagactacgc tgaagctggt gacaacatcg gtgctttatt 540

acgtggtgtt gcacgtgaag acgtacaacg tggacaagta ttagcagctc ctggctctat 600

tactccacac acaaaattca aagctgatgt ttacgtttta tctaaagaag aaggtggacg 660

tcatactcca ttcttcacta actaccgccc acaattctac ttccgtacta ctgacgtaac 720

tggcgttgtt cacttaccag aaggtactga aatggttatg cctggcgata acgtagaaat 780

gactgttgaa ttgatcgctc caatcgcgat tgaagac 817

<210> 144
<211> 817
<212> DNA
<213> Streptococcus agalactiae

<400> 144
cggagctatc cttgtagttg cttcaactga tggaccaatg ccacaaactc gtgagcacat 60

ccttctttca cgtcaagttg gtgttaaaca ccttatcgta ttcatgaaca aagttgacct 120

tgttgatgat gaagaattgc ttgaattggt tgaaatggaa attcgtgacc ttctttcaga 180

atacgacttc ccaggtgatg accttccagt tatccaaggt tcagctctta aagcacttga 240

aggcgacgaa aaatacgaag acatcatcat ggaattgatg agcactgttg atgagtacat 300

tccagaacca gaacgtgata ctgacaaacc tttacttctt ccagttgaag atgtattctc 360

aatcactgga cgtggtacag ttgcttcagg acgtatcgac cgtggtactg ttcgtgtcaa 420

cgacgaagtt gaaatcgttg gtattaaaga agatatccaa aaagcagttg ttactggtgt 480

tgaaatgttc cgtaaacaac ttgacgaagg tcttgcaggg gacaacgttg gtgttcttct 540

tcgtggtgtt caacgtgatg aaatcgaacg tggtcaagtt cttgctaaac caggttcaat 600

caacccacac actaaattta aaggtgaagt ttacatcctt tctaaagaag aaggtggacg 660

tcatactcca ttcttcaaca actaccgtcc acaattctac ttccgtacaa ctgacgtaac 720

aggttcaatc gaacttccag caggaacaga aatggttatg cctggtgata acgttactat 780

cgaagttgaa ttgattcacc caatcgccgt agaacaa 817

<210> 145
<211> 817

<212>  DNA
<213>  Streptococcus pneumoniae

<400>  145
cggagctatc cttgtagtag cttcaactga cggaccaatg ccacaaactc gtgagcacat      60

ccttctttca cgtcaggttg gtgttaaaca ccttatcgtc ttcatgaaca aagttgactt     120

ggttgacgac gaagaattgc ttgaattggt tgaaatggaa atccgtgacc tattgtcaga     180

atacgacttc ccaggtgacg atcttccagt tatccaaggt tcagcactta aagctcttga     240

aggtgactct aaatacgaag acatcgttat ggaattgatg aacacagttg atgagtatat     300

cccagaacca gaacgtgaca ctgacaaacc attgcttctt ccagtcgagg acgtattctc     360

aatcactgga cgtggtacag ttgcttcagg acgtatcgac cgtggtatcg ttaaagtcaa     420

cgacgaaatc gaaatcgttg gtatcaaaga agaaactcra aaagcagttg ttactggtgt     480

tgaaatgttc cgtaaacaac ttgacgaagg tcttgctgga gataacgtag gtgtccttct     540

tcgtggtgtt caacgtgatg aaatcgaacg tggacaagtt atcgctaaac caggttcaat     600

caacccacac actaaattca aaggtgaagt ctacatcctt actaaagaag aaggtggacg     660

tcacactcca ttcttcaaca actaccgtcc acaattctac ttccgtacta ctgacgttac     720

aggttcaatc gaacttccag caggtactga aatggtaatg cctggtgata acgtgacaat     780

cgacgttgag ttgattcacc caatcgccgt agaacaa                              817


<210>  146
<211>  817
<212>  DNA
<213>  Streptococcus salivarius

<400>  146
cggtgcgatc cttgtagtag catctactga cggaccaatg ccacaaactc gtgagcacat      60

ccttctttca cgtcaggttg gtgttaaaca ccttatcgtc ttcatgaaca aagttgactt     120

ggttgacgat gaagaattgc ttgaattggt tgaaatggaa atccgtgacc ttctttcaga     180

atacgatttc ccaggtgatg acattccagt tatccaaggt tcagctctta aagctcttga     240

aggtgattct aaatacgaag acatcatcat ggacttgatg aacactgttg acgaatacat     300

cccagaacca gaacgtgaca ctgacaaacc attgttgctt ccagtcgaag acgtattctc     360

aatcactggt cgtggtactg ttgcttcagg acgtatcgac cgtggtgttg ttcgtgtcaa     420

tgacgaagtt gaaatcgttg gtcttaaaga agacatccaa aaagcagttg ttactggtgt     480

tgaaatgttc cgtaaacaac ttgacgragg tattgccgga gataacgtcg gtgttcttct     540

tcgtggtatc caacgtgatg aaatcgaacg tggtcaagta ttggctgcac ctggttcaat 600

caacccacac actaaattca aaggtgaagt ttacatcctt tctaaagaag aaggtggacg 660

tcacactcca ttcttcaaca actaccgtcc acagttctac ttccgtacaa ctgacgtaac 720

aggttcaatc gaacttcctg caggtactga aatggttatg cctggtgata acgtgactat 780

cgacgttgag ttgatccacc caatcgccgt tgaacaa 817

<210> 147
<211> 897
<212> DNA
<213> Agrobacterium tumefaciens

<400> 147
aacatgatca ccggtgctgc cgagatggac ggcgcgatcc tggtttgctc ggctgccgac 60

ggcccgatgc cacagacccg cgagcacatc ctgcttgccc gtcaggtggg cgttccggcc 120

atcgtcgtgt tcctcaacaa ggtcgaccag gttgacgacg ccgagcttct cgagctcgtc 180

gagcttgaag ttcgcgaact tctgtcgtcc tacgacttcc cgggcgacga tatcccgatc 240

atcaagggtt cggcacttgc tgctcttgaa gattctgaca agaagatcgg tgaagacgcg 300

atccgcgagc tgatggctgc tgtcgacgcc tacatcccga cgcctgagcg tccgatcgac 360

cagccgttcc tgatgccgat cgaagacgtg ttctcgatct cgggtcgtgg tacggttgtg 420

acgggtcgcg ttgagcgcgg tatcgtcaag gttggtgaag aagtcgaaat cgtcggcatc 480

cgtccgacct cgaagacgac tgttaccggc gttgaaatgt tccgcaagct gctcgaccag 540

ggccaggccg gcgacaacat cggtgcactc gttcgcggcg ttacccgtga cggcgtcgag 600

cgtggtcaga tcctgtgcaa gccgggttcg gtcaagccgc acaagaagtt catggcagaa 660

gcctacatcc tgacgaagga agaaggcggc cgtcatacgc cgttcttcac gaactaccgt 720

ccgcagttct acttccgtac gactgacgtt accggtatcg tttcgcttcc tgaaggcacg 780

gaaatggtta tgcctggcga caacgtcact gttgaagtcg agctgatcgt tccgatcgcg 840

atggaagaaa agctgcgctt cgctatccgc gaaggcggcc gtaccgtcgg cgccggc 897

<210> 148
<211> 885
<212> DNA
<213> Bacillus subtilis

<400> 148
atgatcactg gtgctgcgca aatggacgga gctatccttg tagtatctgc tgctgatggc 60

ccaatgccac aaactcgtga gcacatcctt ctttctaaaa acgttggtgt accatacatc 120

gttgtattct taaacaaatg cgacatggta gacgacgaag agcttcttga actagttgaa 180

atggaagttc gcgatcttct tagcgaatac gacttccctg gtgatgatgt accagttgtt 240

aaaggttctg ctcttaaagc tcttgaagga gacgctgagt gggaagctaa aatcttcgaa 300

cttatggatg cggttgatga gtacatccca actccagaac gcgacactga aaaaccattc 360

atgatgccag ttgaggacgt attctcaatc actggtcgtg gtacagttgc tactggccgt 420

gtagaacgcg gacaagttaa agtcggtgac gaagttgaaa tcatcggtct tcaagaagag 480

aacaagaaaa caactgttac aggtgttgaa atgttccgta agcttcttga ttacgctgaa 540

gctggtgaca acattggtgc ccttcttcgc ggtgtatctc gtgaagaaat ccaacgtggt 600

caagtacttg ctaaaccagg tacaatcact ccacacagca aattcaaagc tgaagtttac 660

gttctttcta aagaagaggg tggacgtcat actccattct tctctaacta ccgtcctcag 720

ttctacttcc gtacaactga cgtaactggt atcatccatc ttccagaagg cgtagaaatg 780

gttatgcctg gagataacac tgaaatgaac gttgaactta tttctacaat cgctatcgaa 840

gaaggaactc gtttctctat tcgtgaaggc ggacgtactg ttggt 885

<210> 149
<211> 882
<212> DNA
<213> Bacteroides fragilis

<400> 149
atggttactg gtgctgctca gatggacggt gctatcattg tagttgctgc tactgatggt 60

ccgatgcctc agactcgtga gcacatcctt ttggctcgtc aggtaaacgt tccgaagctg 120

gttgtattca tgaacaagtg cgatatggtt gaagatgctg agatgttgga acttgttgaa 180

atggaaatga gagaattgct ttcattctat gatttcgacg gtgacaatac tccgatcatt 240

cagggttctg ctcttggtgc attgaacggc gtagaaaaat gggaagacaa agtaatggaa 300

ctgatggaag ctgttgatac ttggattcca ctgcctccgc gcgatgttga taaacctttc 360

ttgatgccgg tagaagacgt gttctctatc acaggtcgtg gtactgtagc tacaggtcgt 420

atcgaaactg gtgttatcca tgtaggtgat gaaatcgaaa tcctcggttt gggtgaagat 480

aagaaatcag ttgtaacagg tgttgaaatg ttccgcaaac ttctggatca gggtgaagct 540

ggtgacaacg taggtctgtt gcttcgtggt gttgacaaga acgaaatcaa acgtggtatg 600

gttctttgta aaccgggtca gattaaacct cactctaaat tcaaagcaga ggtttatatc 660

ctgaagaaag aagaaggtgg tcgtcacact ccattccata acaaatatcg tcctcagttc 720

tacctgcgta ctatggactg tacaggtgaa atcactcttc cggaaggaac tgaaatggta 780

atgccgggtg ataacgtaac tatcactgta gagttgatct atccggttgc actgaacatc 840

ggtcttcgtt tcgctatccg cgaaggtgga cgtacagtag gt 882

<210> 150
<211> 888
<212> DNA
<213> Borrelia burgdorferi

<400> 150
aatatgatta caggagcagc tcaaatggat gcagcgatac ttttagttgc tgctgatagt 60

ggtgctgagc ctcaaacaaa agagcatttg cttcttgctc aaagaatggg aataaagaaa 120

ataatagttt ttttaaataa attggactta gcagatcctg aacttgttga gcttgttgaa 180

gttgaagttt tagaacttgt tgaaaaatat ggcttttcag ctgatactcc aataatcaaa 240

ggttcagctt ttggggctat gtcaaatcca gaagatcctg aatctacaaa atgcgttaaa 300

gaacttcttg aatctatgga taattatttt gatcttccag aaagagatat tgacaagcca 360

tttttgcttg ctgttgaaga tgtattttct atttcaggaa gaggcactgt tgctactggg 420

cgtattgaaa gaggtattat taaagttggt caagaagttg aaatagttgg aattaaagaa 480

accagaaaaa ctactgttac tggtgttgaa atgttccaga aaattcttga gcaaggtcaa 540

gcaggggata atgttggtct tcttttgaga ggcgttgata aaaaagacat tgagaggggg 600

caagttttgt cagctccagg tacaattact ccacacaaga aatttaaagc ttcaatttat 660

tgtttgacta aagaagaagg cggtaggcac aagccatttt tcccagggta tagaccacag 720

ttctttttta gaacaaccga tgttactgga gttgttgctt tagagggcaa agaaatggtt 780

atgcctggtg ataatgttga tattattgtt gagctgatct cttcaatagc tatggataag 840

aatgtagaat ttgctgttcg agaaggtgga agaaccgttg cttcagga 888

<210> 151
<211> 894
<212> DNA
<213> Brevibacterium linens

<400> 151
aacatgatca ccggtgccgc tcagatggac ggtgcgatcc tcgtcgtcgc cgctaccgac 60

ggaccgatgc cccagacccg tgagcacgtg ctgctcgcgc gtcaggtcgg cgttccctac 120

atcgtcgtgg ctctgaacaa gtccgacatg gtcgatgacg aggagctcct cgagctcgtc 180

gaattcgagg tccgcgacct gctctcgagc caggacttcg acggagacaa cgctccggtc 240

attccggtgt ccgctctcaa ggcgctggaa ggcgacgaga agtgggtcaa gagcgttcag 300

gatctcatgg ctgccgtcga tgacaacgtt ccggagccgg agcgcgatgt cgacaagccg 360

ttcctcatgc ccgtcgagga cgtcttcacg atcaccggtc gtggaaccgt cgtcaccggt 420

cgtgtcgagc gcggcgtgct cctgcctaac gacgaaatcg aaatcgtcgg catcaaggag 480

aagtcgtcca agacgactgt caccgctatc gagatgttcc gcaagaccct gccggatgcc 540

cgtgcaggtg agaacgtcgg tctgctcctc cgcggcacca agcgcgagga tgttgagcgc 600

ggtcaggtca tcgtgaagcc gggttcgatc accccgcaca ccaagttcga ggctcaggtc 660

tacatcctga gcaaggacga gggcggacgt cacaacccgt tctactcgaa ctaccgtccg 720

cagttctact tccggaccac ggacgtcacc ggtgtcatca cgctgcccga gggcaccgag 780

atggtcatgc ccggcgacaa caccgatatg tcggtcgagc tcatccagcc gatcgctatg 840

gaggaccgcc tccgcttcgc aatccgcgaa ggtggccgca ccgtcggcgc cggt 894

<210> 152
<211> 888
<212> DNA
<213> Burkholderia cepacia

<400> 152
atgatcacgg gcgcagcgca gatggacggc gcgatcctgg tttgctcggc agcagacggc 60

ccgatgccgc aaacgcgtga gcacatcctg ctggcgcgtc aggttggtgt tccgtacatc 120

atcgtgttcc tgaacaagtg cgacagtgtg gacgacgctg aactgctcga gctggtcgag 180

atggaagttc gcgaactcct gtcgaagtac gacttcccgg gcgacgacac gccgatcgtg 240

aagggttcgg ccaagctggc gctggaaggc gacacgggcg agctgggcga agtggcgatc 300

atgagcctgg cagacgcgct ggacacgtac atcccgacgc cggagcgtgc agttgacggc 360

gcgttcctga tgccggtgga agacgtgttc tcgatctcgg gccgtggtac ggtggtgacg 420

ggtcgtgtcg agcgcggcat cgtgaaggtc ggcgaagaaa tcgaaatcgt cggtatcaag 480

ccgacggtga agacgacctg cacgggcgtt gaaatgttcc gcaagctgct ggaccaaggt 540

caggcaggcg acaacgtcgg tatcctgctg cgcggcacga agcgtgaaga cgtggagcgt 600

ggccaggttc tggcgaagcc gggttcgatc acgccgcaca cgcacttcac ggctgaagtg 660

tacgtgctga gcaaggacga aggcggccgt cacacgccgt tcttcaacaa ctaccgtccg 720

cagttctact tccgtacgac ggacgtgacg ggctcgatcg agctgccgaa ggacaaggaa 780

atggtgatgc cgggcgacaa cgtgtcgatc acggtgaagc tgattgctcc gatcgcgatg 840

gaagaaggtc tgcgcttcgc aatccgtgaa ggcggccgta cggtcggc 888

<210> 153
<211> 891
<212> DNA
<213> Chlamydia trachomatis

<400> 153
aacatgatca ccggtgcggc tcaaatggac ggggctattc tagtagtttc tgcaacagac 60

ggagctatgc ctcaaactaa agagcatatt cttttggcaa gacaagttgg ggttccttac 120

atcgttgttt ttctcaataa aattgacatg atttccgaag aagacgctga attggtcgac 180

ttggttgaga tggagttggc tgagcttctt gaagagaaag gatacaaagg gtgtccaatc 240

atcagaggtt ctgctctgaa agctttggaa ggagatgctg catacataga gaaagttcga 300

gagctaatgc aagccgtcga tgataatatc cctactccag aaagagaaat tgacaagcct 360

ttcttaatgc ctattgagga cgtgttctct atctccggac gaggaactgt agtaactgga 420

cgtattgagc gtggaattgt taaagtttcc gataaagttc agttggtcgg tcttagagat 480

actaaagaaa cgattgttac tggggttgaa atgttcagaa aagaactccc agaaggtcgt 540

gcaggagaga acgttggatt gctcctcaga ggtattggta agaacgatgt ggaaagagga 600

atggttgttt gcttgccaaa cagtgttaaa cctcatacac agtttaagtg tgctgtttac 660

gttctgcaaa aagaagaagg tggacgacat aagcctttct tcacaggata tagacctcaa 720

ttcttcttcc gtacaacaga cgttacaggt gtggtaactc tgcctgaggg agttgagatg 780

gtcatgcctg gggataacgt tgagtttgaa gtgcaattga ttagccctgt ggctttagaa 840

gaaggtatga gatttgcgat tcgtgaaggt ggtcgtacaa tcggtgctgg a 891

<210> 154
<211> 891
<212> DNA
<213> Escherichia coli

<400> 154
aacatgatca ccggtgctgc gcagatggac ggcgcgatcc tggtagttgc tgcgactgac 60

ggcccgatgc cgcagactcg tgagcacatc ctgctgggtc gtcaggtagg cgttccgtac 120

atcatcgtgt tcctgaacaa atgcgacatg gttgatgacg aagagctgct ggaactggtt 180

gaaatggaag ttcgtgaact tctgtctcag tacgacttcc cgggcgacga cactccgatc 240

gttcgtggtt ctgctctgaa agcgctggaa ggcgacgcag agtgggaagc gaaaatcctg 300

gaactggctg gcttcctgga ttcttacatt ccggaaccag agcgtgcgat tgacaagccg 360

ttcctgctgc cgatcgaaga cgtattctcc atctccggtc gtggtaccgt tgttaccggt 420

cgtgtagaac gcggtatcat caaagttggt gaagaagttg aaatcgttgg tatcaaagag 480

actcagaagt ctacctgtac tggcgttgaa atgttccgca aactgctgga cgaaggccgt 540

gctggtgaga acgtaggtgt tctgctgcgt ggtatcaaac gtgaagaaat cgaacgtggt 600

caggtactgg ctaagccggg caccatcaag ccgcacacca agttcgaatc tgaagtgtac 660

attctgtcca aagatgaagg cggccgtcat actccgttct tcaaaggcta ccgtccgcag 720

ttctacttcc gtactactga cgtgactggt accatcgaac tgccggaagg cgtagagatg 780

gtaatgccgg gcgacaacat caaaatggtt gttaccctga tccacccgat cgcgatggac 840

gacggtctgc gtttcgcaat ccgtgaaggc ggccgtaccg ttggcgcggg c 891

<210> 155
<211> 891
<212> DNA
<213> Fibrobacter succinogenes

<400> 155
aacatggtga ctggtgctgc tcagatggac ggcgctatcc tcgttgttgc cgctactgac 60

ggtccgatgc cgcagactcg cgaacacatc cttctcgctc accaggttgg cgtgccgaag 120

atcgtcgtgt tcatgaacaa gtgcgacatg gttgacgatg ctgaaattct cgacctcgtc 180

gaaatggaag ttcgcgaact cctctccaag tatgacttcg acggtgacaa caccccgatc 240

atccgtggtt ccgctctcaa ggccctcgaa ggcgatccgg aataccagga caaggtcatg 300

gaactcatga acgcttgcga cgaatacatc ccgctcccgc agcgcgatac cgacaagccg 360

ttcctcatgc cgatcgaaga cgtgttcacg attactggcc gcggcactgt cgctactggc 420

cgtatcgaac gcggtgtcgt tcgcttgaac gacaaggttg aacgtatcgg tctcggtgaa 480

accaccgaat acgtcatcac cggtgttgaa atgttccgta agctcctcga cgacgctcag 540

gcaggtgaca acgttggtct cctcctccgt ggtgctgaaa agaaggacat cgtccgtggc 600

atggttctcg cagctccgaa gtctgtcact ccgcacaccg aatttaaggc tgaaatctac 660

gttctcacga aggacgaagg tggccgtcac acgccgttca tgaatggcta ccgtccgcag 720

ttctacttcc gcaccaccga cgttactggt acgatccagc tcccggaagg tgtcgaaatg 780

gttactccgg gtgacacggt cacgatccac gtgaacctca tcgctccgat cgctatggaa 840

aagcagctcc gcttcgctat ccgtgaaggt ggacgtactg ttggtgctgg c 891

<210>  156
<211>  894
<212>  DNA
<213>  Flavobacterium ferrugineum

<400>  156
aacatgatca ccggtgctgc ccagatggac ggtgctatct tagttgtggc tgcatcagac      60

ggtcctatgc ctcaaacaaa agaacacatc ctgcttgctg cccaggtagg tgtacctaaa     120

atggttgtgt ttctgaataa agttgacctc gttgacgacg aagagctcct ggagctggtt     180

gagatcgagg ttcgcgaaga actgactaaa cgcggtttcg acggcgacaa cactccaatc     240

atcaaaggtt ccgctacagg cgccctcgct ggtgaagaaa agtgggttaa agaaattgaa     300

aacctgatgg acgctgttga cagctacatc ccactgcctc ctcgtccggt tgatctgccg     360

ttcctgatga gcgtagagga cgtattctct atcactggtc gtggtactgt tgctaccggt     420

cgtatcgagc gtggccgtat caaagttggt gagcctgttg agatcgtagg tctgcaggag     480

tctcccctga actctaccgt tacaggtgtt gagatgttcc gcaaactcct cgacgaaggt     540

gaagctggtg ataacgccgg tctcctcctc cgtggtgttg aaaaaacaca gatccgtcgc     600

ggtatggtaa tcgttaaacc cggttccatc actccgcaca cggacttcaa aggcgaagtt     660

tacgtactga gcaaagacga aggtggccgt cacactccat tcttcaacaa ataccgtcct     720

caattctact tccgtacaac tgacgttaca ggtgaagtag aactgaacgc aggaacagaa     780

atggttatgc ctggtgataa caccaacctg accgttaaac tgatccaacc gatcgctatg     840

gaaaaaggtc tgaaattcgc gatccgcgaa ggtggccgta ccgtaggtgc agga           894


<210>  157
<211>  891
<212>  DNA
<213>  Haemophilus influenzae

<400>  157
aatatgatta ctggtgcggc acaaatggat ggtgctattt tagtagtagc agcaacagat      60

ggtcctatgc cacaaactcg tgaacacatc ttattaggtc gccaagtagg tgttccatac     120

atcatcgtat tcttaaacaa atgcgacatg gtagatgacg aagagttatt agaattagtc     180

gaaatggaag ttcgtgaact tctatctcaa tatgacttcc caggtgacga tacaccaatc     240

gtacgtggtt cagcattaca agcgttaaac ggcgtagcag aatgggaaga aaaaatcctt     300

gagttagcaa accacttaga tacttacatc ccagaaccag aacgtgcgat tgaccaaccg     360

ttccttcttc caatcgaaga tgtgttctca atctcaggtc gtggtactgt agtaacaggt 420

cgtgtagaac gaggtattat ccgtacaggt gatgaagtag aaatcgtcgg tatcaaagat 480

acagcgaaaa ctactgtaac gggtgttgaa atgttccgta aattacttga cgaaggtcgt 540

gcaggtgaaa acatcggtgc attattacgt ggtaccaaac gtgaagaaat cgaacgtggt 600

caagtattag cgaaaccagg ttcaatcaca ccacacactg acttcgaatc agaagtgtac 660

gtattatcaa aagatgaagg tggtcgtcat actccattct tcaaaggtta ccgtccacaa 720

ttctatttcc gtacaacaga cgtgactggt acaatcgaat taccagaagg cgtggaaatg 780

gtaatgccag gcgataacat caagatgaca gtaagcttaa tccacccaat tgcgatggat 840

caaggtttac gtttcgcaat ccgtgaaggt ggccgtacag taggtgcagg c 891

<210> 158
<211> 906
<212> DNA
<213> Helicobacter pylori

<400> 158
aacatgatca ccggtgcggc gcaaatggac ggagcgattt tggttgtttc tgcagctgat 60

ggccctatgc ctcaaactag ggagcatatc ttattgtctc gtcaagtagg cgtgcctcac 120

atcgttgttt tcttaaacaa acaagacatg gtagatgacc aagaattgtt agaacttgta 180

gaaatggaag tgcgcgaatt gttgagcgcg tatgaatttc ctggcgatga cactcctatc 240

gtagcgggtt cagctttaag agctttagaa gaagcaaagg ctggtaatgt gggtgaatgg 300

ggtgaaaaag tgcttaaact tatggctgaa gtggatgcct atatccctac tccagaaaga 360

gacactgaaa aaactttctt gatgccggtt gaagatgtgt tctctattgc gggtagaggg 420

actgtggtta caggtaggat tgaaagaggc gtggtgaaag taggcgatga agtggaaatc 480

gttggtatca gacctacaca aaaaacgact gtaaccggtg tagaaatgtt taggaaagag 540

ttggaaaaag gtgaagccgg cgataatgtg ggcgtgcttt tgagaggaac taaaaaagaa 600

gaagtggaac gcggtatggt tctatgcaaa ccaggttcta tcactccgca caagaaattt 660

gagggagaaa tttatgtcct ttctaaagaa gaaggcggga gacacactcc attcttcacc 720

aattaccgcc cgcaattcta tgtgcgcaca actgatgtga ctggctctat cacccttcct 780

gaaggcgtag aaatggttat gcctggcgat aatgtgaaaa tcactgtaga gttgattagc 840

cctgttgcgt tagagttggg aactaaattt gcgattcgtg aaggcggtag gaccgttggt 900

gctggt 906

<210>  159
<211>  891
<212>  DNA
<213>  Micrococcus luteus

<400>  159
aacatgatca ccggcgccgc tcagatggac ggcgcgatcc tcgtggtcgc cgctaccgac      60

ggcccgatgg cccagacccg tgagcacgtg ctcctggccc gccaggtcgg cgtgccggcc     120

ctgctcgtgg ccctgaacaa gtcggacatg gtggaggacg aggagctcct cgagcgtgtc     180

gagatggagg tccggcagct gctgtcctcc aggagcttcg acgtcgacga ggccccggtc     240

atccgcacct ccgctctgaa ggccctcgag ggcgaccccc agtgggtcaa gtccgtcgag     300

gacctcatgg atgccgtgga cgagtacatc ccggacccgg tgcgcgacaa ggacaagccg     360

ttcctgatgc cgatcgagga cgtcttcacg atcaccggcc gtggcaccgt ggtgaccggt     420

cgcgccgagc gcggcaccct gaagatcaac tccgaggtcg agatcgtcgg catccgcgac     480

gtgcagaaga ccactgtcac cggcatcgag atgttccaca agcagctcga cgaggcctgg     540

gccggcgaga actgcggtct gctcgtgcgc ggtctgaagc gcgacgacgt cgagcgcggc     600

caggtgctgg tggagccggg ctccatcacc ccgcacacca acttcgaggc gaacgtctac     660

atcctgtcca aggacgaggg tgggcgtcac accccgttct actcgaacta ccgcgcgcag     720

ttctacttcc gcaccaccga cgtcaccggc gtcatcacgc tgcccgaggg caccgagatg     780

gtcatgcccg gcgacaccac cgagatgtcg gtcgagctca tccagccgat cgccatggag     840

gagggcctcg gcttcgccat ccgcgagggt ggccgcaccg tgggctccgg c              891


<210>  160
<211>  891
<212>  DNA
<213>  Mycobacterium tuberculosis

<400>  160
aacatgatca ccggcgccgc gcagatggac ggtgcgatcc tggtggtcgc cgccaccgac      60

ggcccgatgc cccagacccg cgagcacgtt ctgctggcgc gtcaagtggg tgtgccctac     120

atcctggtag cgctgaacaa ggccgacgca gtggacgacg aggagctgct cgaactcgtc     180

gagatggagg tccgcgagct gctggctgcc caggaattcg acgaggacgc cccggttgtg     240

cgggtctcgg cgctcaaggc gctcgagggt gacgcgaagt gggttgcctc tgtcgaggaa     300

ctgatgaacg cggtcgacga gtcgattccg gacccggtcc gcgagaccga caagccgttc     360

ctgatgccgg tcgaggacgt cttcaccatt accggccgcg gaaccgtggt caccggacgt     420

gtggagcgcg gcgtgatcaa cgtgaacgag gaagttgaga tcgtcggcat tcgcccatcg 480

accaccaaga ccaccgtcac cggtgtggag atgttccgca agctgctcga ccagggccag 540

gcgggcgaca acgttggttt gctgctgcgg ggcgtcaagc gcgaggacgt cgagcgtggc 600

caggttgtca ccaagcccgg caccaccacg ccgcacaccg agttcgaagg ccaggtctac 660

atcctgtcca aggacgaggg cggccggcac acgccgttct tcaacaacta ccgtccgcag 720

ttctacttcc gcaccaccga cgtgaccggt gtggtgacac tgccggaggg caccgagatg 780

gtgatgcccg gtgacaacac caacatctcg gtgaagttga tccagcccgt cgccatggac 840

gaaggtctgc gtttcgcgat ccgcgagggt ggccgcaccg tgggcgccgg c 891

<210> 161
<211> 891
<212> DNA
<213> Mycoplasma genitalium

<400> 161
aatatgatca caggtgctgc acaaatggat ggagctattc tagttgtttc agcaactgat 60

agtgtgatgc cccaaacccg cgagcacatc ttacttgccc gccaagtagg ggttcctaaa 120

atggtagttt ttctaaacaa gtgtgatatt gctagtgatg aagaggtaca agaacttgtt 180

gctgaagaag tacgtgatct gttaacttcc tatggttttg atggtaagaa cactcctatt 240

atttatggct cagctttaaa agcattggaa ggtgatccaa agtgggaggc taagatccat 300

gatttgatta aagcagttga tgaatggatt ccaactccta cacgtgaagt agataaacct 360

ttcttattag caattgaaga tacgatgacc attactggta gaggtacagt tgttacagga 420

agagttgaaa gaggtgaact caaagtaggt caagaagttg aaattgttgg tttaaaacca 480

attagaaaag cagttgttac tggaattgaa atgttcaaaa aggaacttga ttcagcaatg 540

gctggtgaca atgctggggt attattacgt ggtgttgaac gtaaagaagt tgaaagaggt 600

caagttttag caaaaccagg ctctattaaa ccgcacaaga aatttaaagc tgagatctat 660

gctttaaaga aagaagaagg tggtagacac actggttttt taaacggtta ccgtcctcaa 720

ttctatttcc gtaccactga tgtaactggt tctattgctt tagctgaaaa tactgaaatg 780

gttctacctg gtgataatgc ttctattact gttgagttaa ttgctcctat cgcttgtgaa 840

aaaggtagta agttctcaat tcgtgaaggt ggtagaactg taggggcagg c 891

<210> 162
<211> 891

<212>  DNA
<213>  Neisseria gonorrhoeae

<400>  162
aacatgatta ccggcgccgc acaaatggac ggtgcaatcc tggtatgttc tgctgccgac      60

ggccctatgc cgcaaacccg cgaacacatc ctgctggccc gtcaagtagg cgtaccttac     120

atcatcgtgt tcatgaacaa atgcgacatg gtcgacgatg ccgagctgtt ccaactggtt     180

gaaatggaaa tccgcgacct gctgtccagc tacgacttcc ccggcgacga ctgcccgatc     240

gtacaaggtt ccgcactgaa agccttggaa ggcgatgccg cttacgaaga aaaaatcttc     300

gaactggcta ccgcattgga cagatacatc ccgactcccg agcgtgccgt ggacaaacca     360

ttcctgctgc ctatcgaaga cgtgttctcc atttccggcc gcggtaccgt agtcaccggc     420

cgtgtagagc gaggtatcat ccacgttggt gacgagattg aaatcgtcgg tctgaaagaa     480

acccaaaaaa ccacctgtac cggcgttgaa atgttccgca aactgctgga cgaaggtcag     540

gcgggcgaca acgtaggcgt attgctgcgc ggtaccaaac gtgaagacgt agaacgcggt     600

caggtattgg ccaaacgggg tactatcact cctcacacca agttcaaagc agaagtgtac     660

gtattgagca aagaagaggg cggcccccat accccgtttt tcgccaacta ccgtccccaa     720

ttctacttcc gtaccactga cgtaaccggc acgattactt tggaaaaagg tgtggaaatg     780

gtaatgccgg gtgagaacgt aaccattact gtagaactga ttgcgcctat cgctatggaa     840

gaaggtctgc gctttgcgat tcgcgaaggc ggccgtaccg tgggtgccgg c              891


<210>  163
<211>  891
<212>  DNA
<213>  Rickettsia prowazekii

<400>  163
aatatgataa ctggtgccgc tcagatggat ggtgctatat tagtagtttc tgctgctgat      60

ggtcctatgc ctcaaactag agaacatata ttactggcaa aacaggtagg tgtacctgct     120

atggtagtat ttttgaataa agtagatatg gtagatgatc ctgacctatt agaattagtt     180

gagatggaag taagagaatt attatcaaaa tatggtttcc ctggtaatga aatacctatt     240

attaaaggtt ctgcacttca agctttagaa ggaaaacctg aaggtgaaaa agctattaat     300

gagttaatga atgcagtaga tacgtatata cctcagccta tagagctaca agataaacct     360

tttttaatgc caatagagga tgtattttct atttcaggca gaggtaccgt tgtaactggt     420

agagtggagt caggcataat taaggtgggt gaagaaattg aaatagtagg tctaaaaaat     480

acgcaaaaaa cgacttgtac aggtgtagaa atgttcagaa aattacttga tgaaggacaa 540

tctggagata atgtcggtat attactacgt ggtacaaaaa gagaagaagt agaaagagga 600

caagtacttg caaaacctgg gagcataaaa ccgcatgata aatttgaagc tgaagtgtat 660

gtgcttagta aagaggaagg tggacgtcat accccattta ctaatgatta tcgcccacag 720

ttctatttta gaacaacaga tgttaccggc acaataaaat tgccttctga taagcagatg 780

gttatgcctg gagataatgc tactttttca gtagaattaa ttaagccgat tgctatgcaa 840

gaagggttaa aattctctat acgtgaaggt ggtagaacag taggagccgg t 891

<210> 164
<211> 891
<212> DNA
<213> Salmonella typhimurium

<400> 164
aacatgatca ccggtgctgc tcagatggac ggcgcgatcc tggttgttgc tgcgactgac 60

ggcccgatgc cgcagacccg tgagcacatc ctgctgggtc gtcaggtagg cgttccgtac 120

atcatcgtgt tcctgaacaa atgcgacatg gttgatgacg aagagctgct ggaactggtt 180

gagatggaag ttcgcgaact gctgtctcag tacgacttcc cgggcgacga cactccgatc 240

gttcgtggtt ctgctctgaa agcgctggaa ggcgacgcag agtgggaagc gaaaatcatc 300

gaactggctg gcttcctgga ttcttatatt ccggaaccag agcgtgcgat tgacaagccg 360

ttcctgctgc cgatcgaaga cgtattctcc atctccggtc gtggtaccgt tgttaccggt 420

cgtgtagagc gcggtatcat caaagtgggc gaagaagttg aaatcgttgg tatcaaagag 480

actcagaagt ctacctgtac tggcgttgaa atgttccgca aactgctgga cgaaggccgt 540

gccggtgaga acgtaggtgt tctgctgcgt ggtatcaaac gtgaagaaat cgaacgtggt 600

caggtactgg ctaagccggg caccatcaag ccgcacacca agttcgaatc tgaagtgtac 660

attctgtcca aagatgaagg cggccgtcat actccgttct tcaaaggcta ccgtccgcag 720

ttctacttcc gtactactga cgtgactggt accatcgaac tgccggaagg cgtagagatg 780

gtaatgccgg gcgacaacat caaaatggtt gttaccctga tccacccgat cgcgatggac 840

gacggtctgc gtttcgcaat ccgtgaaggc ggccgtaccg ttggcgcggg c 891

<210> 165
<211> 881
<212> DNA
<213> Shewanella putida

<400>  165
atgatcactg gtgctgcaca gatggacggc gcgattctgg tagtcgcttc aacagacggt      60

ccaatgccac agactcgtga gcacatcctg ctttctcgtc aggttggcgt accattcatc     120

atcgtattca tgaacaaatg tgacatggta gatgacgaag agctgttaga gctagttgag     180

atggaagtgc gtgaactgtt atcagaatac gatttcccag gtgatgactt accggtaatc     240

caaggttcag ctctgaaagc gctagaaggc gagccagagt gggaagcaaa aatccttgaa     300

ttagcagcgg cgctggattc ttacattcca gaaccacaac gtgacatcga taagccgttc     360

ctactgccaa tcgaagacgt attctcaatt tcaggccgtg gtacagtagt aacaggtcgt     420

gttgagcgtg gtattgtacg cgtaggcgac gaagttgaaa tcgttggtgt acgtgcgaca     480

actaagacaa cgtgtactgg tgtagaaatg ttccgtaaac tgcttgacga aggtcgtgca     540

ggtgagaact gtggtatttt gttacgtggt actaagcgtg atgacgtaga acgtggtcaa     600

gtattagcga agccaggttc aatcaaccca cacactactt ttgaatcaga agtttacgta     660

ctgtcaaaag aagaaggtgg tcgtcacacg ccattcttca aaggctaccg tccacagttc     720

tacttccgta caactgacgt aaccggtact atcgaactgc cagaaggcgt agagatggta     780

atgccaggcg ataacatcaa gatggtagtg acactgattt gcccaatcgc gatggacgaa     840

ggtttacgct tcgcaatccg tgaaggcggt cgtacagtgg t                         881


<210>  166
<211>  897
<212>  DNA
<213>  Stigmatella aurantiaca

<400>  166
aacatgatca cgggcgcggc gcagatggac ggagcgattc tggtggtgtc cgcggccgac      60

ggcccgatgc cccagacgcg tgagcacatc ctgctggcca ggcaggtggg cgtgccctac     120

atcgtcgtct tcctgaacaa ggtggacatg ctggacgatc cggagctgcg cgagctggtg     180

gagatggagg tgcgcgacct gctcaagaag tacgagttcc cgggcgacag catccccatc     240

atccctggca gcgcgctcaa ggcgctggag ggagacacca gcgacatcgg cgagggagcg     300

atcctgaagc tgatggcggc ggtggacgag tacatcccga cgccgcagcg tgcgacggac     360

aagccgttcc tgatgccggt ggaagacgtg ttctccatcg caggccgagg aacggtggcg     420

acgggccgag tggagcgcgg caagatcaag gtgggcgagg aagtggagat cgtggggatc     480

cgtccgacgc agaagacggt catcacgggg gtggagatgt tccgcaagct gctggacgag     540

ggcatggcgg gagacaacat cggagcgctg ctgcgaggcc tgaagcgcga ggacctggag 600

cgtgggcagg tgctggcgaa ctgggggagc atcaacccgc acacgaagtt caaggcgcag 660

gtgtacgtgc tgtcgaagga agagggaggg cggcacacgc cgttcttcaa gggataccgg 720

ccgcagttct acttccggac gacggacgtg accggaacgg tgaagctgcc ggacaacgtg 780

gagatggtga tgccgggaga caacatcgcc atcgaggtgg agctcattac tccggtcgcc 840

atggagaagg agctgccgtt cgccatccgt gagggtggcc gcacggtggg cgccggc 897

<210> 167
<211> 894
<212> DNA
<213> Streptococcus pyogenes

<400> 167
aacatgatca ctggtgccgc tcaaatggac ggagctatcc ttgtagttgc ttcaactgat 60

ggaccaatgc cacaaactcg tgagcacatc cttctttcac gtcaggttgg tgttaaacac 120

cttatcgtgt tcatgaacaa agttgacctt gttgatgacg aagagttgct tgaattagtt 180

gagatggaaa ttcgtgacct tctttcagaa tacgatttcc caggtgatga ccttccagtt 240

atccaaggtt cagctcttaa agctcttgaa ggcgacacta aatttgaaga catcatcatg 300

gaattgatgg atactgttga ttcatacatt ccagaaccag aacgcgacac tgacaaacca 360

ttgcttcttc cagtcgaaga cgtattctca attacaggtc gtggtacagt tgcttcagga 420

cgtatcgacc gtggtactgt tcgtgtcaac gacgaaatcg aaatcgttgg tatcaaagaa 480

gaaactaaaa aagctgttgt tactggtgtt gaaatgttcc gtaaacaact tgacgaaggt 540

cttgcaggag acaacgtagg tatccttctt cgtggtgttc aacgtgacga aatcgaacgt 600

ggtcaagtta ttgctaaacc aagttcaatc aacccacaca ctaaattcaa aggtgaagta 660

tatatccttt ctaaagacga aggtggacgt cacactccat tcttcaacaa ctaccgtcca 720

caattctact tccgtacaac tgacgtaaca ggttcaatcg aacttccagc aggtacagaa 780

atggttatgc ctggtgataa cgtgacaatc aacgttgagt tgatccaccc aatcgccgta 840

gaacaaggta ctactttctc aatccgtgaa ggtggacgta ctgttggttc aggt 894

<210> 168
<211> 897
<212> DNA
<213> Thiobacillus cuprinus

<400> 168
aacatgatca ccggtgcggc ccagatggac ggcgccatcc tggtcgtgtc cgccgccgac 60

ggccccatgc cccaaacccg cgagcacatc ctgctggcgc gtcaggtggg cgtgccctac 120

atcatcgtgt tcctcaacaa gtgcgacatg gtcgacgacg ccgagctgct cgaactcgtc 180

gagatggaag tgcgcgagct gctgtccaag tacgacttcc ccggtgacga cacccccatc 240

atcaagggct cggccaagct ggccctcgaa ggcgacaagg gcgaactggg cgaaggcgcc 300

attctcaagc tggccgaggc cctggacacc tacatcccca cgcccgagcg ggccgtcgac 360

ggcgcgttcc tcatgcccgt ggaagacgtg ttctccatct ccgggcgcgg cacggtggtc 420

accgggcgtg tggagcgcgg catcatcaag gtcggcgagg aaatcgagat tgtcggcctc 480

aagcccaccc tcaagaccac ctgcaccggc gtggaaatgt tcaggaagct gctcgaccag 540

ggccaggccg gcgacaacgt cggcatcttg ctgcgcggca ccaagcgcga ggaagtcgag 600

cgcggccagg tgctgtgcaa acccggctcg atcaagcccc acacccactt caccgccgag 660

gtgtacgtgc tgagcaagga cgagggcggc cgccacaccc ccttcttcaa caactaccgc 720

ccgcagttct acttccgcac caccgacgtc accggcgcca tcgaactgcc caaggacaag 780

gaaatggtca tgcccggcga taatgtgagc atcaccgtca agctcatcgc ccccatcgcc 840

atggaagaag gcctgcgctt cgccatccgc gaaggcggcc gcaccgtcgg cgccggc 897

<210> 169
<211> 894
<212> DNA
<213> Treponema pallidum

<400> 169
aatatgatca cgggtgctgc gcagatggac ggtggtattc tcgtcgtgtc tgcgcctgac 60

ggcgttatgc cacagacgaa ggagcatctt ctgctcgccc gtcaggttgg tgttccctcc 120

atcattgttt ttttgaacaa ggttgatttg gttgatgatc ctgagttgct agagctggtg 180

gaagaagagg tgcgtgatgc gcttgctgga tatgggtttt cgcgtgagac gcctatcgtc 240

aaggggtctg cgtttaaagc tctgcaggat ggcgcttccc cggaggatgc agcttgtatt 300

gaggaactgc ttgcggccat ggattcctac tttgaagacc cagtgcgtga cgacgcaaga 360

cctttcttgc tctctatcga ggatgtgtac actatttctg ggcgtggtac cgttgtcacg 420

gggcgcatcg aatgtggggt aattagtctg aatgaagagg tcgagatcgt cgggattaag 480

cccactaaga aaacagtggt tactggcatt gagatgttta ataagttgct tgatcaggga 540

attgcaggtg ataacgtggg gctgcttttg cgcggggtgg ataaaaaaga ggttgagcgc 600

ggtcaggtgc tttctaagcc cggttctatt aagccacaca ccaagtttga ggcgcagatc 660

tacgtgctct ctaaggaaga gggtggccgt cacagtcctt tttttcaagg ttatcgtccg 720

cagttttatt ttagaactac tgacattacc ggtacgattt ctcttcctga aggggtagac 780

atggtgaagc cgggggataa caccaagatt ataggtgagc tcatccaccc gatagctatg 840

gacaagggtc tgaagcttgc gattcgtgaa ggggggcgca ctattgcttc tggt 894

<210> 170
<211> 891
<212> DNA
<213> Ureaplasma urealyticum

<400> 170
aatatgatta caggggcagc acaaatggat ggagcaattt tagttattgc tgcatctgat 60

ggggttatgg ctcaaactaa agaacatatt ttattagcac gtcaagttgg tgttccaaaa 120

atcgttgttt tcttaaacaa atgtgatttc atgacagatc cagatatgca agatcttgtt 180

gaaatggaag ttcgtgaatt attatctaaa tatggatttg atggcgataa cacaccagtt 240

attcgtggtt caggtcttaa ggctttagaa ggagatccag tttgagaagc aaaaattgat 300

gaattaatgg acgcagttga ttcatgaatt ccattaccag aacgtagtac tgacaaacca 360

ttcttattag caattgaaga tgtattcaca atttcaggac gtggtacagt agtaactgga 420

cgtgttgaac gtggtgtatt aaaagttaat gatgaggttg aaattgttgg tctaaaagac 480

actcaaaaaa ctgttgttac aggaattgaa atgtttagaa aatcattaga tcaagctgaa 540

gctggtgata atgctggtat tttattacgt ggtattaaaa aagaagatgt tgaacgtggt 600

caagtacttg taaaaccagg atcaattaaa cctcaccgta cttttactgc taaagtttat 660

attcttaaaa aagaagaagg tggacgtcat acacctattg tttcaggata ccgtccacaa 720

ttctatttta gaacaacaga tgtaacaggt gctatttcat tacctgctgg tgttgatttg 780

gttatgccag gtgatgacgt tgaaatgact gtagaattaa ttgctccagt tgcgattgaa 840

gatggatcta aattctcaat ccgtgaaggt ggtaaaactg taggtcatgg t 891

<210> 171
<211> 909
<212> DNA
<213> Wolinella succinogenes

<400> 171
aacatgatta caggtgctgc tcaaatggat ggcgcgattc ttgttgtttc tgcggcggat 60

ggccccatgc cccaaactag ggagcacatt cttctttctc gacaagtagg cgttccttac 120

atcgtggttt tcttgaacaa agaagatatg gttgatgacg ctgagcttct tgagcttgtt    180

gaaatggaag ttagagaact tcttagcaac tacgacttcc ctggagatga cactcctatc    240

gttgcaggtt ccgctcttaa agctcttgaa gaggctaacg accaggaaaa tgttggcgag    300

tggggcgaga aagtattgaa gcttatggct gaggttgacc gatatattcc tacgcctgag    360

cgagatgtgg ataagccttt ccttatgcct gttgaagacg tattctccat cgcgggtcgt    420

ggaaccgttg tgacaggaag aattgaaaga ggcgtggtta aagtcggtga cgaagtagaa    480

atcgttggta tccgaaacac acaaaaaaca accgtaactg gcgttgagat gttccgaaaa    540

gagctcgaca agggtgaggc gggtgacaac gttggtgttc ttttgagagg caccaagaaa    600

gaagatgttg agagaggtat ggttctttgt aaaataggtt ctatcactcc tcacactaac    660

tttgaaggtg aagtttacgt tctttccaaa gaggaaggcg gacgacacac tccattcttc    720

aatggatacc gacctcagtt ctatgttaga actacagacg ttaccggttc tatctctctt    780

cctgagggcg tagagatggt tatgcctggt gacaacgtta agatcaatgt tgagcttatc    840

gctcctgtag ccctcgaaga gggaacacga ttcgcgatcc gtgaaggtgg tcgaaccgtt    900

ggtgcgggt                                                            909

<210>  172
<211>  26
<212>  DNA
<213>  Artificial Sequence

<220>
<223>  Oligonucleotide that binds to any fungal species
       (tuf gene)

<220>
<221>  misc_feature
<222>  (6)..(6)
<223>  n = inosine

<220>
<221>  misc_feature
<222>  (12)..(12)
<223>  n = inosine

<220>
<221>  misc_feature
<222>  (18)..(18)
<223>  n = inosine

<400>  172
tartcngtra angcytcnac rcacat                                          26

<210>  173
<211>  21
<212>  DNA
<213>  Artificial Sequence

<220>
<223>  Oligonucleotide that binds to antibiotic resistance gene of Enterococcus
spp.

<400>  173
tctttagcag aacaggatga a                                                 21


<210>  174
<211>  20
<212>  DNA
<213>  Artificial Sequence

<220>
<223>  Oligonucleotide that binds to antibiotic resistance gene of Enterococcus
spp.

<400>  174
gaataattcc atatcctccg                                                   20